(19) Europäisches Patentamt
European Patent Office
Office européen des brevets

(11) **EP 3 662 265 B1**

(12) **FASCICULE DE BREVET EUROPEEN**

(45) Date de publication et mention de la délivrance du brevet:
**01.07.2026 Bulletin 2026/27**

(21) Numéro de dépôt: **18750426.1**

(22) Date de dépôt: **03.08.2018**

(51) Classification Internationale des Brevets (IPC):
***G01N 21/64*** *(2006.01)* ***C09K 11/77*** *(2006.01)*

(52) Classification Coopérative des Brevets (CPC):
**G01N 21/6428; C09K 11/7776; C09K 11/7794;**
G01N 21/6452; G01N 2021/6439

(86) Numéro de dépôt international:
**PCT/EP2018/071194**

(87) Numéro de publication internationale:
**WO 2019/025618 (07.02.2019 Gazette 2019/06)**

(54) **PROCÉDÉ DE DÉTECTION ULTRA-SENSIBLE À L'AIDE DE PARTICULES PHOTOLUMINESCENTES**

ULTRAEMPFINDLICHES NACHWEISVERFAHREN UNTER VERWENDUNG VON FOTOLUMINESZENTEN TEILCHEN

ULTRA-SENSITIVE DETECTION METHOD USING PHOTOLUMINESCENT PARTICLES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.08.2017 FR 1757554**

(43) Date de publication de la demande:
**10.06.2020 Bulletin 2020/24**

(73) Titulaires:
- **Ecole Polytechnique**
  **91128 Palaiseau Cedex (FR)**
- **Centre National de la Recherche Scientifique**
  **75794 Paris Cedex 16 (FR)**

(72) Inventeurs:
- **PREIRA, Pascal**
  **91600 Savigny-sur-Orge (FR)**
- **RICHLY, Maximilian**
  **75006 Paris (FR)**
- **BOUZIGUES, Cédric**
  **75116 Paris (FR)**
- **ALEXANDROU, Antigoni**
  **91120 Palaiseau (FR)**
- **GACOIN, Thierry**
  **91440 Bures-sur-Yvette (FR)**

(74) Mandataire: **Ipsilon**
**12 Avenue d'Italie**
**75013 Paris (FR)**

(56) Documents cités:
**WO-A1-2013/132197 US-A1- 2009 079 978**
**US-A1- 2010 009 458**

- **ESCUDERO ALBERTO ET AL: "Rare earth based nanostructured materials: synthesis, functionalization, properties and bioimaging and biosensing applications", NANOPHOTONICS, vol. 6, no. 5, 29 June 2017 (2017-06-29), pages 881 - 921, XP055899017, DOI: 10.1515/nanoph-2017-0007**
- **DOMITILLE GIAUME ET AL: "Functionalized Luminescent Oxide Nanoparticles as Biological Probes", MRS PROCEEDINGS, vol. 942, 1 January 2006 (2006-01-01), XP055445327, DOI: 10.1557/PROC-0942-W12-05**
- **NAN WANG ET AL: "Journal of Colloid and Interface Science", JOURNAL OF COLLOID AND INTERFACE SCIENCE, vol. 407, 20 June 2013 (2013-06-20), pages 22 - 28, XP055505913**
- **DOMITILLE GIAUME ET AL: "Functionalized Luminescent Oxide Nanoparticles as Biological Probes", MRS PROCEEDINGS, vol. 942, 1 January 2006 (2006-01-01), XP055445327, DOI: 10.1557/PROC-0942-W12-05**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

• DROZDOWICZ-TOMSIA K ET AL: "Europium and Samarium doped Ga2O3 Nanoparticles as Potential New Fluorescent Labels", NANOSCIENCE AND NANOTECHNOLOGY, 2006. ICONN '06. INTERNATIONAL CO NFERENCE ON, IEEE, PI, 3 July 2006 (2006-07-03), XP031333689, ISBN: 978-1-4244-0452-0

## Description

La présente invention concerne le domaine de la recherche, de la bioanalyse et du diagnostic *in vitro*. Elle a plus particulièrement pour objet l'utilisation de nanoparticules inorganiques photoluminescentes à propriétés optiques et physico-chimiques contrôlées pour la quantification ultra-sensible *in vitro* de substances d'intérêt biologique ou chimique, par exemple de biomarqueurs, d'anticorps, d'ADN, d'ARN et autres composés, dans un échantillon, en particulier un échantillon biologique, par détection de l'émission de luminescence .

La détection et/ou la quantification des concentrations de biomarqueurs, d'anticorps ou d'ADN et d'ARN dans des échantillons biologiques (sang, sérum, salive, urine, liquide cérébro-spinal, etc.) sont indispensables pour le diagnostic médical.

Dans le domaine de la recherche, des diagnostics *in vitro* ou *ex vivo,* de l'analyse médicale et de la bioanalyse, un certain nombre de méthodes ont été proposées pour détecter et/ou doser la présence de substances spécifiques.

Ces méthodes reposent, d'une manière générale, sur l'utilisation d'une sonde mise en œuvre pour détecter et/ou quantifier une concentration en solution. Ces sondes sont couplées à un composé de reconnaissance, ou agent de ciblage, permettant de se fixer à l'espèce moléculaire à analyser. Ce composé de reconnaissance peut être une molécule, ADN, aptamère, protéine ou anticorps. Ensuite, les sondes qui se sont fixées sur l'espèce moléculaire à analyser grâce au composé de reconnaissance peuvent être détectées au moyen d'une ou plusieurs méthodes basées par exemple sur leur luminescence, leur absorbance, leur réactivité chimique, leur radioactivité, etc.

Les essais biochimiques les plus couramment mis en œuvre sont des essais d'immuno-absorption enzymatique (en langue anglaise « Enzyme-Linked Immuno Assay » ou ELISA), qui reposent généralement sur l'utilisation de la peroxydase de raifort comme enzyme pour provoquer une réaction avec un substrat et quantifier la réaction chimique ayant lieu par mesure de l'absorbance du produit de la réaction dans la solution. Le choix du composé de reconnaissance moléculaire auquel la sonde est couplée est déterminant pour l'efficacité de ces sondes. Plus précisément, l'efficacité de ces méthodes dépend de l'affinité spécifique du composé de reconnaissance avec la substance cible. A titre d'exemples, les publications référencées [1] et [2] détaillent les caractéristiques de ces mécanismes.

Les sondes luminescentes mènent généralement à une détection plus sensible que les sondes détectées par leur absorbance car, dans le premier cas, les mesures de l'intensité lumineuse se font sur fond noir, tandis que, dans le deuxième, il s'agit de mesurer une variation de l'intensité lumineuse (mesures sur fond clair).

Parmi les autres méthodes de dosage proposées à l'heure actuelle, on peut également citer le dosage par électrochimiluminescence (ECLIA), le dosage immunologique par fluorescence (FIA) et le dosage radio-immunologique (RIA).

Ces méthodes présentent toutefois divers inconvénients qui limitent leur sensibilité finale de détection, en particulier des limitations en termes de propriétés de luminescence des sondes mises en œuvre (ECLIA, FIA), des risques pour la sécurité, un équipement coûteux et la nécessité d'utilisateurs spécialisés (pas de machines automatisées) pour conduire des essais de type RIA.

En particulier, les sondes luminescentes actuellement disponibles présentent plusieurs désavantages qui ne permettent pas d'exploiter pleinement leur potentiel en tant que sondes de diagnostic. Parmi ces inconvénients, on peut citer par exemple le phénomène de photoblanchiment dans le cas de fluorophores organiques qui, suite à des modifications structurales irréversibles induites par l'illumination, se traduit par une disparition de la fluorescence, ou encore le phénomène de clignotement de l'émission pour les nanocristaux de semi-conducteurs, ou « quantum dots », les sondes cessant alors périodiquement d'émettre et étant par conséquent inadaptées pour produire un signal constant. D'autres inconvénients résultent par exemple de la largeur du spectre d'émission des sondes luminescentes. De fait, un spectre d'émission trop large rend difficile le filtrage du signal de fond éventuellement présent, et a une incidence sur la qualité du signal et, en particulier, sur le rapport signal à bruit. Il convient également de prendre en compte, en plus des facteurs optiques qui contribuent à l'efficacité de la sonde dans un test biologique, le caractère pratique et la facilité d'utilisation de la sonde. Ainsi, certaines particules, comme c'est le cas des nanocristaux de semi-conducteurs, perdent leurs caractéristiques de luminescence après congélation, ce qui représente un inconvénient pour le stockage des agents bio-conjugués. La facilité de couplage des sondes au composé moléculaire permettant de cibler les molécules souhaitées est également un aspect à prendre en considération pour le choix de la sonde adéquate. Ainsi, un certain nombre de particules, dont les nanocristaux de semi-conducteurs, sont synthétisées dans des solvants organiques. Il en découle qu'une utilisation pour des applications biologiques nécessite des étapes supplémentaires de préparation de la surface pour réaliser une dispersion dans l'eau de ces particules, processus qui peut être complexe à mettre en œuvre et être peu stable dans le temps [3].

Par ailleurs, les propriétés colloïdales des particules/sondes sont déterminantes pour la conduite des essais biologiques. De fait, des solutions de bonne stabilité colloïdale sont à même de fournir des milieux de bonne homogénéité pour les essais, et donc une meilleure reproductibilité au niveau des résultats de ces essais.

Enfin, la complexité et le coût sont des aspects importants dans le choix des sondes pour le diagnostic. Par exemple, des nanoparticules d'or, et leurs propriétés en termes de résonance plasmonique de surface, ont été proposées

comme sondes de diagnostic, mais n'ont pas percé comme sondes pour le diagnostic *in vitro,* possiblement à cause de la complexité de la méthode de détection [4], ou éventuellement d'un coût élevé. Quant aux cristaux de semi-conducteurs, ils sont synthétisés dans des solvants organiques et requièrent des procédures de dispersion en milieu aqueux, ce qui rend leur synthèse complexe et donc coûteuse. Leur fonctionnalisation avec des groupements chimiques, permettant le couplage aux composés moléculaires de reconnaissance des molécules cibles, repose en outre sur des liaisons chimiques faibles, ce qui limite par conséquent leur stabilité et est préjudiciable pour la reproductibilité des tests de détection.

D'autre part, les méthodes de détection *in vitro* actuellement disponibles ne donnent pas entière satisfaction, notamment en termes de sensibilité de détection pouvant être atteinte, afin d'élargir le champ d'application des méthodes de diagnostic *in vitro,* par exemple en permettant une détection plus précoce des maladies ou en permettant un diagnostic sur l'évolution d'une maladie ou sur l'effet d'un traitement thérapeutique.

Pour améliorer la sensibilité de détection, deux techniques commerciales d'immuno-essais ultrasensibles ont été développées. Il s'agit des méthodes développées par Quanterix et Singulex. Elles reposent sur l'utilisation de billes magnétiques fonctionnalisées comme surfaces réactives pour la capture des molécules cibles. La technologie de Quanterix procède ensuite à la capture des billes individuelles fonctionnalisées avec des anticorps dirigés contre les antigènes. Chaque bille est piégée dans un puit et leur analyse est faite par un test standard ELISA. Quant à Singulex, il utilise les billes pour concentrer les analytes piégés et détermine ensuite leur concentration par comptage des signaux fluorescents à l'aide d'un dispositif de détection confocale et avec un laser d'excitation qui balaie l'échantillon de manière hélicoïdale.

Ces deux techniques, bien qu'elles permettent d'atteindre des performances en termes de sensibilité de détection plus élevées que les technologies conventionnelles de détection discutées précédemment, sont toutefois hautement complexes et coûteuses. Elles nécessitent l'utilisation d'un appareillage spécifiquement dédié à ces techniques de détection, et non compatible avec les dispositifs actuels de diagnostic *in vitro* automatisés.

Les nanocristaux de semi-conducteurs ou « quantum dots » ont également été proposés dans des tests de détection ultrasensibles ([5], [6], [7], [8] et [9]). Toutefois, l'efficacité de ces tests est limitée par les inconvénients liés à ce type de sondes luminescentes, comme discuté précédemment : complexité et coût élevé de leur synthèse et fonctionnalisation, stabilité colloïdale non satisfaisante, pertes des propriétés de luminescence après congélation.

Enfin, les méthodes basées sur l'utilisation de nanoparticules d'or en exploitant des phénomènes comme la détection des plasmons de surface, le quenching de fluorescence, le dépôt d'argent sur les nanoparticules d'or etc. ([9] à [13]) permettent d'atteindre des sensibilités de détection élevées mais sont généralement hautement complexes.

La demande WO 2013/132197 décrit des nanoparticules pourvues d'une partie magnétique et d'une partie luminescence, en particulier une partie de formule $X_aL_b(M_pO_q)$ et une partie de formule $A_eX'_f(M'_pO_q')$ à des fins d'agent de contraste des techniques d'imagerie.

La publication « Functionalized luminescent oxide nanoparticles as biological probes » Domitille Giaume et al, MRS Proceedings, vol 942, 01.11.2006 décrit une méthode permettant le couplage d'un agent de ciblage sur la surface de nanoparticules à base d'oxydes de terres rares et mentionne leur usage comme sonde biologique. Les particules Y0,95 Eu0.05 VO4 sont utilisées comme sondes biologiques pour localiser les canaux transmembranaires sodique d'une cellule.

Il demeure par conséquent un besoin de développer une méthode de détection/quantification permettant d'atteindre des performances en termes de sensibilité de détection plus élevées que les technologies conventionnelles, de type ELISA, ECLIA, FIA ou RIA, et ne présentant pas les inconvénients, notamment en termes de complexité et de coût, des méthodes ultrasensibles déjà proposées.

La présente invention vise précisément à proposer une nouvelle méthode de détection ultrasensible, reposant sur l'utilisation de nanoparticules luminescentes dopées par des ions de terres rares aux propriétés optiques et physico-chimiques contrôlées.

Ainsi, l'invention concerne, selon un premier de ses aspects, l'utilisation pour la quantification ultra-sensible *in vitro* d'une teneur inférieure à 10 pM d'une substance d'intérêt biologique ou chimique dans un échantillon, en particulier un échantillon biologique, de nanoparticules inorganiques photoluminescentes telles que décrites ci-après , formées en tout ou partie d'une nanoparticule inorganique photoluminescente formée d'une matrice cristalline présentant au moins $10^3$ ions de terres rares, et couplées à au moins un agent de ciblage de la substance à analyser,
lesdites nanoparticules présentant une taille moyenne supérieure ou égale à 20 nm et strictement inférieure à 1 $\mu$m, en particulier comprise entre 20 nm et 500 nm, de préférence entre 20 nm et 200 nm et notamment entre 20 nm et 100 nm, et étant aptes à émettre de la luminescence après absorption d'un photon ; ladite utilisation mettant en œuvre

(i) une excitation des ions de terres rares des particules associées avec la substance à analyser par un dispositif d'illumination, en particulier de type laser, d'une puissance d'au moins 50 mW, de préférence d'au moins 500 mW et d'une intensité d'excitation d'au moins 1 W/cm$^2$, de préférence d'au moins 10 W/cm$^2$ ;
(ii) une détection de l'émission de luminescence par les particules, et

(iii) la détermination de la concentration de la substance par interprétation de ladite mesure de luminescence, par référence à un étalon ou étalonnage.

L'échantillon peut être un échantillon biologique, en particulier un échantillon de prélèvement humain, par exemple choisi parmi le sang, le sérum, le plasma, la salive, l'urine et le liquide cérébro-spinal. L'échantillon peut également être de la matière fécale diluée, du frottis vaginal ou nasal ou de l'expectoration.

Un diluant peut être utilisé avec l'échantillon à analyser, notamment lorsque l'échantillon liquide est du plasma, du sérum, du sang total, du frottis nasal ou vaginal ou de l'expectoration par exemple.

Il peut encore s'agir d'une solution contenant des molécules biologiques.

L'utilisation selon l'invention peut ainsi être mise en œuvre pour la quantification de biomarqueurs, d'anticorps, d'ADN et/ou d'ARN dans un échantillon biologique.

L'utilisation selon l'invention met plus particulièrement en œuvre, en étape (ii), la détection de la luminescence par les particules après une absorption à un photon.

Selon l'utilisation selon l'invention, le signal détecté correspond ainsi à l'émission de luminescence par les nanoparticules photoluminescentes après absorption d'un unique photon, autrement dit de l'émission à une longueur d'onde plus grande que celle de l'excitation. Les nanoparticules photoluminescentes selon l'invention sont ainsi distinctes de particules de phosphores à conversion ascendante, dites particules à « up-conversion », pour lesquelles la détection de l'émission de luminescence est réalisée après une absorption à deux photons.

La méthode ultrasensible selon l'utilisation selon l'invention s'avère avantageuse à plusieurs titres.

Tout d'abord, comme illustré dans les exemples, la méthode selon l'utilisation selon l'invention permet d'atteindre une performance en termes de sensibilité de détection hautement plus élevée que les performances des techniques de détection conventionnelles de type ELISA, ECLIA, FIA ou RIA.

De manière avantageuse, la méthode ultrasensible de l'utilisation selon l'invention permet ainsi une détection au moins dix fois, en particulier au moins 100 fois, plus sensible que la méthode d'immuno-détection enzymatique de type ELISA utilisant les mêmes anticorps de reconnaissance et de ciblage.

La méthode ultrasensible selon l'utilisation selon l'invention permet ainsi une quantification d'une substance d'intérêt présente dans un échantillon en une teneur strictement inférieure à 10 pM, voire même inférieure à 1 pM, voire encore inférieure à 0,1 pM, voire encore à 0,01 pm (10 fM). Ces concentrations dépendent de la molécule cible et, en particulier, de l'affinité du composé de reconnaissance, ou composé de ciblage, couplé à la sonde, mais sont comparables à celles détectables par les méthodes ultra-sensibles (Quanterix ou Singulex).

D'autre part, la méthode de l'utilisation selon l'invention repose sur la mise en œuvre de nanoparticules dopées par des ions de terres rares, de formule $(A_{1-x}Ln_x)_a(M_pO_q)$ (I) telles que définies plus précisément dans la suite du texte, telles que des nanoparticules de type $YVO_4$ :Eu ou $GdVO_4$ :Eu, YAG :Ce, qui présentent, à titre de sondes luminescentes, des propriétés particulièrement avantageuses.

Des nanoparticules à base de vanadate d'yttrium dopé par des terres rares ont par exemple été décrites de façon détaillée [14] et [15].

Ces nanoparticules sont particulièrement avantageuses au regard de leur excellente photostabilité, qui autorise l'acquisition d'un signal constant et prolongé, et une absence de phénomène de clignotement de l'émission. De plus, elles présentent un spectre d'émission étroit et un grand décalage de Stokes (de l'ordre de 350 nm pour les nanoparticules dopées à l'Eu) de l'émission. Ces nanoparticules contiennent également plusieurs dizaines de milliers d'ions pouvant être excités et responsables de la luminescence. L'augmentation de l'intensité d'excitation induit ainsi une augmentation de la luminosité des particules, la saturation de l'émission n'étant atteinte que pour des intensités impraticables.

Enfin, ces nanoparticules ne perdent pas leur luminescence après congélation.

Les nanoparticules photoluminescentes à base de terres rares ont déjà été proposées à titre de sondes luminescentes dans diverses applications [16].

Par exemple, Dosev *et al.* [17] tirent profit des propriétés de luminescence de nanoparticules luminescentes de Eu :$Gd_2O_3$ par excitation de la matrice $Gd_2O_3$, pour la détection de microstructures de protéines déposées sur un substrat. Quant à Yi *et al.* [18], ils utilisent des phosphores à conversion ascendante (« up-conversion ») de photons de nature $NaYF_4$:Yb,Er, qui absorbent deux photons proche-IR pour l'émission d'un photon dans le visible.

Des nanoparticules photoluminescentes à base de terres rares ont également déjà été mises en œuvre pour la détection de particules uniques ainsi que pour le suivi de molécules uniques, en tirant profit de l'absence de clignotement pour la détection de particules uniques, par rapport aux nanoparticules de semiconducteurs ou Quantum Dots ([19] et [20]). Toutefois, il n'était nullement prévisible que ces nanoparticules à base d'ions lanthanides puissent être utilisées pour la détection et la quantification ultra-sensible de biomolécules, dans le cas d'une détection d'ensemble de biomolécules. En effet, en dehors de l'absence de clignotement, les propriétés de luminescence des nanoparticules luminescentes à base de terres rares sont considérées comme inférieures à celles des Quantum Dots. Dans ces particules, en particulier celles constituées d'une matrice d'oxyde métallique où certains ions sont substitués par des ions de terres rares, l'excitation de la luminescence peut se faire soit par excitation de la matrice suivie d'un transfert de l'énergie vers les ions

de terres rares luminescents, soit par excitation directe dans le visible des ions de terres rares luminescents. Concernant l'excitation de la matrice, sa bande d'absorption se situe en général dans l'UV ce qui présente deux inconvénients : peu de lasers sont disponibles actuellement à ces longueurs d'onde et les lasers existants sont volumineux et onéreux ; et, à ces longueurs d'onde l'absorption, les biomolécules absorbent et émettent fortement, ce qui peut produire des signaux parasites. Concernant l'excitation directe des ions de terres rares, le coefficient d'extinction pour une nanoparticule est plus faible que celui des Quantum Dots mais peut être comparable à celui d'un fluorophore organique efficace [16]. De plus, les pics d'absorption directe des ions de terres rares dans le visible sont en général très étroits spectralement, ce qui impose l'utilisation d'un laser étroit spectralement pour une excitation efficace directe des ions terres rares luminescents. Ce type de laser, en particulier sous forme de diode laser compacte et peu onéreuse, n'était pas disponible pour les longueurs d'onde concernées pour l'excitation directe des ions de terres rares luminescents, jusqu'à très récemment.

Yuan *et al.* [21], dans un article de revue, présentent des tests biologiques de luminescence résolus en temps avec mise en œuvre de nanoparticules à base de lanthanides. Les sondes luminescentes à base de lanthanides sont des particules comprenant des complexes de lanthanide, ce qui limite le nombre d'ions lanthanides par particule pour un volume de particule donné, ou des nanoparticules à « up-conversion ». Par exemple, dans les publications mettant en œuvre des nanoparticules comprenant des complexes de lanthanides, les nanoparticules de faible diamètre (8-9 nm) contiennent seulement entre 3000 et 5000 ions [28]. Seules des nanoparticules de tailles bien plus importantes, en particulier supérieure à 100 nm, peuvent contenir de l'ordre de 30 000 ions ou plus [22] et [23].

De même, Corstjens *et al.* [24] utilisent des nanoparticules à « up-conversion » pour la détection *in vitro* de l'IFN-gamma dans des cellules mononucléées du sang périphérique humain. Les nanoparticules à « up-conversion » sont adaptées pour l'imagerie de tissus en profondeur (en général, excitation dans le proche IR de l'$Yb^{3+}$ où les tissus absorbent peu par rapport au visible). En revanche, du fait que l'excitation nécessite l'absorption de deux photons, des intensités d'excitation importantes sont nécessaires et le nombre de photons émis est relativement faible.

Des complexes ou chélates de lanthanides sont également proposés à titre de sondes luminescentes pour des immuno-essais dans les publications [23], [25], [26] et [27]. Cependant, ces complexes ou chélates ne contiennent typiquement qu'un seul ion lanthanide ou, au mieux, quelques (moins de dix) ions lanthanides.

Enfin, on peut encore citer la publication Zhou *et al.* [28] qui propose une méthode de détection de sensibilité améliorée à partir de nanoparticules inorganiques dopées par des lanthanides, à l'issue d'un protocole complexe de dissolution des nanoparticules et de détection de l'émission des micelles contenant les lanthanides ainsi formées.

Quant au document EP 1 282 824, il décrit la mise en œuvre de nanoparticules luminescentes inorganiques, modifiées en surface, à titre de sondes pour détecter une substance biologique ou autre substance organique. La méthode de détection proposée dans ce document repose sur le principe de détection ELISA. Cependant, ce document ne propose nullement leur mise en œuvre pour une détection ultra-sensible.

On peut encore citer le document US 7 550 201 qui propose la mise en œuvre de nanoparticules inorganiques dopées par des ions lanthanide, notamment pour leur application en diagnostic. Ce document ne propose toutefois nullement leur mise en œuvre pour une méthode de détection ultra-sensible.

Egalement, les publications Son *et al.* [36] et Nichkova *et al.* [37] proposent la mise en œuvre de nanoparticules Eu :$Gd_2O_3$ comme alternative aux fluorophores organiques à titre de sondes pour la détection de l'ADN et pour la détection d'acide phénoxybenzoïque, respectivement. Cependant, ces documents ne suggèrent nullement leur mise en œuvre pour une détection ultra-sensible.

Ainsi, il n'a jamais été proposé de tirer profit de nanoparticules photoluminescentes telles que définies précédemment, combinant des propriétés optiques et physico-chimiques spécifiques, dans une méthode de détection ultra-sensible selon l'utilisation selon l'invention.

Ces nanoparticules photoluminescentes peuvent être obtenues par des méthodes de synthèse conventionnelles, connues de l'homme du métier.

La matrice cristalline formant la nanoparticule inorganique photoluminescence peut être une matrice d'oxyde, par exemple une matrice de vanadate ou de phosphate ; une matrice d'halogène, par exemple une matrice de fluorure ; ou encore une matrice de chalcogénure, par exemple de tellure, sulfure, séléniure.

Alternativement, de manière avantageuse, comme détaillé dans la suite du texte, les inventeurs ont développé une nouvelle voie de synthèse de nanoparticules dopées par des ions terres-rares mettant en œuvre des cations tétraalkylammonium en surface des nanoparticules, et permettant d'accéder à une stabilité améliorée des nanoparticules en milieu aqueux et, avantageusement, à une meilleure reproductibilité de leur synthèse et des étapes ultérieures de fonctionnalisation et de couplage à un agent de ciblage.

Enfin, la méthode ultra-sensible de l'utilisation selon l'invention s'avère particulièrement avantageuse en termes de facilité de mise en œuvre et de coût, en comparaison aux technologies ultra-sensibles discutées précédemment.

Avantageusement, et contrairement aux technologies complexes développées par Quanterix et Singulex, elle met en œuvre un appareillage de détection de faible encombrement, de coût limité et dont chaque élément est aisément disponible à l'achat, pour l'excitation et la mesure de luminescence émise par les nanoparticules, comme détaillé dans la suite du texte, et ne requiert aucun élément d'appareillage spécifiquement dédié à la méthode de détection ultra-sensible

de l'invention. Avantageusement, elle est ainsi compatible avec une intégration dans un dispositif d'analyse automatisé, moyennant une adaptation d'ergonomie limitée.

La méthode de l'utilisation selon l'invention est en outre adaptée pour des analyses multiplexées. Ainsi, l'utilisation selon l'invention peut être mise en œuvre pour la quantification simultanée d'au moins deux substances différentes dans un échantillon, suivant une procédure présentée plus loin (Figure 22).

Egalement, comme détaillé dans la suite du texte, il est possible de tirer profit de la durée d'émission longue (supérieure à 1 μs, voire supérieure à 10 μs, voire supérieure à 100 μs), des particules de l'invention pour effectuer une détection résolue en temps, en particulier une détection retardée de l'émission. Une mesure de la luminescence résolue en temps a par exemple été décrite dans le document WO 03008974. l'utilisation selon l'invention permet avantageusement d'effectuer une détection de luminescence résolue en temps, en utilisant du matériel peu sophistiqué et peu onéreux, en particulier un hacheur mécanique, un photomultiplicateur classique et un convertisseur A-D de 100 kHz, comme décrit dans la suite du texte.

l'utilisation selon l'invention permet de réaliser un diagnostic *in vitro.* Avantageusement, la possibilité par l'utilisation selon l'invention de détecter des teneurs ultra faibles de certaines substances dans des échantillons biologiques permet, par exemple, l'utilisation pour une détection plus précoce des maladies, ou encore un diagnostic sur l'évolution d'une maladie ou sur l'effet d'un traitement thérapeutique. De plus, l'utilisation selon l'invention permet l'utilisation comme biomarqueurs de substances dont la concentration est actuellement trop faible pour être détectée avec les méthodes classiques. Elle rend également détectables dans des milieux biologiques facilement accessibles (salive, urine, sang, etc.) des biomarqueurs dont la concentration est trop faible pour être détectée avec les méthodes classiques et nécessite des méthodes invasives comme le prélèvement de liquide céphalorachidien par exemple.

Les maladies pouvant être ne sont pas limitées et comprennent toutes maladies révélées par la présence d'un marqueur spécifique de la maladie, du type molécule d'intérêt biologique (protéine, acide nucléique...), pour lequel il existe un ou des partenaire(s) de liaison spécifique (ligand, anticorps, acides nucléiques complémentaires, aptamères, ...).

A titre d'exemples, on peut citer les maladies infectieuses (bactériennes, parasitaires, ou virales, telles que le SIDA), les maladies inflammatoires et auto-immunes, les maladies cardiologiques, neurologiques, ou oncologiques (par exemple, les cancers solides tels que le cancer du sein ou de la prostate).

L'utilisation selon l'invention peut ainsi être mise en œuvre pour une détection d'ADN d'OGM dans des semences, par exemple, ou pour la détection d'un polluant ou d'un pathogène dans de l'eau ou dans des aliments destinés à la consommation.

Les applications de l'utilisation selon l'invention peuvent ainsi s'étendre des domaines immunologiques à la génétique moléculaire ou à la détection d'ADN et d'ARN, comme illustré dans les figures 24 et 25 annexées. Il peut être utilisé pour marquer un ou plusieurs brins d'ARN d'un prélèvement biologique, avec un fragment partiellement complémentaire sonde lié à une nanoparticule, puis les détecter par hybridation sur des fragments complémentaires d'une autre région greffés sur un substrat solide, suivant une approche voisine des puces à ADN, de type Affymetrix. Un intérêt de l'invention réside alors dans l'absence d'étape d'amplification habituellement nécessaire pour ces approches.

Dans la suite du texte, on désignera plus simplement sous l'appellation « nanoparticule », la nanoparticule inorganique photoluminescente formée d'une matrice cristalline dopée par des ions de terres rares.

On emploie par ailleurs le terme « particule » pour désigner une particule comprenant au moins ladite nanoparticule, par exemple étant formée de ladite nanoparticule, à la surface de laquelle peuvent être localisés, selon une variante de réalisation, des cations tétraalkylammonium, et couplée par un ou plusieurs agents de ciblage de la substance à analyser, comme détaillé dans la suite du texte.

D'autres caractéristiques, variantes et avantages de l'utilisation selon l'invention ressortiront mieux à la lecture de la description, des exemples et des figures qui suivent, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comprenant un(e) » doit être comprise comme « comportant au moins un(e) ».

## PARTICULES LUMINESCENTES DE L'INVENTION

Comme indiqué précédemment, l'utilisation selon l'invention repose sur la détection de l'émission de luminescence de particules photoluminescentes comprenant, en particulier étant formées, d'une nanoparticule inorganique photoluminescente présentant des propriétés optiques et physico-chimiques spécifiques, et couplées à au moins un agent de ciblage de la substance à analyser.

### Nanoparticule inorganique photoluminescente

Les propriétés des nanoparticules mises en œuvre vont conditionner la sensibilité pouvant être atteinte par la

méthode de l'invention.

Les nanoparticules inorganiques de l'invention sont formées d'une matrice cristalline présentant au moins $10^3$ ions de terres rares.

Les ions de terre rares dans les nanoparticules de l'invention ne sont pas sous forme de complexes ou chélates d'ions de terres rares, formés d'ions de terres rares en combinaison avec des ligands organiques appropriés, comme par exemple décrits dans la publication Yuan *et al.* [21].

De préférence, les nanoparticules de l'invention comprennent entre 1000 et 6 000 000 ions de terres rares, en particulier entre 5 000 et 500 00 et plus particulièrement entre 20 000 et 100 0000 ions de terres rares.

Les nanoparticules de l'invention sont dopées par des ions de terres rares de même nature ou de natures différentes.

Les ions lanthanides sont choisis parmi l'europium (Eu), le dysprosium (Dy), le samarium (Sm), le praséodymium (Pr), le néodyme (Nd), l'erbium (Er), l'ytterbium (Yb), le cérium (Ce), l'holmium (Ho), le terbium (Tb), le thulium (Tm) et leurs mélanges.

En particulier, les ions lanthanides peuvent être choisis parmi Eu, Dy, Sm, Nd, Er, Yb, Tm et Tb et leurs mélanges, en particulier parmi Eu, Dy, Sm, Nd, Er, Yb et leurs mélanges, et plus particulièrement Eu.

La matrice cristalline formant la nanoparticule inorganique photoluminescente peut être une matrice d'oxyde, en particulier une matrice de vanadate ou de phosphate ; une matrice d'halogénure, en particulier une matrice de fluorure ; ou encore une matrice de chalcogénure, en particulier une matrice de tellure, sulfure ou séléniure.

Parmi les matrices de fluorure, on peut citer plus particulièrement les matrices de $YF_3$, $GdF_3$, $NaGdF_4$, $NaYF_4$, $NaLuF_4$, $Ba_4Lu_3F_{17}$, $Na_3ZrF_7$, $Na_3Zr_2F_{13}$ et $Na_7Zr_6F_{31}$.

les nanoparticules inorganiques de de l'utilisation selon l'invention sont de formule (I) suivante :

$$(A_{1-x}Ln_x)_a(M_pO_q) \qquad (I)$$

dans laquelle :

- M représente un ou plusieurs éléments choisis parmi V, P, W, Mo, As, Al, Hf, Zr, Ge, Ti, Sn, Mn et Si ;
- Ln correspond à un ou plusieurs ion(s) lanthanide(s) luminescent(s) choisis parmi l'europium (Eu), le dysprosium (Dy), le samarium (Sm), le praséodymium (Pr), le néodyme (Nd), l'erbium (Er), l'ytterbium (Yb), le cérium (Ce), l'holmium (Ho), le terbium (Tb), le thulium (Tm) et leurs mélanges.;
- A correspond à un ou plusieurs ion(s) constituant(s) de la matrice cristalline dont les niveaux électroniques ne sont pas impliqués dans le processus de luminescence et choisi parmi l'yttrium (Y), le gadolinium (Gd), le lanthane (La), le bismuth (Bi), le lutécium (Lu) et leurs mélanges,;
- $0 < x < 1$, en particulier $0,1 \leq x \leq 0,9$, en particulier $0,2 \leq x \leq 0,6$, notamment $0,2 \leq x \leq 0,4$ et plus particulièrement x vaut 0,4 ; et
- les valeurs de p, q et a sont telles que l'électroneutralité de $(A_{1-x}Ln_x)_a(M_pO_q)$ est respectée.

A peut être plus en particulier A peut être choisi parmi Y, Gd, La et leurs mélanges ; en particulier A représente Y ou Gd, de préférence A représente Y.

De préférence, M représente un ou plusieurs éléments choisis parmi V, P, Al, Hf, Zr, Ge, Ti, Sn, Mn et Si, et en particulier choisis parmi V, P, W, Mo, As et Al. La matrice cristalline des nanoparticules mises en œuvre selon l'invention peut incorporer un ou plusieurs types d'anions $M_pO_q$, En particulier, M peut représenter $V_{1-y}P_y$ avec y allant de 0 à 1.

Selon un mode de réalisation particulier, p dans la formule (I) précitée est différent de zéro.

A titre d'exemple, une nanoparticule de l'invention peut être de formule (I) dans laquelle M représente V et/ou P, p vaut 1, de sorte que la matrice de ladite nanoparticule comporte des anions $VO_4^{3-}$ et/ou $PO_4^{3-}$.

Dans un autre exemple de réalisation, M représente Al, A représente Y ou Lu, p vaut 5 et q vaut 12, de sorte que la matrice $A_a(M_pO_q)$ de ladite nanoparticule est le grenat $Y_3Al_5O_{12}$ (YAG) ou $Lu_3Al_5O_{12}$ (LuAG).

Selon un autre mode de réalisation particulier, une nanoparticule de l'invention peut être de formule (I) dans laquelle M représente Hf ou Zr, Ge, Ti, Sn, Mn, p vaut 2 et q vaut 7, de sorte que la matrice de ladite particule est $A_aHf_2O_7$, $A_aZr_2O_7$, $A_aGe_2O_7$, $A_aTi_2O_7$, $A_aSn_2O_7$ ou $A_aMn_2O_7$. En particulier, A peut représenter La, Y, Gd ou Lu, auquel cas a=2.

Dans un autre exemple de réalisation, p vaut zéro et A représente Y ou Gd, de sorte que la matrice $A_a(M_pO_q)$ de ladite nanoparticule est de type $Y_2O_3$ ou $Gd_2O_3$.

Selon un mode de réalisation particulier, les pour l'utilisation selon l'invention peuvent être plus particulièrement des nanoparticules d'oxyde métallique dopé par un ou plusieurs ions de terres rares, en particulier par un ou plusieurs ions lanthanides.

Le taux de substitution des ions de la matrice cristalline des nanoparticules de l'utilisation selon l'invention, en particulier de la matrice d'oxyde métallique, par des ions de terres rares peut être plus particulièrement compris entre 10 % et 90 %, en particulier entre 20 et 60 %, notamment entre 20 et 40 % et plus particulièrement être de 40 %.

Il était contre-intuitif de choisir des taux si élevés de dopage. En effet, d'une manière générale, les taux de dopage usuels des nanoparticules luminescentes dopées par des ions de terres rares sont maintenus à des valeurs inférieures à 10 % pour s'affranchir de l'effet de « quenching » survenant à de plus fortes concentrations [29] à [31].

De manière avantageuse, la cristallinité imparfaite des nanoparticules de l'utilisation selon l'invention, comme décrit plus précisément par la suite, permet de s'affranchir de l'effet de « quenching ». Par ailleurs, l'utilisation selon l'invention, en procédant à l'excitation directe des ions de terres rares des nanoparticules (et non uniquement de la matrice comme c'est le cas de manière classique), permet, en combinaison avec un dopage plus important en ions de terres rares, d'accéder à un nombre de photons d'excitation absorbés plus important, et dès lors à un nombre de photons émis et détectés plus important.

Les nanoparticules photoluminescentes de l'utilisation selon l'invention présentent une taille moyenne supérieure ou égale à 20 nm et strictement inférieure à 1 μm.

En particulier, elles présentent une taille moyenne comprise entre 20 nm et 500 nm, en particulier comprise entre 20 nm et 200 nm et notamment entre 20 nm et 100 nm.

En particulier, la taille moyenne des nanoparticules photoluminescentes de l'utilisation selon l'invention peut être supérieure ou égale à 25 nm, en particulier supérieure ou égale à 30 nm, notamment comprise entre 30 et 60 nm.

En particulier, comme illustré dans les exemples qui suivent, les nanoparticules photoluminescentes de l'utilisation selon l'invention peuvent présenter une taille moyenne de l'ordre de 30 à 50 nm.

Des nanoparticules de taille plus importante peuvent par exemple être obtenues par tri en taille par centrifugation des particules telles qu'exemplifiées, pour ne conserver, dans la distribution de tailles, que les particules de tailles les plus importantes, ou encore être obtenues par broyage du matériau massif. Toute autre technique connue par l'homme du métier peut être également utilisée.

Les nanoparticules photoluminescentes de l'utilisation selon l'invention présentent ainsi un volume suffisant pour contenir un grand nombre d'ions de terres rares, et donc émettre un signal luminescent suffisant pour permettre la détection de faibles concentrations. A titre d'exemple, une nanoparticule sphérique $Y_{0.6}Eu_{0,4}VO_4$ de diamètre de 30 nm contient 70 000 ions $Eu^{3+}$ (calcul du nombre d'ions selon la référence [35] Casanova *et al.* APL 2006). Par ailleurs, les nanoparticules photoluminescentes ne devraient pas être trop volumineuses pour éviter une gêne stérique lors de leur association avec la substance à doser immobilisée par exemple à la surface d'un support, comme décrit dans la suite du texte.

La taille moyenne peut être mesurée par microscopie électronique à transmission. Les images de microscopie électronique à transmission permettent de déterminer la forme des nanoparticules (sphériques, ellipsoïdes) et de déduire les dimensions moyennes des nanoparticules. Dans le cas de particules globalement sphériques, la taille moyenne s'entend du diamètre moyen des particules.

Dans le cas de particules de forme ellipsoïde, la taille moyenne s'entend de la taille moyenne d'une sphère de même volume que l'ellipsoïde. On suppose en général que le troisième axe de l'ellipsoïde, non visible dans les images de transmission qui sont des projections 2D, est de longueur égale à l'axe de plus petite taille.

Selon un mode de réalisation particulier, les nanoparticules de de l'utilisation selon l'invention sont de forme globalement ellipsoïdale allongée (« prolate » en langue anglaise).

Elles peuvent plus particulièrement présenter une longueur du grand axe, notée *a,* comprise entre 20 et 60 nm ; et une longueur du petit axe, notée *b,* comprise entre 10 et 30 nm. En particulier, les nanoparticules de l'utilisation selon l'invention peuvent présenter une valeur moyenne de longueur du grand axe, *a,* de 40 nm et une valeur moyenne de longueur du petit axe, b, de 20 nm.

De manière avantageuse, les nanoparticules de l'utilisation selon l'invention présentent une faible polydispersité. L'indice de polydispersité, qui peut être déduit des mesures MET, peut être en particulier strictement inférieur à 0,2.

Selon un mode de réalisation particulier, le produit entre le taux de dopage en ions de terres rares, par exemple en europium (Eu), et le rendement quantique de l'émission par la nanoparticule est maximisé.

Ceci est particulièrement avantageux pour une excitation, selon l'étape (i) de l'utilisation selon l'invention, des ions de terres rares par excitation directe, *i.e.* en résonance avec les états électroniques de ces ions, et non par excitation de la matrice $A_a(M_pO_q)$ telle que définie ci-dessus, par exemple $AVO_{4(1-y)}(PO_4)_y$, et transfert d'énergie ultérieur vers ces ions.

Cette optimisation du produit entre le taux de dopage x en ions Ln et le rendement quantique peut être effectuée en utilisant un fort dopage en ions Ln, par exemple entre 0,2 et 0,6, et notamment de 0,4, sans pour autant diminuer le rendement quantique, en particulier en limitant les processus de transfert entre ions de dopage conduisant à une extinction de concentration. En particulier, afin de maintenir un rendement quantique élevé, la nanoparticule présente une cristallinité imparfaite. En effet, une excellente cristallinité favorise les processus de transfert entres ions de dopage, en particulier lorsque ceux-ci sont à proximité immédiate entre eux, comme cela est le cas pour des dopages élevés et, par conséquent, favorise les processus de désexcitation des ions par des processus non radiatifs, liés à la surface et à la présence du solvant. En particulier, un procédé de synthèse à température ambiante, ou tout au moins à température n'excédant pas 600°C, est favorable pour la cristallinité imparfaite requise pour ces nanoparticules.

La cristallinité des nanoparticules est considérée comme étant « imparfaite » lorsque la longueur de cohérence déterminée par le diffractogramme des rayons X dans au moins une direction cristallographique donnée est inférieure à 80 % de la taille de la particule dans cette direction-là telle que mesurée à partir des images de microscopie électronique à transmission. Différents types de cristallinité imparfaite peuvent être considérés : polycristallinité, défauts, porosité, etc.

De manière avantageuse, les nanoparticules de l'utilisation selon l'invention sont aptes à émettre plus de $10^8$ photons avant l'arrêt de l'émission, en particulier plus de $10^9$, voire plus de $10^{10}$ photons. Dans de nombreux cas, en particulier dans le cas de particules $YVO_4$ ou $GdVO_4$ dopées à l'Eu, aucun arrêt de l'émission n'est constaté.

Dans une variante de réalisation particulièrement avantageuse de l'invention, la variation relative du signal de luminescence par rapport au signal attendu pour un comportement linéaire dans les conditions d'excitation utilisées dans le cadre du procédé de l'invention, est toujours inférieure à 30 %.

En particulier, les nanoparticules de l'utilisation selon l'invention sont telles qu'il n'y a pas de saturation du signal de luminescence pour de fortes intensités d'excitation (en particulier, supérieures à 1 W/cm$^2$). Ceci est plus particulièrement le résultat de la présence d'un grand nombre d'ions de terres rares (supérieur à $10^3$ ions de terres rares) au sein de la particule.

De manière avantageuse, l'utilisation selon l'invention n'est donc pas restreinte par les phénomènes de saturation et autres effets néfastes, tels que la photo-dégradation, qui peuvent intervenir à forte excitation avec certaines sondes photoluminescentes, comme les fluorophores organiques ou les protéines fluorescentes. L'absence de saturation et autres effets autorise ainsi avantageusement l'excitation selon le procédé de l'invention, à forte puissance (d'au moins 50 mW) et à forte densité d'excitation (au moins 1 W/cm$^2$).

Par ailleurs, de manière avantageuse, les nanoparticules de l'utilisation selon l'invention présentent une longue durée de vie d'émission. En particulier, elles peuvent présenter une durée de vie d'émission supérieure ou égale à 5 μs, en particulier supérieure ou égale à 10 μs, notamment supérieure ou égale à 20 μs, voire supérieure ou égale à 50 μs.

La durée de vie d'émission s'entend comme la durée de vie de l'état excité de la nanoparticule émettrice, et est déterminée en pratique par la durée de l'émission de photons de luminescence après arrêt de l'excitation, soit le temps caractéristique du déclin exponentiel de luminescence après arrêt de l'excitation.

L'utilisation selon l'invention permet avantageusement de tirer profit de la durée d'émission longue des particules de l'invention (quelques centaines de μs dans le cas des particules $Y_{1-x}Eu_xVO_4$, en comparaison aux durées de vie des fluorophores usuels de l'ordre de la nanoseconde), pour effectuer une détection résolue en temps, en particulier une détection retardée de l'émission, avec une résolution temporelle suffisante (10 μs), en utilisant du matériel peu sophistiqué et peu onéreux, en particulier un hacheur mécanique, un photomultiplicateur classique et un convertisseur A-D de 100 kHz, comme décrit dans la suite du texte.

De manière avantageuse, les nanoparticules de l'utilisation selon l'invention présentent une bonne stabilité colloïdale en solution.

La stabilité des nanoparticules en solution est particulièrement déterminante pour répondre aux exigences en termes de reproductibilité des résultats de détection à partir de l'utilisation de ces particules à titre de sondes.

Le « potentiel zêta » est un des éléments représentatifs de la stabilité d'une suspension. Il peut être par exemple directement mesuré à l'aide d'un équipement de type Zetasizer Nano ZS de la société Malvern. Cet équipement mesure à l'aide de dispositifs optiques les vitesses de déplacement des particules en fonction du champ électrique qui leur est appliqué.

En particulier, les nanoparticules présentent avantageusement, à l'issue de leur synthèse, un potentiel zêta, en valeur absolue, notée $|\zeta|$, en milieu aqueux à pH de 5, supérieur à 30 mV.

Plus particulièrement, les nanoparticules présentent avantageusement un potentiel zêta inférieur ou égal à -28 mV, de préférence inférieur ou égal à -30 mV, dans un milieu aqueux de pH $\geq$ 5, en particulier de pH $\geq$ 5,5, et plus particulièrement de pH $\geq$ 6, et de conductivité ionique strictement inférieure à 100 μS.cm$^{-1}$.

En particulier, les nanoparticules possèdent un potentiel zêta inférieur ou égal à -30 mV, dans un milieu aqueux de pH $\geq$ 6,5, en particulier de pH $\geq$ 7, notamment de pH $\geq$ 8, et de conductivité ionique strictement inférieure à 100 μS.cm$^{-1}$.

Le « potentiel zêta », noté $\zeta$, peut être défini comme la différence de potentiel existant entre le sein de la solution, et le plan de cisaillement de la particule. Il est représentatif de la stabilité d'une suspension. Le plan de cisaillement (ou rayon hydrodynamique) correspond à une sphère imaginaire autour de la particule dans laquelle le solvant bouge avec la particule lorsque les particules se déplacent dans la solution. Le potentiel zêta peut être déterminé par des méthodes connues de l'homme du métier, par exemple par déplacement de la particule avec sa couche de solubilisation dans un champ électrique, comme détaillé dans la suite du texte.

Ce potentiel zêta négatif des nanoparticules inférieur ou égal à -28 mV, en particulier inférieur ou égal à -30 mV, en milieu aqueux à pH $\geq$ 5, en particulier à pH $\geq$ 6,5, accroît les phénomènes de répulsion électrostatique des nanoparticules en solution aqueuse les unes par rapport aux autres, ce qui permet ainsi de supprimer les phénomènes de floculation. Il est en effet connu de façon empirique de l'homme du métier qu'un potentiel zêta de valeur absolue élevée, en particulier supérieure à 28 mV permet en général de supprimer les effets de floculation dans des milieux à faible force ionique.

Selon une variante de réalisation, une nanoparticule mise en œuvre dansl'utilisation selon l'invention est une nanoparticule de formule (II) suivante :

$$A_{1-x}Ln_x\,(VO_4)_{(1-y)}(PO_4)_y \qquad (II)$$

dans laquelle :

- A est choisi parmi l'yttrium (Y), le gadolinium (Gd), le lanthane (La), le lutécium (Lu), et leurs mélanges, en particulier A est choisi parmi Y, Gd, La et leurs mélanges et plus particulièrement A représente Y ;
- Ln est choisi parmi l'europium (Eu), le dysprosium (Dy), le samarium (Sm), le néodyme (Nd), l'erbium (Er), l'ytterbium (Yb), le thulium (Tm), le terbium (Tb), et leurs mélanges, de préférence Ln représente Eu ;
- $0 < x < 1$, en particulier $0{,}2 \leq x \leq 0{,}6$ et plus particulièrement x vaut 0,4 ; et
- $0 \leq y < 1$, en particulier y vaut 0.

Selon un mode de réalisation particulier, la nanoparticule répond à la formule (II) précitée dans laquelle y vaut 0. Autrement dit, la nanoparticule mise en œuvre dans le procédé de l'invention est de formule $A_{1-x}Ln_xVO_4$ (III), dans laquelle A, Ln et x sont tels que définis précédemment.
Selon un mode de réalisation particulier, A dans la formule (II) ou (III) précitée représente l'yttrium (Y).
Selon un autre mode de réalisation particulier, Ln dans la formule (II) ou (III) précitée représente Eu.
Ainsi, selon une variante de réalisation, les particules de l'invention comprennent une nanoparticule de formule $Y_{1-x}Eu_xVO_4$ (IV) dans laquelle $0 < x < 1$, en particulier $0{,}2 \leq x \leq 0{,}6$ et plus particulièrement x vaut 0,4.
De préférence, les nanoparticules de formule (II), (III) ou (IV) mises en œuvre selon l'invention présentent en surface des cations tétraalkylammonium.
De manière avantageuse, la mise en œuvre de ces contre-ions volumineux assure une stabilité colloïdale améliorée des particules en solution, et prévient notamment les phénomènes de floculation des particules. Sans vouloir être lié par la théorie, cette amélioration en termes de stabilisation, résultant de la mise en œuvre de contre-ions tétraalkylammonium, comparativement par exemple à la mise en œuvre d'ions sodium, est liée à la différence du potentiel zêta des particules. De fait, la mise en œuvre des contre-ions tétraalkylammonium induit un potentiel zêta négatif de la particule de valeur absolue accrue, comparativement par exemple au cas de la mise en œuvre de contre-ions sodium.
A noter que cette stabilité est importante même dans des milieux de force ionique élevée comme c'est le cas dans le milieu de synthèse même avant purification (force ionique supérieure à 0,1 M). En particulier, une suspension aqueuse de particules selon l'invention présente peu, voire pas, de phénomène de floculation sur des durées de temps de plusieurs semaines.
L'immobilisation des cations de type tétraalkylammonium à la surface des nanoparticules de l'invention résulte plus particulièrement d'interactions électrostatiques entre les ions de surface de la nanoparticule de formule (II) chargés négativement ($O^{2-}$) et les contre-ions de type tétraalkylammonium chargés positivement.
Ainsi, les cations tétraalkylammonium sont associés directement à la nanoparticule *via* des interactions électrostatiques avec les ions de surface chargés négativement de la nanoparticule. En particulier, l'immobilisation des cations tétraalkylammonium à la surface de la nanoparticule ne met en œuvre aucun groupe « espaceur ».
Par cations « tétraalkylammonium », on entend plus particulièrement désigner des cations tétra($C_1$-$C_6$) alkylammonium, c'est-à-dire des cations de formule $NR_4^+$ avec R, identiques ou différents, représentant un groupe $C_1$-$C_6$-alkyl, en particulier $C_1$-$C_4$-alkyl.
De préférence, les cations « tétraalkylammonium » sont des cations tétra($C_1$-$C_3$)alkylammonium, c'est-à-dire des cations de formule $NR_4^+$ avec R, identiques ou différents, représentant un groupe $C_1$-$C_3$-alkyl.
Par « $C_1$-$C_6$-alkyl », on entend un groupe aliphatique saturé, linéaire ou ramifié, comprenant de 1 à 6 atomes de carbone. A titre d'exemples, on peut citer les groupes méthyle, éthyle, propyle, butyle, isopropyle, isobutyle, tertbutyle, etc.
Les cations tétraalkylammonium sont de préférence choisis parmi les cations tétraméthylammonium, tétraéthylammonium, tétrapropylammonium, tétrabutylammonium et leurs mélanges.
Selon un mode de réalisation particulier, les cations présents à la surface des nanoparticules de l'invention sont des cations tétraméthylammonium.
Comme indiqué précédemment, les cations tétraalkylammonium sont présents en surface des nanoparticules de l'invention en une quantité suffisante pour procurer le résultat souhaité en termes de stabilité colloïdale de la suspension aqueuse desdites particules synthétisées, autrement dit la valeur du potentiel zêta souhaitée.
Les cations tétraalkylammonium, présents à la surface des nanoparticules de l'invention, peuvent être présents à raison de 100 à 10000 cations tétraalkylammonium par nanoparticule.
Les nanoparticules de l'invention peuvent ainsi être des nanoparticules de formule (II') suivante :

$$A_{1-x}Ln_x\,(VO_4)_{(1-y)}(PO_4)_y \cdot (NR_4^+)_z \qquad (II')$$

dans laquelle :

- A est choisi parmi Y, Gd, La, Lu et leurs mélanges, en particulier A est choisi parmi Y, Gd, La et leurs mélanges, de préférence A représente Y ;
- Ln est choisi parmi Eu, Dy, Sm, Nd, Er, Yb, Tm, Tb et leurs mélanges, de préférence Ln représente Eu ;
- $0<x<1$, en particulier $0,2 \leq x \leq 0,6$ et plus particulièrement x vaut 0,4 ;
- $0 \leq y<1$ ;
- R, identiques ou différents, sont tels que définis précédemment, de préférence représentent un groupe $C_1$-$C_3$-alkyl, en particulier méthyle ; et
- z représente le nombre de cations tétraalkylammonium $NR_4^+$ localisés à la surface de ladite nanoparticule, en particulier z est compris entre 100 et 10000.

Selon un mode de réalisation particulier, la nanoparticule répond à la formule (II') précitée dans laquelle y vaut 0. Autrement dit, une nanoparticule de l'invention peut répondre plus particulièrement à la formule $A_{1-x}Ln_xVO_4 . (NR_4^+)_z$ (III'), dans laquelle A, x, Ln, R et z sont tels que définis précédemment.

Selon un mode de réalisation particulier, A dans la formule (II') ou (III') précitée représente l'yttrium (Y).

Selon un autre mode de réalisation particulier, Ln dans la formule (II') ou (III') précitée représente Eu.

Ainsi, selon une variante de réalisation particulière, une nanoparticule de l'invention peut répondre plus particulièrement à la formule $Y_{1-x}Eu_xVO_4 . (NR_4^+)_z$ (IV'), dans laquelle x, R et z sont tels que définis précédemment.

Il est entendu que les différents modes de réalisation précités, notamment en ce qui concerne la nature de la nanoparticule photoluminescente et des cations tétraalkylammonium de surface, peuvent être combinés.

A titre d'exemple, une particule photoluminescente mise en œuvre selon l'invention peut comprendre une nanoparticule de formule $Y_{0,6}Eu_{0,4}VO_4$ à la surface de laquelle sont éventuellement immobilisés des cations tétraméthylammonium.

En particulier, elle peut répondre à la formule $Y_{0,6}Eu_{0,4}VO_4 \cdot (NR_4^+)_z$, z représentant le nombre de cations tétraalkylammonium.

Les nanoparticules, en particulier les nanoparticules de formule (II) précitée, sont de nature majoritairement cristalline et polycristalline, en particulier de taille moyenne de cristallites, déduite par diffraction de rayons X, comme détaillé en exemple 1 qui suit, comprise entre 3 et 40 nm.

*Préparation des nanoparticules*

Les nanoparticules à matrice cristalline dopée par des ions de terres rares mises en œuvre dans l'utilisation selon l'invention peuvent être préparées par toute méthode conventionnelle connue de l'homme du métier.

De manière avantageuse, les nanoparticules de l'invention sont synthétisées de manière aisée, dans un milieu aqueux, ce qui présente l'avantage de s'affranchir de toute étape ultérieure de transfert de solvant.

En particulier, les nanoparticules de formule $A_{1-x}Ln_x (VO_4)_{(1-y)}(PO_4)_y$ (II) peuvent être formées par réaction de co-précipitation, en milieu aqueux, à partir de précurseurs desdits éléments A et Ln, et en présence d'ions orthovanadate ($VO_4^{3-}$) et éventuellement d'ions phosphate ($PO_4^{3-}$).

Les précurseurs des éléments A et Ln peuvent se présenter, de manière classique, sous la forme de sels desdits éléments, par exemple de nitrates, chlorures, perchlorates ou acétates, en particulier de nitrates. Les précurseurs des éléments A et Ln, et leur quantité, sont bien entendu choisis de manière adéquate au regard de la nature de la nanoparticule souhaitée.

Par exemple, la synthèse de nanoparticules de formule $Y_{1-x}Eu_xVO_4$ (IV) peut mettre en œuvre, à titre de composés précurseurs de l'yttrium et de l'europium, les nitrates d'yttrium ($Y(NO_3)_3$) et d'europium ($Eu(NO_3)_3$).

De manière avantageuse, la réaction de co-précipitation est réalisée en présence d'une quantité efficace de cations tétraalkylammonium.

Les cations tétraalkylammonium sont plus particulièrement tels que définis précédemment. Ils sont de préférence choisis parmi les cations tétraméthylammonium, tétraéthylammonium, tétrapropylammonium, tétrabutylammonium et leurs mélanges. Il s'agit de préférence de cations tétraméthylammonium.

Par « quantité efficace », on entend plus particulièrement signifier que les cations sont mis en œuvre en une quantité suffisante pour procurer le résultat souhaité en termes de stabilité colloïdale de la suspension aqueuse desdites particules synthétisées.

En particulier, les cations tétraalkylammonium sont mis en œuvre en une quantité telle que les nanoparticules obtenues à l'issue du procédé de synthèse de l'invention présentent un potentiel zêta tel qu'indiqué précédemment. Comme illustré en exemple 1 qui suit, les cations tétraalkylammonium, dans la réaction de co-précipitation selon l'invention, sont des contre-ions des ions orthovanadate ($VO_4^{3-}$), et éventuellement phosphate ($PO_4^{3-}$) (dans le cas

où $y \neq 0$).

Selon un mode de réalisation particulièrement préféré, les ions orthovanadate ($VO_4^{3-}$) sont générés *in situ* à partir d'un sel de métavanadate, de préférence du métavanadate d'ammonium ($NH_4VO_3$).

Les ions orthovanadate peuvent être plus particulièrement formés *in situ* par réaction dudit sel de métavanadate avec une base (plus précisément avec deux équivalents de base forte).

En particulier, la base mise en œuvre pour la formation *in situ* des ions orthovanadate à partir du sel de métavanadate est une source de cations tétraalkylammonium. Il peut s'agir par exemple d'un hydroxyde de tétraalkylammonium, par exemple de l'hydroxyde de tétraméthylammonium.

La mise en œuvre d'un sel de métavanadate tel que par exemple le métavanadate d'ammonium pour la synthèse des nanoparticules selon l'invention s'avère particulièrement avantageuse, comparativement à la mise en œuvre de l'orthovanadate de sodium, en ce qu'elle permet de s'affranchir des problèmes de reproductibilité liés aux variations de la teneur en carbonate de l'orthovanadate de sodium.

A titre d'exemple, la réaction de synthèse à partir du métavanadate d'ammonium et de l'hydroxyde de tétraméthylammonium est représentée en exemple 1.

Il peut être également envisagé d'utiliser du métavanadate d'alkylammonium (non disponible commercialement) et une autre base forte, comme l'hydroxyde d'ammonium.

Le procédé de préparation des particules peut plus particulièrement mettre en œuvre au moins les étapes suivantes, consistant en :

(i) disposer d'une solution aqueuse, notée solution (1), comprenant des ions orthovanadate ($VO_4^{3-}$), et éventuellement des ions phosphates ($PO_4^{3-}$), et des cations tétraalkylammonium ;
(ii) ajouter à la solution aqueuse (1), une solution aqueuse, dite solution (2), comprenant lesdits précurseurs des éléments A et Ln, en particulier sous forme de sels, notamment de nitrates, dans des conditions propices à la formation par co-précipitation des nanoparticules de formule (II) ; et
(iii) récupérer lesdites nanoparticules de formule (II) à la surface desquelles sont localisés des cations tétraalkylammonium, formées à l'issue de l'étape (ii).

La solution aqueuse (1) peut être plus particulièrement préparée par mélange d'au moins un sel de métavanadate, en particulier du métavanate d'ammonium et d'au moins une base, source de cations tétraalkylammonium, par exemple d'un hydroxyde de tétraalkylammonium.

Dans le cas des ions phosphates, on ajoute un sel de phosphate, tel que le phosphate de sodium ou le phosphate d'ammonium.

Ainsi, selon une variante de réalisation particulièrement avantageuse, le procédé de l'invention comprend au moins les étapes consistant en :

(i) préparer une solution aqueuse (1) par mélange, en milieu aqueux, d'un sel de métavanadate, en particulier du métavanadate d'ammonium ($NH_4VO_3$), et éventuellement un sel de phosphate, et d'une base, source de cations tétraalkylammonium, en particulier un hydroxyde de tétraalkylammonium ;
(ii) ajouter à la solution aqueuse (1), une solution aqueuse (2) comprenant lesdits précurseurs des éléments A et Ln, en particulier sous forme de sels, notamment de nitrates ; dans des conditions propices à la formation par co-précipitation desdites nanoparticules de formule (II) ; et
(iii) récupérer les nanoparticules de formule (II) à la surface desquelles sont localisés des cations tétraalkylammonium, formées à l'issue de l'étape (ii).

Selon un mode de réalisation particulier, l'ajout en étape (ii) de la solution (2) à la solution (1) est opéré goutte à goutte.

Selon une autre variante de réalisation, la solution (2) peut être ajoutée à la solution (1) en une seule fois, et non pas au goutte à goutte.

Le milieu aqueux des solutions (1) et (2) est plus particulièrement formé d'eau.

Dans un mode de réalisation particulier, la solution aqueuse (2) contenant les précurseurs des éléments A et Ln peut également comprendre des complexants de ces éléments, tel que le citrate, par exemple le citrate de tétraalkylammonium.

Il appartient à l'homme du métier d'ajuster de manière adéquate les quantités des différents réactifs, en particulier des précurseurs des ions orthovanadate, éventuellement phosphate, et desdits éléments A et Ln, au regard de la nature souhaitée pour la nanoparticule de formule (II) selon l'invention.

En particulier, les proportions stœchiométriques des différents réactifs selon les formules (II), (II'), (III), (III'), (IV) et (IV') sont à respecter. Lorsque l'on prévoit une excitation optique des ions Ln par excitation directe, i.e. en résonance avec les états électroniques de ces ions, comme c'est le cas dans la méthode ultra-sensible selon l'invention, et non par

excitation de la matrice $AVO_{4(1-y)}(PO_4)_y$ et transfert d'énergie ultérieur vers ces ions, comme évoqué précédemment, il est préférable de disposer d'une valeur élevée de x, de préférence entre 0,2 et 0,6, et de préférence 0,4.

De manière avantageuse, le procédé de préparation des nanoparticules ne requiert aucun chauffage de la solution, à la différence notamment des méthodes hydrothermales proposées dans les publications [26] à [29]. En particulier, l'ensemble des étapes (i) à (iii) pour la synthèse des particules selon l'invention peut être avantageusement opéré à température ambiante (20-25°C).

Néanmoins, des procédés de préparation à plus haute température, par exemple de type hydrothermal, peuvent produire des nanoparticules de cristallinité similaire, à condition que la température n'excède pas 600°C, ce qui permet ainsi d'accéder à des nanoparticules présentant une cristallinité imparfaite comme évoqué précédemment, et ainsi obtenir un rendement quantique élevé.

L'étape (iii) consiste plus particulièrement à purifier la solution de particules obtenues, notamment pour éliminer l'excès de contre-ions.

Les étapes de purification peuvent plus particulièrement comprendre des étapes de dialyse ou de centrifugation et redispersion des particules en milieu aqueux, par exemple par sonification.

Les particules peuvent être redispersées dans un milieu aqueux, en particulier dans l'eau. Comme évoqué précédemment, la suspension colloïdale aqueuse des particules de l'invention présente une très bonne stabilité, même après stockage pendant plusieurs mois.

A titre d'exemple, les particules formées en tout ou partie d'une nanoparticule de formule $Y_{1-x}Eu_xVO_4$ (IV), avec $0<x<1$, peuvent être préparées *via* au moins les étapes consistant en :

(i) disposer d'une solution aqueuse (1), comprenant des ions orthovanadate et des cations tétraalkylammonium, ladite solution aqueuse (1) étant de préférence obtenue à partir du mélange, en milieu aqueux, du métavanadate d'ammonium ($NH_4VO_3$) et d'un hydroxyde de tétraalkylammonium ;

(ii) ajouter à la solution aqueuse (1), une solution aqueuse, dite solution (2), comprenant des précurseurs de Y et Eu, en particulier des nitrates d'yttrium et d'europium, dans des conditions propices à la formation par co-précipitation des nanoparticules de formule (IV) ; et

(iii) récupérer lesdites nanoparticules de formule (IV) à la surface desquelles sont localisés des cations tétraalkylammonium, formées à l'issue de l'étape (ii).

Comme indiqué précédemment, à l'issue de leur synthèse, les nanoparticules présentent une faible polydispersité. L'indice de polydispersité, qui peut être déduit des mesures MET, peut être en particulier strictement inférieur à 0,2.

La synthèse de nanoparticules luminescentes selon l'invention, en particulier de tailles plus importantes, supérieures à quelques dizaines de nanomètres, peut être opérée par toute autre approche connue de l'homme du métier, par exemple par broyage du matériau massif.

## Agent de ciblage

Les particules mises en œuvre à titre de sondes luminescentes selon l'utilisation de l'invention sont couplées (ou greffées) à au moins un agent de ciblage de la substance à doser dans l'échantillon à analyser.

Par « agent de ciblage », on entend un composé permettant une liaison avec une substance d'intérêt biologique ou chimique, et dont l'identification est recherchée.

La nature des agents de ciblage mis en œuvre est bien entendu choisie au regard de la substance à analyser dans l'échantillon.

Les particules mises en œuvre dans l'utilisation selon l'invention sont parfaitement adaptées à une grande diversité de ciblages biologiques, les spécificités étant dépendantes de la nature du ou des agents de ciblage greffés à la surface de la nanoparticule.

L'agent de ciblage peut être plus particulièrement choisi parmi un anticorps polyclonal ou monoclonal, un fragment d'anticorps, un nanobody, un oligonucléide, un peptide, une hormone, un ligand, une cytokine, un peptidomimétique, une protéine, un carbohydrate, une protéine modifiée chimiquement, un acide nucléique modifié chimiquement, un carbohydrate modifié chimiquement qui cible une protéine de surface cellulaire connue, un aptamère, un assemblage de protéines et d'ADN/ARN ou un chloroalcane utilisé par les marquages de type HaloTag. Une approche de type SNAP-Tag ou CLIP-Tag peut également être utilisée.

Selon un mode de réalisation particulier, il s'agit d'un anticorps ou fragment d'anticorps.

Des fragments d'anticorps appropriés comprennent au moins un domaine variable d'une immunoglobuline, tels que des domaines variables simples Fv, scFv, Fab, $(Fab')^2$ et autres fragments protéolytiques ou des « nanobody » (anticorps à domaine unique tels que les fragments $V_HH$ obtenus à partir d'anticorps de camélidés ou $V_{NAR}$ obtenus à partir d'anticorps de poissons cartilagineux).

Le terme « anticorps » selon l'invention inclut des anticorps chimériques ; des anticorps humains ou humanisés,

des anticorps recombinants et modifiés, des anticorps conjugués, et leurs fragments.

Selon un mode de réalisation particulier, les anticorps ou fragments d'anticorps mis en œuvre selon l'invention ciblent des marqueurs spécifiques de cellules cancéreuses.

L'agent de ciblage peut également être dérivé d'une molécule connue pour lier un récepteur de surface cellulaire. Par exemple, le fragment de ciblage peut dériver de lipoprotéines de faible densité, transferrine, EGF, insuline, PDGF, enzymes fibrinolytiques, anti-HER2, anti-HER3, anti-HER4, annexines, interleukines, interférons, érythropoïétines ou facteurs stimulant les colonies.

*Couplage de la particule avec l'agent de ciblage*

Il appartient à l'homme du métier de mettre en œuvre les méthodes de couplage/greffage adaptées pour préparer de manière adéquate les particules couplées à un ou plusieurs agents de ciblage. La quantité d'agent(s) de ciblage mise en œuvre est ajustée au regard de la quantité de particules.

L'agent de ciblage peut être greffé directement, ou *via* un espaceur (encore désigné sous les termes « linker » ou « spaceur »), à la nanoparticule.

Les méthodes de couplage (encore appelé greffage) des particules à des biomolécules sont bien connues de l'homme de l'art. Il s'agit en général d'un couplage par liaison covalente, par complexation de surface, par interactions électrostatiques, par encapsulation, ou par adsorption.

Dans certains cas, dont le cas d'un couplage par liaison covalente, les particules peuvent être préalablement fonctionnalisées par des groupements chimiques capables ensuite de réagir avec un autre groupement chimique porté par l'agent de ciblage pour former une liaison covalente.

Comme exemples de groupements chimiques pouvant être présents à la surface des nanoparticules, on peut citer les groupements carboxyle, amino, thiol, aldéhyde et époxy.

Des groupements amino peuvent être apportés par des molécules comme les organosilane aminés, tel que l'aminotriéthoxysilane (APTES). L'avantage de l'APTES réside dans le fait qu'il forme *via* des liaisons covalentes une capsule autour de la nanoparticule. Les amines apportées par l'APTES sont ainsi très stables dans le temps. Les groupements amino peuvent être transformés en groupements carboxyle par réaction avec de l'anhydride succinique.

Des groupements carboxyle peuvent être apportés par des molécules telles que l'acide citrique ou un polyacide acrylique (PAA).

Par exemple, les nanoparticules de l'invention peuvent être fonctionnalisées en surface avec du citrate, comme illustré dans l'exemple 1 qui suit.

Selon un autre mode de réalisation particulier, les nanoparticules de l'invention peuvent être fonctionnalisées en surface par du polyacide acrylique (PAA). Dans le cas d'une fonctionnalisation avec du PAA, le nombre de liaisons de coordination (liaisons datives) formées par chaque molécule de PAA est d'autant plus important que le PAA est long. Le PAA peut par exemple présenter un degré de polymérisation allant de 3 à 10000.

De manière avantageuse, la fonctionnalisation des nanoparticules avec du PAA conduit à des nanoparticules fonctionnalisées, et à des nanoparticules résultant du couplage de ces nanoparticules fonctionnalisées avec un ou plusieurs agents de ciblage, qui présentent d'excellentes propriétés en termes de stabilité dans le temps.

Dans d'autres cas, les particules peuvent être préalablement couplées à des molécules aptes à permettre un couplage ultérieur avec un agent de ciblage.

Par exemple, les particules peuvent être couplées à la streptavidine apte à permettre un couplage avec un agent de ciblage biotinylé.

A titre d'exemple, l'exemple 1 illustre le couplage de nanoparticules avec des anticorps biotinylés par couplage des nanoparticules couplées avec la streptavidine avec des anticorps biotinylés. Il peut être également réalisé directement par couplage des anticorps sur des nanoparticules fonctionnalisées avec du citrate ou du polyacide acrylique.

Dans d'autres cas, le couplage des nanoparticules avec des anticorps peut être réalisé directement par couplage des anticorps sur des nanoparticules fonctionnalisées avec de l'APTES. Les groupements amino apportés par l'APTES peuvent être transformés dans un premier temps en groupements carboxyle par réaction avec de l'anhydride succinique, comme cité ci-dessus. Puis, les groupements carboxyle peuvent être activés selon toute technique connue de l'homme de l'art, en particulier par réaction avec le 1-éthyl-3-(3-diméthylaminopropyl)carbodiimide (EDC) et le N-hydroxysuccinimide (NHS), pour réagir ensuite avec les fonctions amines à la surface d'un polypeptide et former une liaison amide covalente, lorsque l'agent de ciblage est une protéine ou un anticorps.

La fonctionnalisation des nanoparticules par de l'APTES peut se faire avantageusement suite au recouvrement des nanoparticules par un couche de silice.

Le couplage de l'agent de ciblage sur la surface des nanoparticules peut également se faire par toute autre méthode connue de l'homme du métier.

Il peut également se faire avantageusement par recouvrement des nanoparticules d'une couche de silice, suivi d'une réaction de recouvrement par de l'APTES (3-aminopropyltriéthoxysilane), dont les fonctions amines servent à

réagir avec un agent réticulant bifonctionnel comportant deux fonctions NHS. Par la suite les nanoparticules couplées aux agents réticulants peuvent réagir avec les fonctions amines à la surface d'une protéine (anticorps, streptavidine, etc.). Ce type de procédé de couplage est notamment décrit dans les références [32] et [33].

De manière avantageuse, les particules mises en œuvre dans l'utilisation selon l'invention présentent une faible polydispersité. Il est préférable que l'indice de polydispersité, qui peut être déduit des mesures MET ou des mesures de diffusion dynamique de la lumière (DDL), soit strictement inférieur à 0,2. Lorsque cela n'est pas le cas à l'issue de la synthèse ou de la fonctionnalisation des particules, une polydispersité plus faible peut être obtenue par tri en taille par centrifugation ou par toute autre technique connue par l'homme du métier.

## ASSOCIATION DES SONDES LUMINESCENTES AVEC LA SUBSTANCE À ANALYSER

Dans l'utilisation selon l'invention, les particules photoluminescentes sont associées à la substance de l'échantillon à analyser.

Cette étape peut être opérée, de manière similaire aux méthodes conventionnelles, par exemple à la méthode immuno-enzymatique ELISA, à la surface d'un support, comme représenté schématiquement en Figure 1.

Cette variante de réalisation sera plus particulièrement exposée dans la suite du texte.

Elle implique notamment l'immobilisation préalable, comme décrit en exemple 2, de la substance de l'échantillon à analyser, à la surface d'un support.

En particulier, elle peut comprendre les étapes suivantes :

(a) disposer d'un support dont la surface est préalablement passivée et fonctionnalisée avec un agent de ciblage de la substance à détecter/quantifier, par exemple un premier anticorps monoclonal, dit anticorps de capture ;

(b) mettre en contact ledit échantillon à analyser avec le support de l'étape (a) dans des conditions propices à l'association de ladite substance avec l'agent de ciblage ; et

(c) mettre en contact les particules photoluminescentes couplées à au moins un agent de ciblage avec ledit support issu de l'étape (b) pour associer les particules avec ladite substance immobilisée à la surface du support.

Le support peut être de différentes natures. Il peut s'agir de lamelles, par exemple de lamelles de verre comme celles mises en œuvre dans les exemples, de plaques multipuits, microplaques, gels membranes, bandelettes ou microcanaux. Il peut s'agir également d'un plastique de bonne qualité optique ou de tout autre matériau de qualité optique suffisante.

La surface du support est préalablement passivée de manière à ce que les particules luminescentes ne s'y fixent pas en l'absence de la substance à analyser.

La passivation de surface peut être opérée par toute méthode connue de l'homme du métier.

Il peut s'agir par exemple d'une passivation de la surface en verre à l'aide d'une molécule comprenant un polyéthylène glycol (PEG), par exemple à l'aide de molécules silane-PEG. De préférence, le PEG peut présenter une masse molaire de 3000 à 20000 $g.mol^{-1}$. Plus le PEG sera long, plus la passivation résultante sera meilleure. Cependant, le PEG ne devrait avantageusement pas être trop long afin de ne pas présenter un encombrement stérique pour la fixation de la substance à analyser.

Le support est en outre fonctionnalisé en surface par un premier agent de ciblage de la substance à détecter/quantifier. Il peut s'agir plus particulièrement d'un anticorps, dit anticorps de capture, comme représenté en phase 1 de la Figure 1, notamment lorsque la substance à analyser est de type biomarqueur, protéine ou polypeptide.

Lorsque la substance à analyser est de type anticorps, l'agent de ciblage peut être de type antigène spécifique de l'anticorps à détecter.

La fonctionnalisation de surface peut être opérée par toute méthode connue de l'homme de métier. Elle peut par exemple être opérée par impression par « spotter » (dépôt de microgouttes de solutions contenant les molécules de ciblage sur la surface à l'aide d'un robot), par une technique d'impression par contact (« contact printing » en langue anglaise) permettant de transférer des molécules par contact entre les motifs topologiques d'un tampon (par exemple un tampon en polydiméthylsiloxane PDMS) et la surface du substrat, ou par d'autres moyens connus de l'homme du métier permettant le dépôt des agents de ciblage en surface du support.

L'échantillon à analyser est ensuite mis en contact avec la surface fonctionnalisée dudit support de manière à permettre l'association de la substance à détecter/doser avec l'agent de ciblage porté par le support (phases 2 et 3 de la Figure 1).

Cette étape implique, à l'instar par exemple d'un test classique d'ELISA, une étape d'incubation de l'échantillon à la surface du support, et de lavage/rinçage du support afin de retirer la solution et les molécules non-liées. Après rinçage, seuls les complexes agent de ciblage/substance à doser, par exemple anticorps/antigène demeurent attachés à la surface du support.

Enfin, les particules photoluminescentes, telles que décrites précédemment, formées en tout ou partie d'une

nanoparticule photoluminescente et couplées à un agent de ciblage de la substance à analyser, par exemple un anticorps, sont couplées avec la substance à analyser immobilisée à la surface du support (phase 4 de la Figure 1).

Cette étape implique de laisser incuber la solution de particules photoluminescentes sur la surface du support fonctionnalisée et ensuite de laver/rincer le support afin de retirer les particules non liées au support. Après rinçage, seuls les complexes agent de ciblage/substance à doser/particules couplées à au moins un agent de ciblage, par exemple anticorps monoclonal/antigène/anticorps polyclonal-nanoparticule demeurent attachés à la surface du support. Le temps d'incubation peut être ajusté par des essais préalables de manière à maximiser le signal l'émission de luminescence, comme illustré dans l'exemple 4. D'une manière générale, il peut être compris entre 45 minutes et 2 heures.

Le couplage des particules couplées à un agent de ciblage avec la substance à analyser peut par exemple impliquer une reconnaissance d'un couple ligand/anti-ligand, par exemple biotine ou composés biotinylés/avidine ou streptavidine, haptène/anticorps, antigène/anticorps, peptide/anticorps, tel que digoxygénine (DIG)/anticorps anti-DIG, sucre/lectine, polynucléotide/complémentaire de polynucléotide, etc. étant entendu que l'un des éléments de ces couples constitue la substance à analyser, ou peut également être couplé à la substance à analyser.

Il est entendu que la substance à analyser peut être immobilisée en plusieurs zones prédéfinies et distinctes de la surface du support.

Ceci peut être notamment réalisé en mettant en œuvre une fonctionnalisation localisée de la surface du support par ledit agent de ciblage (par exemple, anticorps de capture), par exemple par impression par « spotter » (dépôt de microgouttes de solutions contenant les molécules de ciblage sur la surface du support à l'aide d'un robot) ou par une technique d'impression par contact (« contact printing » en langue anglaise) permettant une fonctionnalisation de la surface du substrat suivant certains motifs, par exemple définis par le tampon utilisé pour transférer les molécules, comme illustré en exemple 2. Il peut s'agir d'un dépôt en plusieurs zones prédéfinies du même agent de ciblage. Dans ce cas, ces zones multiples servent à détecter la même substance dans plusieurs échantillons différents.

Une telle mise en œuvre est plus particulièrement opérée lors de l'utilisation de la méthode de détection ultra-sensible selon l'invention pour une analyse multiplexée.

Dans le cadre de l'analyse multiplexée, permettant la détection et/ou quantification simultanée d'au moins deux substances différentes dans un échantillon, les différentes substances à analyser de l'échantillon peuvent être immobilisées en des zones prédéfinies et distinctes à la surface du support, par exemple en fonctionnalisant localement la surface du support par des agents de ciblage spécifiques de chacune des substances à analyser. La surface est préalablement passivée de manière à ce que les particules photoluminescentes ne s'y fixent pas en l'absence des substances à analyser.

Dans ce cas, les particules utilisées devront comporter sur leur surface de multiples agents de ciblage différents, et, plus particulièrement, au moins un agent de ciblage spécifique de chacune des substances à analyser. De cette façon, les substances à analyser seront quantifiées grâce à l'intensité d'émission de la particule sur chaque zone, la localisation spatiale de la zone en question indiquant la nature de la substance. La mise en œuvre de la méthode ultra-sensible de l'invention selon un multiplexage spatial est illustrée schématiquement en figure 22.

Il est également possible de combiner les approches multiplexées pour plusieurs échantillons et pour plusieurs substances à analyser en fonctionnalisant localement des zones prédéfinies et distinctes de la surface du support par des agents de ciblage spécifiques de chacune des substances à analyser et cela de façon répétée autant de fois que le nombre d'échantillons à analyser. Dans ce cas, l'intensité d'émission de la particule sur chaque zone renseigne sur la présence et/ou la concentration de chaque substance à analyser dans chaque échantillon, la localisation spatiale de la zone en question indiquant à la fois la nature de la substance et le nombre d'échantillons.

Les zones concernant différentes substances de chaque échantillon peuvent être séparées entre elles par une barrière étanche. De cette façon, la mise en contact de l'échantillon, contenant les différentes substances à analyser, avec l'ensemble des zones concernant un même échantillon à la surface du support (étape b) ci-dessus) peut se faire en une seule étape et sans augmentation du volume requis.

Egalement, il est possible pour une détection multiplexée de mettre en œuvre au moins deux types de nanoparticules, dopées par des ions de terres rares distincts, présentant des longueurs d'onde d'émission distinctes, par exemple $YVO_4$ :Eu et YAG :Ce, et couplées à des agents de ciblage de chacune des substances à analyser. La détection du signal de luminescence en utilisant deux filtres d'émission différents, permet de détecter et/ou de doser chacune des substances à analyser.

De préférence, dans le cadre d'une telle variante de détection multiplexée, au moins deux types de nanoparticules ayant des longueurs d'onde d'émission distinctes, et couplées chacune à des agents de ciblage de chacune des substances à analyser peuvent être utilisées, de manière à séparer les signaux de luminescence obtenus.

La combinaison des deux approches peut également être utilisée (analyse de plusieurs échantillons différents et de plusieurs substances différentes dans chaque échantillon), notamment pour la comparaison des concentrations de molécules cibles entre au moins deux échantillons, celle-ci étant réalisée en comparant les intensités des couleurs d'émission de chacune des nanoparticules, couplées chacune à l'agent de ciblage spécifique de chaque substance à analyser, et ce pour plusieurs zones de dépôt correspondant chacune à un échantillon différent.

Alternativement, la combinaison des deux approches peut également être utilisée - analyse dans plusieurs échantillons différents (prélèvement d'origine différente, ou de même origine à différents temps, dans différentes conditions, sous différents stimuli...) de plusieurs substances différentes dans chaque échantillon -, notamment pour la comparaison des concentrations de molécules cibles entre au moins deux échantillons. Celle-ci est réalisée en comparant les intensités des couleurs d'émission de chacune des nanoparticules, couplées chacune aux agents de ciblage spécifiques de chaque substance à analyser, et ce pour plusieurs zones de dépôt correspondant à des molécules cibles différentes. Dans ce cas, la comparaison des couleurs d'émission à un spot donné fournit une comparaison des concentrations d'une molécule entre les deux échantillons.

Différentes autres combinaisons de ces approches peuvent être envisagées : par exemple, analyser quatre substances en utilisant deux types de nanoparticules avec deux couleurs d'émission différentes, couplées chacune à deux des quatre différents agents de ciblage nécessaires pour la reconnaissance des quatre substances à analyser, et deux zones distinctes de la surface du support fonctionnalisées localement par deux des quatre agents de ciblage spécifiques de chacune des substances à analyser.

L'invention n'est bien entendu pas limitée à la variante de mise en œuvre décrite ci-dessous dans laquelle la substance à analyser est immobilisée à la surface d'un support (de type lamelles de verre ou plaques multi-puits par exemple). D'autres configurations sont envisageables pour la mesure de luminescence des particules photoluminescentes associées avec la substance à analyser de l'échantillon.

D'autres configurations sont envisageables pour l'association des particules photoluminescentes de l'invention à la substance à analyser.

Des variantes de réalisation peuvent mettre en œuvre par exemple des bandelettes d'analyse dans lesquelles l'analyte migre d'une extrémité à l'autre en réagissant avec les molécules cibles, ou encore un gel pour séparer les molécules biologiques et une membrane où les molécules sont spécifiquement liées et détectées, à l'instar de la méthode « Western blot ».

Dans d'autres variantes, la surface de réaction n'est pas de type support solide, mais peut être par exemple une autre nanoparticule, une micro-bille magnétique, etc. La mesure peut être par exemple effectuée directement au sein de l'échantillon à analyser. Dans le cas où l'échantillon est gazeux, ce support d'échantillon peut prendre la forme d'un volume fermé pour empêcher la dispersion de l'échantillon à tester. Le support d'échantillon peut également prendre la forme d'une cuve ou cuvette, en particulier dans le cas où l'échantillon est sous forme de solution.

L'utilisation selon l'invention peut également être adaptée pour être mis en œuvre dans des technologies de type cytométrie en flux (en anglais « Fluorescence-activated cell sorting » ou FACS). Dans ce cas, des particules de l'invention, couplées à des agents de ciblage visant la reconnaissance de substances composant le type cellulaire à analyser, sont mises en contact avec les cellules et le système de cytométrie doit être adapté pour comporter une source d'excitation, de préférence un laser, à une longueur d'onde adaptée à l'excitation des ions terres rares luminescents constituant les nanoparticules.

L'utilisation selon l'invention peut également être adaptée pour être mis en œuvre dans des technologies de type immunocytochimie et immunohistochimie.

## MESURE DE LA LUMINESCENCE

Comme indiqué précédemment, l'utilisation selon l'invention met plus particulièrement en œuvre une étape (i) d'excitation des ions de terres rares des particules photoluminescentes par un dispositif d'illumination d'une puissance d'au moins 50 mW et d'une intensité d'excitation d'au moins 1 $W/cm^2$ et une étape (iii) de détection de l'émission de luminescence par les particules après une absorption à un photon.

### Montage de détection

l'utilisation selon l'invention est avantageusement mise en œuvre à l'aide d'un appareillage peu sophistiqué et peu onéreux.

Plus précisément, il comporte, d'une manière générale :

- un dispositif d'illumination, de préférence de type laser, d'une puissance d'au moins 50 mW et d'une intensité d'excitation d'au moins 1 $W/cm^2$ ; et
- un appareil de détection de l'intensité lumineuse émise par les particules.

La source d'excitation peut également être une lampe ou une diode électroluminescente (LED).

Il sera fait référence, dans la suite du texte, aux figures 2 et 12-13 annexées, qui représentent, de manière schématique et partielle, des installations adaptées à la mise en œuvre du procédé ultra-sensible de l'invention.

L'appareillage peut en outre comporter un support adéquat pour l'immobilisation de la substance à analyser dudit

échantillon, comme décrit ci-dessus.

Selon une variante de réalisation, le système de détection utilisé selon l'invention comporte un système de translation du support ou du dispositif d'illumination laser, permettant d'illuminer successivement différentes zones localisées du support. Une telle variante est notamment utilisée pour la mise en œuvre de la méthode de détection de l'invention pour un multiplexage spatial (figure 22), le système de translation permettant l'illumination laser successivement de chacune des zones prédéfinies comportant les substances à analyser dans un même échantillon, voire dans plusieurs échantillons différents. Le signal d'émission est alors enregistré en fonction de la position d'illumination du support.

Le dispositif d'illumination laser peut être plus particulièrement composé d'une source (1) laser de puissance d'au moins 50 mW, en particulier d'au moins 500 mW, de préférence d'au moins 1 W, et d'un montage optique (2) de mise en forme du faisceau laser permettant d'obtenir une intensité d'excitation au niveau des particules luminescentes d'au moins 1 $W/cm^2$, de préférence d'au moins 10 $W/cm^2$.

Des diodes laser de forte puissance permettant cette réalisation, émettant notamment à 465 nm, sont disponibles dans le commerce depuis peu, pour l'excitation des ions $Eu^{3+}$.

Une telle intensité d'excitation assure l'excitation directe des ions de terres, *i.e.* en résonance avec les états électroniques de ces ions, et non par excitation de la matrice $AVO_{4(1-y)}(PO_4)_y$, ou d'une autre matrice d'oxyde métallique, et transfert d'énergie ultérieur vers ces ions.

Il n'était nullement évident de pouvoir utiliser ces particules photoluminescentes à titre de sondes luminescentes au travers une excitation directe des ions de terres rares. En effet, pour la plupart des ions lanthanides, la transition électronique liée à l'absorption de photons est une transition, dite « interdite », d'une configuration d'électrons *4f* à une autre configuration d'électrons *4f*. L'absorption n'est ainsi que très légèrement permise du fait d'un faible mélange d'orbitales d (les transitions d -> f ou f -> d étant permises). Le fait que la transition électronique liée à l'absorption est « interdite » se traduit par une faible largeur spectrale du pic d'absorption et par un faible coefficient d'extinction de ces nanoparticules en solution.

De fait, même si des particules photoluminescentes formées d'une matrice cristalline dopée par des ions de terres rares ont déjà été mises en œuvre, elles sont généralement utilisées au travers une excitation de leur matrice cristalline, cette dernière absorbant généralement bien plus fortement que les ions absorbants. Toutefois, la matrice cristalline absorbant généralement dans l'UV, la mise en œuvre d'une excitation de la matrice cristalline présente plusieurs inconvénients : d'une part, des lasers émettant dans l'UV sont peu disponibles dans le commerce, souvent onéreux et peu compacts et, d'autre part, l'excitation dans l'UV est susceptible de provoquer simultanément l'excitation d'autres biomolécules pouvant être présentes dans l'échantillon à analyser. De plus, l'absorption et la diffusion par les substrats solides utilisés (verres, plastiques, ...) durant la mesure sont connues pour absorber significativement dans l'UV et, par conséquent, pour induire des signaux parasites d'amplitude importante.

Le dispositif d'illumination laser peut également comprendre un montage optique, en particulier un système d'au moins une lentille, disposé sur la trajectoire du faisceau laser de manière à contrôler la taille du faisceau au niveau de la zone du support présentant les particules associées à la substance à analyser.

L'utilisation selon l'invention, qui repose sur une excitation directe des ions de terres rares des nanoparticules, permet avantageusement de s'affranchir des inconvénients liés à une excitation de la matrice cristalline.

Par ailleurs, comme évoqué précédemment, l'utilisation d'une forte puissance et d'une forte densité d'excitation selon l'invention n'est pas préjudiciable à l'émission de luminescence par les nanoparticules mises en œuvre selon l'invention qui ne sont pas sujettes à des phénomènes de saturation ou de photodégradation.

De préférence, le laser est monochromatique, d'une largeur spectrale comparable à la largeur spectrale du pic d'absorption des ions lanthanides luminescents.

L'excitation est réalisée dans le visible ou dans le proche infra-rouge.

L'excitation peut être par exemple réalisée à la longueur d'onde de 465 nm pour des particules de type $Y_{0,6}Eu_{0,4}VO_4$.

Le montage optique de mise en forme du faisceau laser peut comporter, de manière classique, un système de collimation et de diminution de la taille du faisceau laser, par exemple à l'aide de lentilles, en particulier de deux lentilles, comme décrit en exemple 3. Il permet alors de contrôler la zone illuminée au niveau de la zone du support présentant les particules associées à la substance à analyser de façon à obtenir une intensité appropriée (par exemple 10 $W/cm^2$) et une illumination dont les dimensions sont voisines ou plus petites que celles du spot de dépôt (par exemple 1 mm de diamètre).

L'excitation en étape (ii) peut être plus particulièrement opérée avec un faisceau d'excitation laser orienté de sorte à former un angle d'incidence avec la verticale du support présentant en surface lesdites particules associées à la substance à analyser, supérieur ou égal à 55° pour une illumination à grande angle de l'échantillon ; en particulier inférieur à 60° dans le cas d'une mesure effectuée en milieu aqueux sur un substrat de verre ou de plastique.

L'utilisation d'un grand angle d'incidence pour le faisceau laser d'excitation permet de réduire le volume de milieu excité au-dessus de la surface, et ainsi de réduire les signaux parasites.

Selon un mode de réalisation particulièrement avantageux, l'utilisation selon l'invention met en œuvre une

excitation des particules par onde évanescente, en particulier à l'aide d'un montage d'illumination laser de type TIRF (fluorescence par réflexion totale interne). Ceci est permis par la réalisation d'un angle d'incidence du faisceau excitateur supérieur ou égal à 61°, en particulier compris entre 61° et 63°, dans le cas d'une interface verre/eau entre le support et l'échantillon à analyser.

Il appartient à l'homme du métier d'opérer les ajustements nécessaires au dispositif de détection pour obtenir cette incidence pour une excitation par onde évanescente.

Comme illustré en figure 13, il peut comporter notamment un parallélépipède d'indice de réfraction supérieur à celui de la solution de réaction, par exemple, proche de celui du matériau utilisé pour le support, permettant un angle d'incidence du faisceau excitateur supérieur à l'angle critique de l'interface substrat solide/échantillon, en particulier supérieur ou égal à 61° dans le cas d'une interface verre/solution aqueuse, de sorte à obtenir une réflexion totale du faisceau à l'interface support/échantillon et une excitation par onde évanescente des particules associées à la substance à analyser.

Selon un autre mode de réalisation particulièrement avantageux, dans le cadre de la mise en œuvre de particules à durée de vie d'émission longue (en particulier présentant une durée de vie d'émission supérieure ou égale à 1 μs, en particulier supérieure ou égale à 50 μs), il est possible d'utiliser une détection résolue en temps de l'émission, en particulier une détection retardée du signal émis par les particules photoluminescentes.

L'homme du métier est à même d'adapter le système de détection mis en œuvre pour permettre une exploitation de l'émission de luminescence résolue en temps.

Pour ce faire, un hacheur mécanique (4) peut être par exemple placé sur le trajet du faisceau laser incident, comme illustré en figure 13.

Le principe de fonctionnement d'un tel hacheur mécanique et les méthodes d'analyse du signal obtenu pour isoler le signal de luminescence des nanoparticules sont décrits plus précisément dans l'exemple 3.2 qui suit. L'utilisation d'un hacheur mécanique et d'une fréquence de détection du signal par le photomultiplicateur du dispositif de détection de 100 kHz permet de s'affranchir de la fluorescence parasite.

Une telle détection résolue temporellement permet ainsi avantageusement de limiter la contribution au signal de luminescence d'espèces parasites présentes dans l'échantillon (sérum, sang, etc.) ou dans les substrats solides utilisés (verre, plastique, etc.), en séparant temporellement des signaux de luminescence parasites du signal émis par les nanoparticules, car ces signaux parasites ont généralement des durées de vie caractéristiques inférieures à 1 μs, voire inférieures à 100 ns, voire inférieure à 10 ns.

Il est entendu que les différents modes de réalisation particuliers décrits ci-dessus peuvent être combinés pour réaliser différentes variantes de mise en œuvre de l'utilisation selon selon l'invention.

En particulier, la détection résolue en temps et une excitation des nanoparticules par onde évanescente (montage TIRF) peuvent être mis en œuvre conjointement, comme illustré en figure 13, ou non.

Ainsi, selon un mode de réalisation préféré, l'utilisation selon l'invention combine avantageusement une excitation des nanoparticules par onde évanescente, en particulier à l'aide d'un montage d'illumination laser de type TIRF, et une détection résolue en temps de l'émission, en particulier une détection retardée de l'émission (mise en œuvre de nanoparticules à longue durée d'émission, utilisation d'un hacheur).

Ainsi, avantageusement, dans l'utilisation selon l'invention, la durée de vie d'émission par les nanoparticules est supérieure ou égale à 1 μs, en particulier supérieure ou à égale à 50 μs, la détection de l'intensité lumineuse en étape (iii) comprenant une détection résolue en temps de l'émission, en particulier une détection retardée de l'émission.

La combinaison de ces deux modes permet avantageusement d'éliminer l'émission due aux particules en solution, autres que celles à analyser immobilisées à la surface du support, ainsi que toute émission d'espèces parasites présentes dans l'échantillon (sérum, sang, etc.) ou dans les substrats solides utilisés (verre, plastique, etc.).

Une telle variante de réalisation est particulièrement avantageuse, en ce qu'elle permet de s'affranchir de certaines étapes classiques pour la préparation des échantillons à analyser, et en particulier d'étapes de rinçage et/ou de centrifugation, par exemple, dans le cas d'un rinçage lors de l'analyse pour éliminer les substances (molécules, protéines, peptides, etc.) contenues dans du sérum de sang, autres que celles concernées par l'analyse à effectuer (rinçage permettant d'aller de la phase 2 à la phase 3 indiquées dans la figure 1) ou pour éliminer les particules qui ne se sont pas fixées sur la surface du support et restent en solution lors de la phase 4 indiquée dans la figure 1) ou, par exemple, des étapes de centrifugation précédant l'analyse proprement dite, telle qu'illustrée dans la figure 1, pour éliminer les cellules sanguines dans les prélèvements de sang, étapes qui sont généralement longues et effectuées manuellement, en particulier pour les étapes de centrifugation.

La détection de l'émission de luminescence peut être opérée par mesure de l'intensité lumineuse émise à une longueur d'onde de luminescence des particules photoluminescentes mises en œuvre. A titre d'exemple, dans le cas de la mise en œuvre de nanoparticules $Y_{1-x}Eu_xVO_4$, l'intensité lumineuse émise peut être mesurée à la longueur d'onde de luminescence de $Eu^{3+}$, à savoir 617 nm.

Le dispositif de détection de l'intensité lumineuse peut comprendre un détecteur unique, en particulier de type photomultiplicateur, photodiode, photodiode à avalanche, ou un détecteur de type réseau de dispositifs photosensibles

consistant en une surface 2D de pixels de détection tel qu'une caméra CCD ou EM-CCD ou caméra CMOS. Un dispositif de détection 2D permet de mesurer simultanément le signal d'émission de nanoparticules provenant des différentes zones correspondant à différents échantillons et/ou différentes substances à analyser sur la surface du support et ne nécessite pas de déplacement du support ou du faisceau d'excitation.

De préférence, le dispositif de détection de l'intensité lumineuse comprend un détecteur unique, en particulier un photomultiplicateur, ce qui permet de réaliser un dispositif de détection moins onéreux.

Il peut également comprendre un montage optique, en particulier un système d'au moins une lentille à grande ouverture numérique, pour focaliser l'émission de luminescence vers le détecteur, en particulier vers le photomultiplicateur.

Des filtres interférentiels peuvent également être placés sur le trajet du faisceau émis pour éliminer spectralement les signaux parasites.

La détection peut être opérée en réflexion, vers le bas, comme représenté schématiquement en figure 12-a, autrement dit du côté de la face du support recevant le faisceau d'excitation laser.

Alternativement, elle peut être opérée en transmission, vers le haut, comme représenté schématiquement en figure 12-b. La collection de la luminescence par le haut est préférée dans le cas d'une excitation en onde évanescente, de façon à éviter le passage des photons de luminescence à travers le parallélépipède permettant la réalisation de l'onde évanescente.

## ANALYSE DE LA MESURE DE LUMINESCENCE

L'utilisation selon l'invention comprend enfin une étape (iii) de détermination de la concentration de la substance par interprétation de la mesure de luminescence.

Il est entendu que le système de détection selon l'invention peut comprendre en outre tout moyen permettant d'analyser l'émission de luminescence, par exemple un convertisseur permettant d'enregistrer et d'exploiter le signal de luminescence.

L'interprétation de la mesure de la luminescence peut être effectuée par référence à un étalon ou étalonnage préétablis.

Plus précisément, la quantité de la substance à analyser dans l'échantillon peut être déterminée par référence à une courbe d'étalonnage préétablie au moyen de mesures réalisées avec des échantillons à quantité connue en ladite substance, de préférence dans des conditions identiques à celles de l'étude de l'échantillon, ces conditions identiques incluant notamment le solvant et le pH du milieu.

De manière avantageuse, comme illustré en exemple 3, le procédé ultra-sensible selon l'invention permet de détecter et de quantifier une substance d'intérêt dans un échantillon en une teneur strictement inférieure à 10 pM, en particulier inférieure à 1 pM, voire inférieure à 0,1 pM, ou encore inférieures à 0,01 pM (i.e. 10 fM).

De fait, il permet une détection au moins dix fois, en particulier au moins 100 fois, voire 1000 fois plus sensible que la méthode d'immuno-détection enzymatique de type ELISA, utilisant les mêmes anticorps de reconnaissance et de ciblage.

Les exemples et figures présentés ci-dessous sont uniquement donnés à titre illustratif et non limitatif de l'invention.

## FIGURES

Figure 1 : Représentation schématique du principe de détection des biomolécules : surface fonctionnalisée avec un anticorps de capture (phase 1) ; mise en contact de l'échantillon à analyser (phase 2), lavage (phase 3) et association des particules photoluminescentes couplées à un agent de ciblage (ici un anticorps) avec la substance immobilisée à la surface du support suivie d'un lavage pour éliminer les particules non immobilisées (phase 4) ;

Figure 2 : Représentation schématique d'un dispositif de détection selon l'invention, composé notamment d'une source laser 1, d'un système de collimation et de diminution de la taille du faisceau laser 2, d'un miroir 3 pour orienter le faisceau laser, d'un support 6 pour les échantillons, et d'un système de détection des photons émis comportant une lentille à grande ouverture numérique 10 et un photomultiplicateur 12 ;

Figure 3 : Images obtenues par microscopie électronique à transmission (MET) des nanoparticules obtenues selon l'exemple 1 (Barre d'échelle : 60 nm (figure 3a) et 5 nm (figure 3b), respectivement) ;

Figure 4 : Histogramme de taille des nanoparticules déterminé à partir d'images de MET pour un ensemble d'environ 300 nanoparticules selon l'exemple 1 ;

Figure 5 : Diffractogrammes des rayons X obtenus pour les nanoparticules synthétisées selon l'exemple 1 (trait noir), et 3 (trait gris) ;

Figure 6 : Représentation schématique des nanoparticules de l'invention fonctionnalisées avec du citrate (figure 6a) ou avec du polyacide acrylique (PAA) (figure 6b). A des fins de clarté, une seule molécule de citrate ou de PAA est représentée. Il est entendu que chaque nanoparticule peut comporter sur sa surface de nombreuses molécules de citrate ou de PAA.

Figure 7 : Représentation schématique des réactions pour le couplage d'une nanoparticule selon l'invention avec la streptavidine, selon l'exemple 1 ;

Figure 8 : Représentation schématique du couplage des nanoparticules couplées avec la streptavidine, avec un anticorps biotinylé ;

Figure 9 : Représentation schématique de l'utilisation d'un hacheur mécanique rotatif pour l'obtention d'une illumination périodique, et profil temporel de l'intensité d'excitation obtenue. Dans le cas du profil temporel illustré, l'intensité d'excitation obtenue augmente ou baisse de façon progressive à cause de la taille finie du faisceau et du temps nécessaire à la pale du hacheur mécanique pour dévoiler ou cacher la totalité du faisceau d'excitation ;

Figure 10 : Signaux obtenus en mode d'excitation par onde évanescente (TIRF) à l'issue d'un demi-cycle de fermeture du hacheur avec de l'eau pure (a), des nanoparticules (goutte de concentration en ions vanadate de 3 mM) dans de l'eau (b) et dans du sérum (c) : signal brut (Figure 10-a) et signal de luminescence en détection retardée (détection restreinte à 150 µs après le début d'occultation du faisceau d'excitation) (Figure 10-b) ;

Figure 11 : A gauche : modélisation de la luminescence avec un système à 2 niveaux. A droite : signal moyenné émis par des nanoparticules ($Y_{0,6}Eu_{0,4}VO_4$) déposées sur une lamelle, collecté au cours de 500 cycles d'ouverture-fermeture (cercles) du hacheur, comparé à son ajustement (ligne pleine), déterminant la quantité de nanoparticules. Résultats obtenus en mode d'excitation HI-LO (i.e. le faisceau d'excitation formant un grand angle avec la verticale sur le support de l'échantillon) ;

Figure 12 : Représentation schématique d'un dispositif de détection selon l'invention, avec détection de l'émission vers le bas (Figure 12-a), et détection de l'émission vers le haut (Figure 12-b) ;

Figure 13 : Représentation schématique d'un dispositif de détection selon l'invention, avec hacheur du faisceau laser 4 et excitation en réflexion interne totale (à l'aide d'un parallélépipède de plexiglas 13 d'indice de réfraction supérieur ou égal à celui du matériau qui sert de support d'échantillon) ;

Figure 14 : Courbe de calibration de l'appareil de détection selon la variante 2, avec hacheur et avec excitation par onde évanescente, suivant le protocole décrit en exemple 3.2.ii (Figure 14-a) ; et selon la variante 3, sans hacheur et sans excitation par onde évanescente, suivant le protocole décrit en exemple 3.2.ii. (Figure 14-b) ;

Figure 15 : Détection d'insuline recombinante en solution par un kit ELISA (ABCAM Réf. ab100578). La limite de détection indiquée par le fournisseur du kit est de 9 pM (50 pg/mL) ;

Figure 16: Détection d'insuline recombinante en solution jusqu'à des concentrations de 9 fM (0,05 pg/mL) avec la variante 1 de l'appareil de détection décrite en exemple 3 (temps d'acquisition : 30 s ; lecture du signal toutes les 100 ms). Dans l'encart, le signal correspondant à une concentration égale à zéro a été soustrait ;

Figure 17: Détection d'insuline recombinante en solution jusqu'à des concentrations de 9 fM (0,05 pg/mL) avec la variante 2 de l'appareil de détection décrite en exemple 3, sans hacheur et sans excitation par onde évanescente (temps d'acquisition : 1 s ; lecture du signal toutes les 10 µs) ;

Figure 18 : Détection d'insuline recombinante en solution jusqu'à des concentrations de 9 fM (0,05 pg/mL) avec la variante 2 de l'appareil de détection décrite en exemple 3, avec hacheur et avec excitation par onde évanescente (temps d'acquisition : 1 s ; lecture du signal toutes les 10 µs) ;

Figure 19 : Détection d'insuline dans différents échantillons de sérum sur plaque multipuits suivant la variante 1 de l'appareil de détection décrite en exemple 3 (temps d'acquisition : 30 s ; lecture du signal toutes les 100 ms) ;

Figure 20: Détection de TSH recombinante en solution jusqu'à des concentrations de 3,2 fM suivant la variante 1 de l'appareil de détection décrite en exemple 3 avec hacheur (temps d'acquisition : 1 s ; lecture du signal toutes les 10 µs) ;

Figure 21 : Evolution du signal de luminescence détecté suivant la variante 1 de l'appareil de détection en fonction du temps d'incubation avec les conjugués anticorps-nanoparticules suivant l'exemple 4 ;

Figure 22 : Schéma du principe du multiplexage spatial ;

Figure 23 : Représentation schématique des deux « spots » de nanoparticules formés suivant l'exemple 5, et signal de luminescence détecté pour les différentes localisations ;

Figure 24 : Représentation schématique de la détection et quantification d'ADN/ARN à l'aide des particules selon l'invention couplées à de l'ADN simple brin. De l'ADN simple brin partiellement complémentaire au brin à détecter est fixé sur un support. Ensuite, l'échantillon contenant l'ADN ou l'ARN à détecter est incubé avec le support fonctionnalisé (gauche). Après rinçage, les particules couplées à de l'ADN simple brin, partiellement complémentaire à la partie non appariée de l'ADN ou ARN à détecter, sont incubées avec le support (milieu). Après rinçage, seules les nanoparticules immobilisées sur la surface du support après appariement avec l'ADN ou ARN à détecter sont présentes (droite). Elles peuvent être détectées et quantifiées comme décrit dans le texte.

Figure 25 : Représentation schématique de la détection et quantification d'ADN/ARN à l'aide des particules selon

l'invention couplées à des molécules de streptavidine.

(a) Cette variante de détection/quantification de l'ADN ou ARN utilise un ADN simple brin « de reconnaissance » couplé à une molécule de streptavidine à chaque bout à fixer sur le support de détection, l'ADN ou ARN simple brin à détecter et un ADN simple brin partiellement complémentaire à l'ADN couplé à une molécule de biotine. L'ADN couplé à la streptavidine est, avec sa partie non complémentaire à l'ADN couplé à la biotine, au moins partiellement complémentaire à l'ADN à détecter.
(b) La surface du support est passivée avec du PEG et fonctionnalisé avec de la biotine. L'ADN couplé à une streptavidine à chaque bout se fixe sur la surface du support fonctionnalisée. Ensuite, suite à une incubation avec l'ADN à détecter, celui-ci s'apparie avec l'ADN immobilisé sur la surface du support. Après rinçage, les biotines restant sur la surface sont passivées avec de la streptavidine. L'ADN « de révélation » fixé à une biotine est incubé avec la surface et s'apparie avec la partie complémentaire de l'ADN couplé à deux molécules de streptavidine.
(c) Après rinçage, les particules de l'invention couplées à des molécules de streptavidine sont incubées avec la surface et se fixent sur l'ADN simple brin « de révélation » *via* l'interaction avec la biotine.

Après rinçage, les particules immobilisées sur la surface peuvent être détectées et quantifiées comme décrit dans le texte. Il est également possible d'utiliser un ADN « de reconnaissance » couplé à une seule molécule de streptavidine à l'un de ses deux bouts. Dans ce cas, les ADN « de reconnaissance » sont fixés à la surface du support par un seul de leurs deux bouts, comme dans la variante 1.
Figure 26 : Spectres d'excitation et d'émission de la solution de nanoparticules $YVO_4$ :Dy 3% préparée en exemple 6 : spectre d'excitation dans la zone de l'absorption directe des ions $Dy^{3+}$ pour une détection à $\lambda$ = 572 nm (figure 26-a) et spectre d'émission pour une excitation à $\lambda$ = 278 nm correspondant à l'excitation de la matrice de vanadate (figure 26-b).
Figure 27 : Spectres d'excitation et d'émission de la solution de nanoparticules $YVO_4$ :Sm 3% préparée en exemple 7 : spectre d'excitation dans la zone de l'absorption directe des ions $Sm^{3+}$ pour une détection à $\lambda$ = 600 nm (figure 27-a) et spectre d'émission pour une excitation à $\lambda$ = 278 nm correspondant à l'excitation de la matrice de vanadate (figure 27-b).

## EXEMPLES

### EXEMPLE 1 hors invention

### Préparation des particules luminescentes

#### 1.1. Synthèse des nanoparticules $Y_{0,6}Eu_{0,4}VO_4$

Comme source d'ions de métavanadate $VO_3^-$, on utilise du métavanadate d'ammonium $NH_4VO_3$, l'orthovanadate $VO_4^{3-}$ étant obtenu *in situ* suite à une réaction avec une base, ici l'hydroxyde de tétraméthylammonium, $N(CH_3)_4OH$. Des nitrates d'yttrium et d'europium ont été utilisés comme sources d'ions $Y^{3+}$ et $Eu^{3+}$.
Une solution aqueuse de 10 mL de $NH_4VO_3$ à 0,1M et 0,2 M de $N(CH_3)_4OH$ (solution 1) est fraichement préparée.
Un volume de 10 mL d'une autre solution de $Y(NO_3)_3$ et de $Eu(NO_3)_3$ à 0,1 M en ions ($Y^{3+}$ + $Eu^{3+}$) est ajoutée goutte à goutte à l'aide d'un pousse seringue dans la solution 1 à un flux de 1 mL/min.
Le rapport en concentration molaire entre $Y(NO_3)_3$ et $Eu(NO_3)_3$ est choisi en fonction du rapport entre ions $Y^{3+}$ et $Eu^{3+}$ souhaité dans la nanoparticule, typiquement le rapport molaire $Y^{3+}$ :$Eu^{3+}$ est de 0,6 : 0,4.
Dès l'ajout de la solution $Y(NO_3)_2/Eu(NO_3)_3$, la solution devient diffusante et apparaît blanche/laiteuse sans formation de précipité. La synthèse se poursuit jusqu' à l'ajout total de la solution $Y(NO_3)_2/Eu(NO_3)_3$.
La solution finale de 20 mL doit à présent être purifiée pour éliminer l'excès de contre-ions. Pour ce faire, des centrifugations (typiquement trois) à 11000 g (Sigma 3K10, Bioblock Scientific) pendant 80 minutes suivies chacune d'une redispersion par sonification (Bioblock Scientific, Ultrasonic Processor fonctionnant à 50 % à une puissance de 400 W pendant 40 s) sont utilisées jusqu'à atteindre une conductivité strictement inférieure à 100 $\mu$S.cm$^{-1}$.
La conductivité est mesurée à l'aide d'un conductimètre de chimie.
La synthèse des nanoparticules $Y_{0,6}Eu_{0,4}VO_4$ à la surface desquelles sont immobilisés les cations tétraméthylammonium peut être schématisée comme suit :

$$NH_4VO_3 + 2\,(N(CH_3)_4)OH \leftrightarrows VO_4^{3-} + 2\,N(CH_3)_4^+ + NH_4^+$$

$$VO_4^{3-} + 2\,N(CH_3)_4^+ + NH_4^+ + 0.6\,Y(NO_3)_3 + 0.4\,Eu(NO_3)_3 \rightarrow Y_{0.6}Eu_{0.4}VO_4 + 2\,N(CH_3)_4^+ + NH_4^+ + 3NO_3^-$$

Deux essais de synthèse (« synthèse 1 » et « synthèse 2 ») sont effectués.

Résultat

L'observation visuelle de la solution de nanoparticules selon l'invention, après avoir été laissée au repos pendant 16 heures dans un flacon, montre une solution uniformément diffusante.

La solution finale reste très stable dans l'eau, même après plusieurs mois dans le pH final de la synthèse (environ pH 5). La solution reste stable y compris dans le milieu de synthèse (avant l'élimination de l'excès de contre-ions), de force ionique pourtant élevée (>0,1 M).

Le potentiel zêta des nanoparticules est déterminé avec un appareil DLS-Zeta Potential (Zetasizer Nano ZS90, Malvern). Les résultats des potentiels zêta mesurés pour les nanoparticules des deux synthèses sont rassemblés dans le tableau 1 ci-après.

**TABLEAU 1**

| | Synthèse 1 | Synthèse 2 |
|---|---|---|
| **Conductivité (μS/cm)** | 93 | 80 |
| **pH** | 4,81 | 4,96 |
| **Potentiel zêta ζ** | -33,3 | -34,6 |

Pour les observations par microscopie électronique à transmission (MET), des solutions diluées de nanoparticules sont déposées sur une grille en carbone. Les observations sont réalisées à l'aide d'un microscope Philips CM30 fonctionnant à 300 kV avec une résolution de 0,235 nm.

L'observation des nanoparticules par MET (figure 3) montre que les nanoparticules sont de forme ellipsoïde allongée. Les dimensions des nanoparticules sont déterminées à partir d'images de MET pour un ensemble d'environ 300 nanoparticules (figure 4). Les nanoparticules de l'invention présentent une longueur du grand axe, notée *a,* comprise entre 20 et 60 nm, avec une valeur moyenne d'environ 40 nm, et une longueur du petit axe, notée b, comprise entre 10 et 30 nm, avec une valeur moyenne d'environ 20 nm.

Les images de MET (figure 3) ne permettent pas de distinguer des plans cristallins, ce qui est probablement attribuable au fait que la nanoparticule est constituée de plusieurs cristallites de taille plus faible que la taille de la nanoparticule. La nature majoritairement cristalline et polycristalline des nanoparticules est confirmée par des expériences de diffraction de rayons X.

Le diffractogramme des rayons X obtenu à l'aide d'un diffractomètre Philips X-pert avec la raie $K_{\alpha 1}$ du Cuivre ($\lambda$=1,5418 Å), est représenté en figure 5. L'intensité diffractée est enregistrée en utilisant un détecteur X'Celerator area detector (PANalytical).

La longueur de cohérence dans une direction cristallographique et donc la taille moyenne des cristallites constituant la nanoparticule dans cette direction cristallographique peut être estimée à partir de la largeur des pics dans le diffractogramme RX par application de la formule de Scherrer. Les valeurs de longueur de cohérence obtenues pour les différentes directions cristallographiques sont comprises entre 3 et 40 nm. La longueur de cohérence dans au moins une direction cristallographique étant inférieure à la dimension de la nanoparticule dans cette direction, on peut en déduire que les nanoparticules sont d'une cristallinité imparfaite (polycristallinité, défauts ou porosité). Dans la direction (200) (Fig. 3, pic à $2\theta \cong 25°$), la longueur de cohérence est de 10,2 nm, légèrement plus faible que la longueur de cohérence pour les nanoparticules de l'exemple 3 (11,1 nm).

**1.2. Couplage des nanoparticules avec la protéine streptavidine**

i.a. Greffage du citrate à la surface des nanoparticules

A l'issue de la synthèse des nanoparticules selon l'exemple 1.1., on prélève 250 μL de particules $Y_{0,6}Eu_{0,4}VO_4$ de concentration 5 mM en ions vanadate, on centrifuge la solution de nanoparticules à 17 000 g pendant 30 minutes..

On prélève le culot et on le disperse dans 1 mL d'une solution d'eau distillée contenant l'ion citrate (concentration 0,2 M).

La solution est ensuite sonifiée pendant 5 minutes dans un bain de glace, centrifugée à 17 000 g pendant 30 minutes et le culot est récupéré et redispersé dans de l'eau distillée contenant l'ion citrate (concentration 0,2 M). Cette étape est répétée 3 fois.

A l'issue de ce greffage, les particules sont dispersées dans de l'eau distillée, solvant dans lequel elles sont stables.

La fonctionnalisation des nanoparticules par du citrate peut être remplacée par une fonctionnalisation par du PAA (par exemple de degré de polymérisation compris entre 3 et 10000), en mettant en œuvre un sel de PAA, comme par exemple un sel de sodium ou d'ammonium.

i.b. Greffage de PAA à la surface des nanoparticules

A l'issue de la synthèse des nanoparticules selon l'exemple 1.1., on prélève 500 μL de particules $Y_{0,6}Eu_{0,4}VO_4$ de concentration 10 mM et on centrifuge la solution de nanoparticules à 17 000 g pendant 30 minutes.

On prélève le culot et on le disperse dans 1 mL d'une solution d'eau distillée contenant le PAA de poids moléculaire 1800 Da (concentration 75 mM).

La solution est ensuite sonifiée pendant 5 minutes, centrifugée à 17 000 g pendant 30 minutes et le culot est récupéré et redispersé dans de l'eau distillée contenant le PAA de poids moléculaire 1800 Da (concentration 75 mM). Cette étape est répétée 3 fois.

A l'issue de ce greffage, les particules sont dispersées dans de l'eau distillée, solvant dans lequel elles sont stables.

En figure 6, sont représentés de manière schématique les nanoparticules de l'invention fonctionnalisées avec (a) du citrate et (b) du polyacide acrylique.

ii. Couplage des nanoparticules avec la streptavidine

On centrifuge les nanoparticules (NPs) greffées avec les ions citrate ou avec le PAA à 16 000 g pendant 1 heure, puis le culot est récupéré.

Le couplage des nanoparticules greffées en surface par du citrate avec la streptavidine est opéré suivant le protocole suivant :

**1.** Préparer fraichement une solution mixte de EDC [1]/Sulfo-NHS [2] (concentration 30 et 30 mg/mL, respectivement) dans du tampon MES [3] (pH 5-6).

**2.** Disperser par sonification (bain ultrasons) le culot des NPs dans 250 μL de la solution préparée en étape 1. Les pertes lors des centrifugations étant faibles, la concentration en ions vanadate reste de l'ordre de 5 mM, ce qui donne une concentration en nanoparticules de 48 nM.
(La concentration en vanadate des solutions de nanoparticules a été déterminée par dissolution des particules dans un milieu acide suivie d'une détermination colorimétrique de la concentration en ions vanadate, telle que décrite dans la référence [34] Abdesselem et al., ACS Nano 8, 11126-11137 (2014). La concentration molaire en nanoparticules a été déterminée à partir de la concentration en ions vanadate, comme décrit dans la référence [35].

**3.** Préparer une solution de streptavidine (SA) de 100 nM dans du tampon phosphate pH 7,4 avec NaCl à 10 mM. Diluer la solution de streptavidine jusqu'à atteindre une concentration déterminée par le nombre de protéines greffées par nanoparticule souhaité (pour un rapport streptavidine : NPs de 1:1, 5:1 et 10:1, choisir, respectivement des concentrations de 4,8 nM, 24 nM et 48 nM). Choisir de préférence un rapport d'au moins 20:1, i.e. une concentration de SA de 96 nM. Rajouter 250 μL de cette solution à la solution de nanoparticules.

**4.** Laisser incuber entre 2 et 4h à température ambiante sous agitation.

**5.** Rajouter 1 mL de PBST [4] et vortexer.

**6.** Faire une centrifugation de 17 000 g pendant 30 min et récupérer le culot pour éliminer les protéines non couplées aux NPs. Enlever totalement le surnageant. Redisperser les NPs couplées aux protéines dans 1mL de PBST et sonifier au bain à ultrasons. Répéter cette étape deux fois.

**7.** Récupérer les NPs couplées aux protéines dans 250 μL de PBS [5] avec 1 % de BSA[6].

**8.** Garder à 4°C pour utilisation immédiate ou aliquoter et garder à -80°C.

Le couplage des nanoparticules avec la streptavidine est représenté schématiquement en figure 7.

Matériel mis en œuvre pour l'essai de fonctionnalisation :

1 *N*-(3-Dimethylaminopropyl)-*N'*-ethylcarbodiimide hydrochloride (EDC) (sigma, cat# E1769).
2 *N*-Hydroxysulfosuccinimide sodium salt (Sulfo-NHS) (sigma, cat# 56485).
3 2-(*N*-morpholino)ethanesulfonic acid (MES) (10mM, pH 5-6).
4 Tampon phosphate salin pH 7,4 (10 mM NaCl)+ 0,05% Tween 20 (PBST).
5 Albumine de sérum de boeuf (BSA) (sigma, cat# A3059).
6 Tampon phosphate salin (PBS) (pH 7,4, 10 mM NaCl).

Le nombre de molécules de streptavidine (SA) par nanoparticule (NP), noté R, caractérisé à l'issue du protocole de couplage, est déterminé *via* le dosage de la streptavidine par la méthode dite de BCA, selon le protocole détaillé ci-dessus.

*Protocole de caractérisation du rapport streptavidine : nanoparticules (SA :NPs)*

*i. Dosage de la SA par la méthode dite de BCA*

Principe

En milieu alcalin, les protéines réduisent $Cu^{2+}$ en Cu. Le sel de l'acide bicinchoninique (BCA) forme un complexe coloré avec les ions $Cu^{2+}$. Ce complexe est quantifiable grâce à son absorption à 562 nm.

Mode opératoire

Kit de test BCA de chez ThermoFisher (Pierce™ BCA Protein Assay Kit Cat. Num. 23225)

- Préparation du réactif du test $Cu^{2+}$/BCA selon le protocole du kit ThermoFisher.
- Préparation de la SA pour la courbe d'étalonnage. Pour la courbe de calibration, le test est réalisé trois fois.

**TABLEAU 2** : concentration en streptavidine des solutions d'étalonnage

| Tubes | Concentration finale en SA (μg/mL) |
|---|---|
| A | 2000 |
| B | 1500 |
| C | 1000 |
| D | 750 |
| E | 500 |
| F | 250 |
| G | 125 |
| H | 25 |
| I | **0 =** blanc |

Le mode opératoire est le suivant :

- Prendre une plaque de 96 puits. Mettre 25 μL de chaque tube d'étalon A à I (de concentration connue en SA) ou de protéines conjuguées aux nanoparticules à doser dans les puits correspondants.
- Rajouter 200 μL du réactif du test $Cu^{2+}$/BCA dans chaque puits. Homogénéiser, couvrir et laisser incuber 30 min à 37°C.
- Lire les absorbances (A) à 562 nm en fonction de la concentration finale en tenant compte de la dilution. Etablir la relation d'étalonnage A= f (Concentration SA en μg/mL) par régression linéaire.

La concentration des protéines conjuguées aux nanoparticules à doser est déduite à partir de l'équation de la régression linéaire obtenue avec la courbe d'étalonnage.

*ii. Caractérisation du rapport SA : NPs*

La concentration massique de la streptavidine obtenue avec le test de BCA est convertie en concentration molaire via la formule suivante :

$$[\mathrm{SA}]_{\mathrm{molaire}}(\mathrm{moles/L}) = \frac{[\mathrm{SA}]\mathrm{massique\ (g/L)}}{\mathrm{Masse\ Molaire\ (g/mole)}}$$

Pour obtenir le rapport (R) du nombre de SA : NPs, nous appliquons enfin l'équation suivante :

$$R = \frac{[SA]molaire}{[NPs]molaire}$$

Le tableau 3 ci-dessous rassemble les valeurs obtenues pour les différents rapports de concentration entre la solution de streptavidine et la solution de nanoparticules de départ.

**TABLEAU 3 :** Caractérisation du couplage nanoparticule-streptavidine pour différents rapports de concentration entre la solution de streptavidine et la solution de nanoparticules.

| | | | |
|---|---|---|---|
| **Rapports de concentration entre la solution de streptavidine et la solution de nanoparticules** | 1 :1 | 5 :1 | 10 :1 |
| **Nombre déterminé de molécules de streptavidine par nanoparticule à l'issue du couplage** | 0,97 | 3,8 | 9,29 |

Comme il ressort des résultats présentés dans le tableau 3, le nombre de molécules de streptavidine par nanoparticule après couplage, est du même ordre que le rapport de concentration dans les solutions initiales, et indique ainsi un couplage de très bonne efficacité.

### 1.3. Couplage des nanoparticules couplées à la streptavidine (Nanoparticules-SA) avec des anticorps biotinylés

Les nanoparticules couplées avec la streptavidine sont ensuite couplées à un anticorps biotinylé, spécifique de la substance à doser (insuline ou hormone thyréostimuline (TSH), comme illustré de manière schématique en figure 8.

Le protocole de couplage est le suivant.

Entre 10 et 50 μg/mL d'anticorps biotinylés sont mis au contact des nanoparticules-SA en excès pendant 1 h à l'aide d'une roue tournante afin de favoriser la diffusion et l'interaction entre la biotine et la streptavidine. La concentration d'anticorps est choisie de sorte à avoir 3 anticorps par NP. Ainsi, il est certain que tous les anticorps vont se fixer sur les nanoparticules-SA et il n'y a pas besoin d'étape d'élimination des anticorps non fixés. La concentration en anticorps est donc trois fois plus élevée que la concentration en nanoparticules.

Alternativement, il est également possible de coupler directement des anticorps aux nanoparticules greffées avec des ions citrate. Dans ce cas, le même protocole que ci-dessus est à utiliser en remplaçant la solution de streptavidine par une solution d'anticorps.

### 1.4. Méthode de couplage alternative par couplage direct des nanoparticules avec les anticorps

Selon une méthode alternative, le couplage des nanoparticules avec les anticorps non biotinylés peut être réalisé directement par couplage des anticorps sur des nanoparticules fonctionnalisées avec de l'APTES (transformation des groupements amino en groupements carboxyle, activation des groupements carboxyle et réaction directe avec les groupements amino à la surface des anticorps), suivant le protocole détaillé suivant.

#### 1.4.1. Recouvrement des nanoparticules par une couche de silice

A l'issue de la synthèse des nanoparticules en étape 1.1., on centrifuge la solution de nanoparticules à 17 000 g pendant 3 minutes, pour précipiter les éventuels agrégats de nanoparticules et on récupère le surnageant. Une sélection en taille est réalisée. Pour ce faire, plusieurs centrifugations à 1900 g pendant 3 min sont réalisées. Chaque centrifugation est suivie d'une redispersion des nanoparticules au sonificateur, puis la taille des nanoparticules est déterminée à l'aide d'un appareil DLS-Zeta Potentiel (Zetasizer Nano ZS90, Malvern).

Un volume de 25 mL de particules $Y_{0,6}Eu_{0,4}VO_4$ de concentration 20 mM en ions vanadate, est préparé. Un volume de 2,5 mL d'une autre solution de silicate de sodium pur (Merck Millipore 1.05621.2500) est ajouté goutte à goutte à l'aide d'une pipette afin d'enrober la surface des particules. Nous laissons agir cette solution sous agitation au moins cinq heures.

La solution est ensuite purifiée afin d'enlever l'excédent de silicate et les contre-ions de sodium. La solution est centrifugée à 11000 g (Sigma 3K10, Bioblock Scientific) pendant 60 minutes puis redispersée par sonification (Bioblock Scientific, Ultrasonic Processor, fonctionnant à 50 % à une puissance de 400 W). Cette étape est répétée jusqu'à ce que la conductivité de la solution soit inférieure à 100 μS/cm.

1.4.2. Greffage d'amines à la surface des nanoparticules

Dans un ballon de type tricol de 500 mL, mettre 225 mL d'éthanol absolu et ajouter 265 µL de APTES (3-Aminopropyltriethoxysilane) (Mw 221,37 g/mol Sigma Aldrich), ce qui correspond à une concentration finale de 1,125 mM. Cette quantité correspond à 5 équivalents de vanadate qui sont introduits. Un réfrigérant est ensuite branché au ballon. L'ensemble est posé sur un chauffe ballon et mis sous une hotte. Le mélange est chauffé à reflux à 90°C. Sur une des entrées du tricol une solution colloïdale de nanoparticules (concentration en ions vanadate [V] = 3 mM) dans 75 mL d'eau à pH 9 est ajoutée goutte à goutte à l'aide d'une pompe péristaltique avec un débit de 1 mL/min. L'ensemble est chauffé sous agitation pendant 24 h.

Après 48 heures, nous utilisons un évaporateur rotatif (rotavapor R-100, BUCHI) afin de concentrer partiellement les nanoparticules. La solution est mise en rotation dans un ballon adapté, et chauffé dans un bain à 50°C.

La solution récupérée est purifiée par plusieurs centrifugations dans un solvant éthanol : eau (3 :1). Après la purification, un tri en taille est réalisé en suivant le protocole décrit ci-dessus.

1.4.3. Greffage de carboxyles à la surface des nanoparticules aminées

Avant de démarrer le greffage un transfert de solvant est réalisé.

Le protocole de greffage est le suivant.

Transférer les NPs aminées du tampon EtOH : $H_2O$ vers le DMF ou DMSO en réalisant plusieurs centrifugations (13000 g, 90 min). Le culot est redispersé par sonification entre chaque centrifugation (20 s à 75%). Mesurer et déterminer la concentration des NPs.

Récupérer les NPS dans 5 mL de DMF puis rajouter 10% de l'acide succinique anhydride dans un bécher en verre (i.e. 0,5 g dans les 5 mL). Laisser réagir au moins toute une nuit sous atmosphère inerte tout en agitant.

Laver les NPs carboxylées au moins 2 fois par des centrifugations (13000 g pendant 60 min, Legend Micro 17R, Thermo Scientific) afin d'enlever le DMF et l'excès de l'acide succinique anhydride.

Resuspendre les particules carboxylées dans de l'eau ou du tampon MES à pH6 par sonification (Bioblock Scientific, Ultrasonic processor).

1.4.4. Couplage direct des nanoparticules avec les anticorps

Le couplage des nanoparticules greffées en surface avec du COOH est opéré suivant le protocole ci-dessous :

i) Préparer fraichement une solution mixte de EDC/Sulfo-NHS (concentration 500 et 500 mg/mL, respectivement) dans du tampon MES (pH 5-6).
ii) Dans 3 mL de solution préparée précédemment, rajouter 90 nM de NPs (ici il s'agit de la concentration en nanoparticules calculée à partir de la concentration en ions vanadate selon la référence Casanova *et al.* [37]) et laisser réagir 25 min à température ambiante tout en agitant.
iii) Laver rapidement les NPs par au moins 2 centrifugations (13000 g pendant 60 min, Legend Micro 17R, Thermo Scientific) avec de l'eau MilliQ pour enlever l'excès de réactifs.
iv) Récupérer le dernier culot après sonification dans du tampon phosphate de sodium à pH 7,3. Rajouter la quantité d'anticorps nécessaire en fonction du ratio recherché (Protéine : Nps), typiquement 2 µM pour un ratio de 20 : 1.
v) Laisser réagir cette solution entre 2 et 4h à température ambiante tout en agitant.
vi) Rajouter l'agent bloquant (glycine à 1%) pour qu'il réagisse avec les COOH libres et qu'il bloque les sites de réaction résiduels à la surface des NPs. Laisser réagir 30 min.
vii) Laver les Nps couplées aux protéines plusieurs fois par centrifugation en utilisant des filtres à centrifuger (Amicon Ultra 0,5mL, *Ref* UFC501096, Millipore) avec du PBS pH 7,2. Transférer les NPs dans leur milieu de stockage : tampon phosphate + Tween 20 (0.05%)+ 0.1% glycin+10% glycérol. Prélever 100 µL pour les tests BCA. Le reste de la solution est aliquoté et congelé à -80°C.

Les particules luminescentes ainsi préparées selon l'une ou l'autre des méthodes précitées, comprenant les nanoparticules photoluminescentes $Y_{0,6}Eu_{0,4}VO_4$ couplées à un anticorps de ciblage de la substance à analyser (NPs-Ac), peuvent être mises en œuvre pour détecter et/ou quantifier ladite substance dans un échantillon biologique.

**EXEMPLE 2 hors invention**

**Préparation du support à la surface duquel est immobilisée la substance à analyser**

**i. Préparation du support de type lamelles de verre**

Dans un premier temps, on prépare un support formé de lamelles de verre dont la surface est préalablement nettoyée, passivée et fonctionnalisée avec des anticorps biotinylés (anticorps de capture).

Le protocole de nettoyage, passivation et fonctionnalisation des lamelles de verre est le suivant. La fonctionnalisation peut se faire soit par « printing » soit par dépôt de gouttes à l'aide d'un spotter.

Nettoyage des lamelles

- Lavage des lamelles dans de l'eau désionisée avec du détergent Hellmanex II à 4% (Hellma) dans un bac à ultrasons chauffant à 40-50°C pendant 15 minutes.
- Rinçage des lamelles à l'eau désionisée dans un bac à ultrasons chauffant à 40-50°C pendant 15 minutes.
- Rinçage à l'éthanol absolu dans un bac à ultrasons chauffant à 40-50°C pendant 15 minutes.
- Séchage dans un flux de $N_2$ ou sous hotte aspirante pendant 10-15 min.
- Juste avant la fonctionnalisation de la surface de verre, nettoyer les lamelles une par une dans un Plasma cleaner (Harrick Plasma, model PDC-002) pendant 2 minutes à intensité médium.

Passivation des lamelles

La molécule de passivation utilisée est un silane-polyéthylène glycol (PEG) avec du PEG de 10 kDa, commercialisé par la Société Laysanbio sous la référence M-SIL-5K.

La passivation comporte uniquement une étape de silanisation du verre. Plus le PEG sera long, plus la passivation résultante sera meilleure.

Le protocole de passivation est le suivant :

- Dissoudre le PEG à 10 mg/mL dans de l'éthanol absolu (EtOHAbs, concentration molaire 2 mM ou 1 mM suivant la taille du PEG respectivement 5 kDa ou 10 kDa). Pour bien dissoudre les PEG les plus longs à 10 mg/mL, il faut parfois chauffer quelques minutes à 40°C.
- Quelques secondes avant utilisation, rajouter dans cette solution un peu d'$H_2O$ et d'acide acétique (AcAc) pour avoir les proportions v/v/v : EtOHAbs/ $H_2O$ / AcAc : 95/5/0,2.
- Au fond d'une boite hermétique poser un carré de parafilm, puis déposer dessus une goutte -30 µL de la solution préparée ci-dessus, puis la lamelle de verre (issue du plasma cleaner) par-dessus pour prendre la goutte en sandwich. Mettre un petit réservoir d'éthanol dans la boite hermétique pour saturer l'air en éthanol et pour que les 30µL ne sèchent pas. Laisser incuber pendant la nuit.
- Rincer à l'eau désionisée, puis sécher dans un flux d'azote. Pour finir, mettre les lamelles 5 min sur plaque chauffante à 110 °C.

Fonctionnalisation des lamelles par incubation pendant une nuit

Le protocole de fonctionnalisation des lamelles préalablement passivées par les anticorps biotinylés est le suivant. Il s'agit du protocole utilisé pour les résultats montrés dans les figures.

Incubation de la lamelle dans une solution contenant les anticorps de capture pendant une nuit à 4°C. La solution d'anticorps contient 10-25 µg/mL d'anticorps dans une solution de PBS à pH 7,4 ou de tampon carbonate à pH 9 selon les instructions du fournisseur.

Alternativement les deux protocoles suivants peuvent être utilisés :

Fonctionnalisation par « printing » de streptavidine et anticorps biotinylés

- Couper un timbre de PDMS (polydiméthylsiloxane) de dimensions correspondant à la surface que l'on souhaite fonctionnaliser.
- Poser le timbre sur une surface plane. Incuber sur le timbre ~15-30 µL de streptavidine à 20 µg/mL dans un tampon PBS (Phosphate Buffer Saline) pendant 1 min.
- Rincer immédiatement à l'$H_2O$ (à la pissette) et passez sous un flux d'$N_2$ (10 sec).

- Retourner le tampon et poser sa surface encrée sur la lamelle pendant 1 min.
- Enlever le tampon en faisant attention à ne pas le traîner sur la surface.
- Après la fonctionnalisation de la lamelle de verre avec de la streptavidine, incuber la quantité nécessaire (1-10 μg/mL dans du tampon bicarbonate pH 7.4) en anticorps biotinylés pendant 2h à température ambiante ou pendant une nuit à 4 °C.
- Enlever la solution d'incubation d'anticorps puis laver avec 100 μL de tampon PBS à 3 reprises.
- Rajouter 100 μL de BSA 5% w/v (5 g dans 100 mL) PBS et laisser incuber durant 1 h.

Fonctionnalisation par spotter

La fonctionnalisation par spotter est réalisée avec l'appareil SPRi-Arrayer de Horiba selon le protocole du fournisseur en utilisant une aiguille de diamètre 500 μm pour le dépôt de gouttes contenant les anticorps (1-10 μg/mL dans du tampon bicarbonate pH 7,4).

**ii. Immobilisation de la substance à analyser des échantillons à la surface du support**

Le protocole est le suivant.

- Ajouter les échantillons à doser (insuline en solution, insuline dans du sérum ou TSH en solution) dans chaque puits et laisser incuber 2 heures. Ce temps est le même que celui indiqué pour les tests classiques d'ELISA correspondants.
- Après 2 heures d'incubation, enlever la solution puis laver avec 100 μL de tampon Wash (PBS 0,2 % Tween 20 2X) à 3 reprises.

**EXEMPLE 3**

**Détection et quantification ultra-sensible**

**3.1. Association des particules luminescentes avec la substance à analyser**

Le protocole est le suivant :

- Ajouter les nanoparticules couplées aux anticorps biotinylés préparés comme décrit en exemple 1 et laisser incuber pendant 1 heure. Le temps d'incubation nécessaire pour maximiser le signal lors de la mesure de luminescence peut être préalablement déterminé, comme décrit en exemple 4.
- Après 1 heure d'incubation, enlever la solution puis laver avec 100 μL de tampon Wash (PBS 0.2 % Tween 20 2X) à 4 reprises.
- Rajouter 100 μL de PBS et faire la mesure de luminescence, comme décrit ci-après.

**3.2. Mesure de luminescence**

**i. Montage expérimental pour la mesure de la luminescence**

Le montage de détection est le suivant. Il est représenté schématiquement en Figures 2 et Figures 12 et 13.

Le dispositif de détection est composé d'une source (1) laser 1 W de longueur d'onde de 465 nm (ML-6500-465, Modulight ou F465-HS-1W, Laser2000), d'un système de collimation et de diminution de la taille du faisceau laser (2) constitué de deux lentilles (deux lentilles bi-convexes Ø1/2" de f =100 mm et de f=30 mm (Thorlabs)), éventuellement d'un hacheur mécanique (4) (MC2000B-EC, Thorlabs), d'un support (6) de lames pour les échantillons, et d'un système de détection des photons émis.

Pour collecter la luminescence émise par les nanoparticules dans l'échantillon, sont utilisés une lentille à grande ouverture numérique (10) (lentille bi-convexe, Ø =50,8 mm, f=100 mm (Thorlabs)) et deux filtres interférentiels 620 ± 14-25 (FF01-620/14-25, Semrock) pour éliminer spectralement les signaux parasites et un photomultiplicateur (12) (PMM02, Thorlabs).

Un convertisseur analogique-digital (NI9215, National Instrument) permet d'enregistrer le signal grâce à un logiciel Labview.

Tous les éléments de détection sont situés sur un même axe. Ainsi, le montage est à la fois plus ergonomique et plus simple à régler. Un système de translation du support des lames (Z8253, KCH301 Thorlabs) a été mis en œuvre afin de pouvoir observer plusieurs échantillons biologiques successivement par balayage (scanning).

*Détection résolue temporellement pour la mesure de la quantité de nanoparticules en présence de signaux parasites*

Un hacheur mécanique peut être placé sur le trajet du faisceau laser pour créer des créneaux d'excitation laser afin d'éliminer les signaux parasites. En effet, lors de l'illumination de l'échantillon biologique par le faisceau laser, il est possible que d'autres molécules que les nanoparticules d'intérêt émettent de la fluorescence. L'utilisation d'un hacheur mécanique et d'une fréquence de détection du signal par le photomultiplicateur de 100 kHz (une acquisition du signal toute les 10 μs) permet de s'affranchir de cette fluorescence parasite.

Il est ainsi possible du fait de la durée d'émission longue par les particules de l'invention, de faire une détection résolue en temps de l'émission, en particulier une détection retardée de l'émission, comme décrit en détails ci-après.

Une illumination modulée dans le temps permet de limiter la contribution au signal de luminescence d'espèces parasites présentes dans l'échantillon (sérum, sang ...) ou dans les substrats solides utilisés (verre, plastique ...). En effet, les nanoparticules utilisées ($YVO_4$ :Eu ou $GdVO_4$ :Eu par exemple) peuvent être placées (par illumination à 465 nm) dans un état excité de durée de vie longue, de l'ordre de quelques centaines de μs, par rapport aux durées de vie des fluorophores usuels, qui se situent dans le domaine nanoseconde. Ceci permet la séparation temporelle des signaux de luminescence parasites du signal émis par les nanoparticules.

La mise en œuvre de la modulation peut se faire par l'utilisation d'un hacheur mécanique rotatif, de façon à obtenir une illumination périodique, à des fréquences comprises entre 100 et 1000 Hz. La figure 9 représente, de manière schématique, le profil temporel d'illumination obtenu par hachage mécanique du laser d'excitation

Le signal modulé obtenu est l'alternance d'une phase de déclin (fermeture du hacheur) et d'une phase de retour de luminescence (ouverture du hacheur) de l'ensemble des émetteurs présents dans l'échantillon. Le déclin/retour du signal de luminescence est déterminé par deux paramètres distincts : (i) les durées de vie des états excités des émetteurs, et (ii) la dynamique d'occultation/de dévoilement du faisceau excitateur par le hacheur mécanique. La figure 10-a présente des exemples de déclin de luminescence après initiation de la fermeture du hacheur (t=0). L'analyse numérique *a posteriori* du signal permet d'isoler le signal dû aux nanoparticules, suivant par exemple les deux méthodes suivantes.

Méthode 1 : Détection retardée.

Dans la phase de déclin de luminescence, la contribution des émetteurs parasites est concentrée aux temps courts, où l'occultation du faisceau excitateur n'est pas encore complète (figure 10-a). En supprimant cette partie (Figure 10-b, détection restreinte à 170 μs après le début de l'occultation du faisceau d'excitation par la pale du hacheur), on obtient un signal dû exclusivement aux nanoparticules (superposition des courbes (b) et (c), la contribution de la fluorescence parasite de la lamelle, du sérum et de l'eau est éliminée), ce qui permet donc une estimation de leur nombre malgré la présence d'autres émetteurs.

Méthode 2 : Détection résolue en temps

La luminescence des nanoparticules et des fluorophores parasites peut être modélisée par un système à deux niveaux (Figure 11). En ce cas, la forme du signal de luminescence PL(t) peut être déterminée à partir des caractéristiques des émetteurs :

$$PL(t) = k\varphi N^* = G[I(t), k, N, \varphi]$$

où I(t) est le profil temporel d'excitation ; N est le nombre total d'émetteurs d'un type et N* est le nombre d'émetteurs sur l'état excité ; k son taux de désexcitation ; φ son rendement quantique ; en résolvant numériquement le système d'équations différentielles résultant du modèle.

Le signal total peut alors s'écrire,

$$PL(t) = G[I(t), k_{NP}, N_{NP}, \varphi_{NP}] + F[I(t), k_F, N_F, \varphi_F] + BG$$

où les indices NP et F désignent respectivement les nanoparticules et les fluorophores parasites et BG le signal de fond.

L'ajustement du signal enregistré par cette fonction permet alors de déterminer les différents paramètres, notamment $N_{NP}$ et $k_{NP}$ (Figure 11). Celui-ci est robuste, dans la mesure où $k_{NP} << k_F$. La détermination du nombre de nanoparticules accrochées à la surface $N_{NP}$ est alors effectuée sans ambiguïté, malgré la présence d'émetteurs parasites dans l'échantillon, contribuant au signal brut. En pratique, la cinétique d'occultation mécanique de l'illumination aux fréquences utilisées (100-1000 Hz) est significativement plus lente que le déclin de fluorescence des émetteurs parasites $k_F \sim 10^9$ $s^{-1}$, de sorte que $F[I(t), k_F, N_F, \varphi_F] \sim K.I(t)$.

La mesure de $N_{NP}$ est alors effectuée par la méthode suivante : (i) détermination expérimentale du profil temporel

d'illumination I(t)/I(0) par la mesure de l'autofluorescence d'un échantillon de calibration sans nanoparticules ; (ii) mesure du signal de luminescence de l'échantillon cible et (iii) ajustement non-linéaire (méthode des moindres carrés) de ce signal par une fonction $PL(t) = M.\frac{I(t)}{I(0)} + G[I(t), k_{NP}, N_{NP}, \varphi_{NP}] + BG$. Cet ajustement permet l'identification des paramètres M, $k_{NP}$ et $\varphi_{NP}N_{NP}$ à un facteur multiplicatif instrumental près : ce dernier paramètre indique alors, pour des conditions de détection fixées, la quantité de nanoparticules déposées à la surface.

Variantes du montage expérimental

Les nanoparticules sont excitées :

. soit en utilisant un fort angle d'incidence (angle entre la direction de propagation du faisceau laser et la verticale au support de l'échantillon) compris entre 60° et 63° (comme représenté schématiquement en Figure 12),
. soit en utilisant un montage d'illumination de type TIRF (fluorescence par réflexion totale interne) notamment à l'aide d'un parallélépipède de plexiglas 13 d'indice de réfraction supérieur ou égal à celui de la lame de verre qui sert de support d'échantillon (comme représenté schématiquement en Figure 13). Cette configuration permet d' obtenir un angle d'incidence sur l'interface verre/eau ou verre/sérum supérieur à 61,04° ce qui produit une réflexion totale du faisceau à cette interface et une excitation par onde évanescente des nanoparticules ancrées à la substance à analyser.

Trois variantes du montage expérimental ont été utilisées.
*Variante 1* : Détection en réflexion (vers le bas) sans hacheur (sauf lorsque cela est précisé) et sans excitation par onde évanescente (Figure 12-a). Dans la majorité des cas, le temps d'acquisition est fixé à 30 s avec une valeur de voltage enregistrée toutes les 100 ms. Le diamètre de la lentille de collection est de 30 mm au lieu de 50,8 mm.
*Variante 2* : Détection en transmission (vers le haut) (Figure 12-b). Il est possible d'effectuer des mesures avec ou sans hacheur et avec ou sans excitation par onde évanescente. Le temps d'acquisition est fixé à 1 s avec une valeur de voltage enregistrée toutes les 10 μs. Cette variante comporte le système de translation du support des lames (Z8253, KCH301 Thorlabs).
*Variante 3* : Montage transportable avec détection en transmission (vers le bas). Il est possible d'effectuer des mesures avec ou sans hacheur et avec ou sans excitation par onde évanescente. Le temps d'acquisition est fixé à 1 s avec une valeur de voltage enregistrée toutes les 10 μs.
Pour chaque concentration d'échantillon à détecter, plusieurs mesures (N mesures, N supérieur ou égal 5) à différentes positions de la lamelle sont effectuées. Chaque mesure est la moyenne de 100 000 valeurs enregistrées pendant 1 s avec un taux d'acquisition de 100 kHz (1 valeur de voltage enregistrée toutes les 10 μs) sauf dans le cas de la variante 1 du montage expérimental où le temps d'acquisition était de 30 s avec une valeur de voltage enregistrée toutes les 100 ms.
La valeur du signal indiquée sur les graphes et sa barre d'erreur correspondent, respectivement, à la moyenne des N mesures et à leur écart-type. Dans la plupart des cas, la valeur du signal pour une concentration de la molécule à détecter égale à zéro, a été soustraite à toutes les valeurs mesurées pour les différentes concentrations. Ainsi la valeur du signal pour une concentration égale à zéro, apparaît à zéro. Cependant, l'écart-type indiqué permet de se prononcer sur la capacité à détecter une concentration donnée. Typiquement, on considère que la limite de détection est déterminée par la concentration générant un signal égal à 3 fois l'écart-type du signal obtenu à concentration nulle (« blanc »). La limite de quantification est déterminée par la concentration générant un signal 10 fois supérieur à cet écart-type à concentration nulle.
Pour les expériences réalisées avec une excitation en onde évanescente, une collection de la luminescence par le haut est préférable. En effet, la luminescence émise par l'échantillon vers le bas est réfractée vers des grands angles par le parallélépipède en plexiglas et, de ce fait, une plus faible fraction est collectée par la lentille de collection en présence du parallélépipède permettant de réaliser l'excitation en onde évanescente.

**ii. Calibration de l'appareil de détection**

Préalablement aux mesures, l'appareil de détection a été calibré avec des nanoparticules en solution, suivant les variantes 2 (détection vers le haut avec hacheur et avec excitation par onde évanescente) et 3 (détection vers le bas, sans hacheur et sans excitation par onde évanescente).
Le protocole de calibration est le suivant :
Les lamelles de verres sont activées au préalable au plasma cleaner.

- dépôt d'une solution de nanoparticules diluée dans du PBS de concentration connue sur la lamelle de verre ;

- incubation pendant 2 heures ;
- rinçage au moins 3 fois avec de l'eau ultra-pure.

Le temps d'acquisition est fixé à 1 s avec une valeur de voltage enregistrée toutes les 10 μs.
Les courbes de calibration de l'appareil de détection obtenues suivant les deux variantes sont représentées en Figure 14-a et 14-b.

**iii. Résultats des essais de détection/quantification**

**Détection et quantification d'une substance dans un échantillon**

La concentration d'une substance dans un échantillon est détectable lorsque le signal obtenu est au moins trois fois supérieur à l'écart-type du signal pour un échantillon de même composition contenant une concentration nulle de la substance.
Pour réaliser la quantification de la substance à analyser (*i.e.* déterminer sa concentration), le protocole suivant doit être mis en œuvre :

i) réaliser une série de mesures de calibration avec la substance à analyser à différentes concentrations connues, par exemple à partir de substances obtenues dans le commerce ou à partir d'une purification. Dans la mesure du possible, les échantillons de calibration doivent être préparés avec la même composition que les échantillons à mesurer ou avec une composition la plus proche possible. Faire un ajustement des points obtenus (signal en mV *versus* concentration de la substance à analyser) ;
ii) réaliser les mesures des échantillons à analyser (obtention de la valeur du signal en mV) ;
iii) attribuer à chaque échantillon mesuré une valeur de concentration de la substance à partir du signal mesuré (en mV) et à partir de la courbe de calibration réalisée en étape i) et son ajustement.

La concentration d'une substance dans un échantillon est quantifiable lorsque le signal obtenu est au moins dix fois supérieur à l'écart-type du signal pour un échantillon de même composition contenant une concentration nulle de la substance.

**Détection de l'insuline**

Les échantillons analysés sont soit des solutions d'insuline recombinante dans du PBS + 5% de BSA ou d'insuline dans du sérum (échantillons fournis par Cerba Specimen Services avec des concentrations d'insuline déterminées préalablement par des techniques de référence).
Pour les échantillons d'insuline recombinante en solution dans du PBS + 5% de BSA, l'insuline recombinante fournie par le kit ELISA pour les expériences de calibration associées a été utilisée. Les différents échantillons ont été préparés par dilutions successives selon le protocole indiqué par le fournisseur du kit.
Pour les échantillons d'insuline dans du sérum, les solutions ont été utilisées telles quelles pour les plus fortes concentrations. Des échantillons de très faibles concentrations n'étant pas disponibles, ils ont été préparés en diluant dans du PBS + 5% de BSA les échantillons contenant les concentrations les plus faibles disponibles.

Détection par ELISA

A titre de comparatif, la détection de l'insuline recombinante a été opérée en solution par un kit ELISA dans une plaque de 96 puits. Les conditions expérimentales suivies sont celles indiquées par le fournisseur du kit ELISA. L'absorbance du puits qui n'a pas été incubé avec de l'insuline (concentration zéro) a été soustraite des valeurs d'absorbance mesurées pour les puits ayant été incubés avec les différentes concentrations d'insuline. Deux puits ont été utilisés pour chaque valeur de concentration et l'écart-type pour ces deux mesures est indiqué comme la barre d'erreur dans la figure 15. La concentration la plus faible mesurée est de 187 pg/mL (ou 33 pM). L'écart-type pour la concentration zéro est de 0,0035. La valeur d'absorbance mesurée pour la concentration 187 pg/mL (ou 33 pM) est de 0,0085, juste en dessous de la valeur limite de 0,0105 égale à 3 fois l'écart-type déterminé pour la concentration zéro. Selon les spécifications du fournisseur (ABCAM Réf. ab100578), la détection par un kit ELISA classique ne permet pas de détecter des concentrations en-deçà de 50 pg/mL (ou 9 pM) (Figure 15).

Détection ultra-sensible selon l'invention

- La Figure 16 montre le signal obtenu pour des échantillons d'insuline recombinante en solution pour différentes concentrations, la concentration la plus faible étant de 0,05 pg/mL (ou 9 fM), avec la variante 1 de l'appareil de détection. Le temps d'acquisition est de 30 secondes, avec une valeur de voltage enregistrée toutes les 100 ms.

Dans l'encart de la Figure 16, le signal sans insuline a été soustrait.
La concentration minimale pouvant être détectée est ainsi 1000 fois plus faible que la concentration détectable par ELISA (50 pg/mL ou 9 pM) en utilisant les mêmes anticorps que le kit ELISA.

- La Figure 17 montre le signal obtenu pour des échantillons d'insuline recombinante en solution pour différentes concentrations, la concentration la plus faible étant de 0,05 pg/mL (ou 9 fM), avec la variante 2 de l'appareil de détection (détection par le haut), sans hacheur et sans excitation par onde évanescente. Le temps d'acquisition est de 1 seconde, avec une valeur de voltage enregistrée toutes les 10 $\mu$s.

La concentration minimale détectée est ainsi 1000 fois plus faible que la concentration détectable par ELISA (50 pg/mL ou 9 pM) en utilisant les mêmes anticorps que le kit ELISA.

- La Figure 18 montre le signal obtenu pour des échantillons d'insuline recombinante en solution pour différentes concentrations, la concentration la plus faible étant de 0,05 pg/mL (ou 9 fM), avec la variante 2 de l'appareil de détection (détection par le haut), avec hacheur et avec excitation par onde évanescente. Le temps d'acquisition est de 1 seconde, avec une valeur de voltage enregistrée toutes les 10 $\mu$s.

La concentration minimale détectée est ainsi 1000 fois plus faible que la concentration détectable par ELISA (50 pg/mL ou 9 pM) en utilisant les mêmes anticorps que le kit ELISA.

- La Figure 19 montre le signal obtenu pour des échantillons de sérum contenant de l'insuline (échantillons fournis par CERBA Specimen Services et dilués dans du PBS + 5% BSA pour les concentrations les plus faibles. Pour obtenir les échantillons aux concentrations les plus faibles, l'échantillon contenant de l'insuline à environ 8 pM a été dilué sur plaque multipuits IBIDI, avec la variante 1 de l'appareil de détection.

Le temps d'acquisition est de 30 secondes, avec une valeur de voltage enregistrée toutes les 100 ms.
La concentration minimale détectée est de 9 fM (ou 0,05 pg/mL), soit 1000 fois plus faible que la concentration détectable par ELISA (50 pg/mL ou 9 pM) en utilisant les mêmes anticorps que le kit ELISA.

**Détection de la TSH**

Les échantillons analysés sont des solutions de TSH recombinante de poids moléculaire 15 639 Da dans du PBS + 5% de BSA. Les anticorps utilisés sont ceux du kit ABCAM Ref. ab 100660.

Détection ultra-sensible selon l'invention

La Figure 20 montre le signal obtenu pour des échantillons de TSH recombinante en solution pour différentes concentrations, la concentration la plus faible étant de 3,2 fM (1,4 fg/mL), avec la variante 1 de l'appareil de détection avec hacheur. Le temps d'acquisition est de 1 seconde, avec une valeur de voltage enregistrée toutes les 10 $\mu$s.
En comparaison, la concentration minimale pouvant être détectée par le kit ELISA est selon le fournisseur typiquement moins de 4 pg/mL. Ainsi, la concentration minimale détectée selon l'invention est plus de 1000 fois plus faible que la concentration détectable par le kit ELISA.

**EXEMPLE 4 hors invention**

**Ajustement du temps d'incubation**

Le temps d'incubation nécessaire pour maximiser le signal dans le cas de la détection de l'insuline recombinante en solution (50 pg/mL d'insuline) peut être préalablement déterminé par des mesures de luminescence obtenues pour différents temps d'incubation entre les supports d'échantillon comportant la substance à analyser (ici l'insuline recombinante) immobilisée sur leurs surfaces (immobilisation effectuée selon le protocole décrit en exemple 2ii) et les conjugués anticorps-nanoparticules (obtenus selon le protocole décrit en exemple 1 avec 10 $\mu$g/mL d'anticorps), étape effectuée selon le protocole décrit en exemple 3.i.
Les mesures ont été faites avec la variante 1 de l'appareil de détection décrite en exemple 3. Le temps

d'acquisition est de 30 secondes, avec une valeur de voltage enregistrée toutes les 100 ms.

La Figure 21 représente l'évolution du signal de luminescence détecté en fonction du temps d'incubation.

Il apparaît nécessaire de laisser incuber les nanoparticules couplées aux anticorps avec la surface sur laquelle est immobilisée la substance à analyser (ici l'insuline recombinante) pendant 45 minutes minimum pour maximiser le signal de luminescence détecté. Ce temps nécessaire peut varier selon la substance à analyser et l'anticorps utilisé.

**EXEMPLE 5 hors invention**

**Expérience de multiplexage spatial**

Pour illustrer la possibilité d'effectuer une détection "multiplexée" selon le procédé de l'invention (schéma de principe du multiplexage spatial représenté en Figure 22), l'expérience suivante a été réalisée.

Deux zones ou « spots » contenant des nanoparticules ont été créés en déposant deux gouttes de deux solutions de nanoparticules telles qu'obtenues suivant le protocole décrit en exemple 1 (partie 1.1, nanoparticules telles qu'obtenues après synthèse) de deux concentrations différentes (4 mM et 8 mM) sur des lamelles en verre nettoyées suivant le protocole décrit en exemple 2. Ici les gouttes contenant les nanoparticules ont été laissées sécher avant le début des mesures de luminescence, sans étape de rinçage.

Le schéma en Figure 23 indique le diamètre du faisceau laser au niveau de l'échantillon selon la direction de déplacement (3 mm), le diamètre (2 mm) et la distance entre les deux « spots « de nanoparticules (1 mm).

Les mesures sont effectuées avec la variante 2 du montage expérimental décrite en exemple 3 (détection vers le haut, avec hacheur et sans excitation par onde évanescente). Grâce au système de déplacement motorisé du porte-échantillon, le signal émis par les nanoparticules a été détecté à plusieurs localisations : avant le premier « spot » (*position 1*), dans le premier « spot » (*position 4*), entre les deux « spots » (*position 6*), dans le deuxième « spot » (*position 7*) et enfin après le deuxième « spot » (position 9). Pour chaque position du faisceau laser excitateur, le temps d'acquisition est de 1 seconde, avec une valeur de voltage enregistrée toutes les 10 $\mu$s.

La Figure 23 représente le signal de luminescence détecté pour les différentes localisations précitées. Les deux pics de localisations et d'amplitudes différentes montrent la possibilité de différencier des dépôts spatialement organisés, comme cela est nécessaire pour une mesure multiplexée.

**EXEMPLE 6 hors invention**

**Synthèse des nanoparticules $YVO_4$ :Dy 3 %**

Le protocole utilisé est même que celui de la synthèse des nanoparticules $Y_{0,6}Eu_{0,4}VO_4$ indiqué au point 1.1. de l'exemple 1 ci-dessus, avec comme seule différence qu'à la place du précurseur $Eu(NO_3)_3$, nous utilisons le précurseur $Dy(NO_3)_3$ à une concentration de 0,03 M.

Résultat

La figure 26 montre les spectres d'excitation de luminescence et d'émission d'une solution de ces nanoparticules à l'issue de leur synthèse. Le spectre d'excitation fait apparaître des pics d'excitation directe des ions $Dy^{3+}$. Les étapes suivantes de fonctionnalisation et de couplage à la streptavidine (exemple 1, point 1.2.), puis à un agent de ciblage biotinylé (exemple 1, point 1.3.), ou le couplage direct à un agent de ciblage (exemple 1, point 1.4.), peuvent être reproduites à l'identique avec ces nanoparticules produisant ainsi des sondes émettant à une longueur d'onde d'émission différente.

**EXEMPLE 7 hors invention**

**Synthèse des nanoparticules $YVO_4$ :Sm 3 %**

Le protocole utilisé est même que celui de la synthèse des nanoparticules $Y_{0,6}Eu_{0,4}VO_4$ indiqué au point 1.1. de l'exemple 1 ci-dessus, avec comme seule différence qu'à la place du précurseur $Eu(NO_3)_3$, nous utilisons le précurseur $Sm(NO_3)_3$ à une concentration de 0,03 M.

Résultat

La figure 27 montre les spectres d'excitation de luminescence et d'émission d'une solution de ces nanoparticules à l'issue de leur synthèse. Le spectre d'excitation fait apparaître des pics d'excitation directe des ions $Sm^{3+}$. Les étapes

suivantes de fonctionnalisation et de couplage à la streptavidine (exemple 1, point 1.2.), puis à un agent de ciblage biotinylé (exemple 1, point 1.3.), ou le couplage direct à un agent de ciblage (exemple 1, point 1.4.), peuvent être reproduites à l'identique avec ces nanoparticules produisant ainsi des sondes émettant à une longueur d'onde d'émission différente.

Références

[1] Hermanson, Bioconjugate Techniques, Academic Press, 1996 ;
[2] Mason, Fluorescent and Luminescent Probes for Biological Activity. A Practical Guide to Technology for Quantitative Real-Time Analysis, Second Edition, Academic Press, 1999 ;
[3] Medintz et al., Nat. Mat., 2005, 4, pp. 435-446;
[4] Nam et al., Science, 2003, 301, pp. 1884-1886 ;
[5] H. Li et al., Analyst, 2011, 136, pp. 1399-1405 ;
[6] Z. Cao et al., Anal. Chim. Acta, 2011, 698, pp. 44-50 ;
[7] Pisanic et al., Analyst, 2014, 139(12), pp. 2968-2981 ;
[8] Geissler et al., Angewandte Chemie International Edition, 2010, 49(8), pp. 1396-1401 ;
[9] Howes et al., Bionanotechnology, 2014, 346(6205), pp.53-63 ;
[10] Giljohann et al., Angewandte Chem, 2010, 49, pp. 3280-3294 ;
[11] De La Rica et al., Nature Nanotechnology, 2012, 7, pp. 821-824;
[12] Zhu et al., Anal. Chem., 2013, 85(2), pp 1058-1064;
[13] Cordeiro et al., Diagnostics, 2016, 6(4):43;
[14] Riwotzki et al., J. Phys. Chem. B, 1998, 105, pp. 12709-127 ;
[15] Huignard et al., Chem. Mater., 2000, 12, pp. 1090-1094 ;
[16] Bouzigues et al., ACS Nano, 2011, 5(11) pp. 8488-8505 ;
[17] Dosev et al., J. Biomed. Opt., 2005, 10(6), 064003 ;
[18] Yi et al., Nano Letters, 2004, 4(11), pp. 2191-2196 ;
[19] Beaurepaire et al., Nano Letters, 2004, 4(11), pp. 2079-2083 ;
[20] Turkcan et al., Biophysical Journal, 2012, 102, pp. 2299-2308;
[21] Yuan et al., Trends in Analytical Chemistry, 2006, 25(5), pp.490-500 ;
[22] Tang et al., Clin Vaccine Immunol, 2009, 16(3), pp. 408-413 ;
[23] Härmä et al., 2001, Clinical Chemistry, 47(3), pp. 561-568 ;
[24] Corstjens et al., Clinical Biochemistry, 2008, 41(6) pp. 440-444 ;
[25] Hemmilä et al., Analytical Biochemistry, 1984, 137(2), pp. 335-343 ;
[26] Jiang et al., Journal of Fluorescence, 2010, 20(1), pp 321-328 ;
[27] Tanja et al., Analytical and Bioanalytical Chemistry, 2004, 380(1), pp 24-30 ;
[28] Zhou et al., Angewandte Chimie, 2014, 126(46), pp. 12706-12710 ;
[29] Riwotzki et al. J. Phys. Chem. B, 1998, 102, 10129 ;
[30] Meza et al. J. Phys. Chem. A, 2014, 118, 1390 ;
[31] Dordevic et al. J. Res. Phys., 2013, 37, 47 ;
[32] Casanova et al., J. Am. Chem. Soc., 2007, 129, 12592 ;
[33] Giaume et al., Langmuir, 2008, 24, pp. 11018-11026 ;
[34] Abdesselem et al., ACS Nano, 2014, 8(11), pp. 11126-11137 ;
[35] Casanova et al., Appl. Phys. Lett, 2006, 89, 253103 ;
[36] Son et al., Journal of Nanoscience and Nanotechnology, vol. 8, 2463-2467, 2008 ;
[37] Nichkova et al., Anal. Chem. 2005, 77, 6864-6873.


## Revendications

**1.** Utilisation pour la quantification ultra-sensible *in vitro* d'une teneur strictement inférieure à 10 pM d'une substance d'intérêt biologique ou chimique dans un échantillon, de nanoparticules inorganiques photoluminescentes,
formées en tout ou partie d'une nanoparticule inorganique photoluminescente formée d'une matrice cristalline présentant au moins $10^3$ ions de terres rares, et couplées à au moins un agent de ciblage de la substance à analyser, lesdites nanoparticules présentant une taille moyenne supérieure ou égale à 20 nm et strictement inférieure à 1 µm, et étant aptes à émettre de la luminescence après absorption d'un photon ; ladite utilisation mettant en outre en œuvre

(i) une excitation des ions de terres rares des particules associées avec la substance à analyser, par un dispositif d'illumination, en particulier de type laser, d'une puissance d'au moins 50 mW, de préférence d'au moins 500 mW, et d'une intensité d'excitation d'au moins 1 $W/cm^2$, de préférence d'au moins 10 $W/cm^2$ ;
(ii) une détection de l'émission de luminescence par les particules après une absorption à un photon, et
(iii) la détermination de la concentration de la substance par interprétation de ladite mesure de luminescence, par référence à un étalon ou étalonnage et dans laquelle les nanoparticules sont de formule (I) suivante :

$$(A_{1-x}Ln_x)_a(M_pO_q) \qquad (I)$$

dans laquelle :

- M représente un ou plusieurs éléments choisis parmi V, P, W, Mo, As, Al, Hf, Zr, Ge, Ti, Sn, Mn et Si, ;
- Ln correspond à un ou plusieurs ion(s) lanthanide(s) luminescent(s) choisis parmi l'europium (Eu), le dysprosium (Dy), le samarium (Sm), le praséodymium (Pr), le néodyme (Nd), l'erbium (Er), l'ytterbium (Yb), le cérium (Ce), l'holmium (Ho), le terbium (Tb), le thulium (Tm) et leurs mélanges
- A correspond à un ou plusieurs ion(s) constituant(s) de la matrice cristalline dont les niveaux électroniques ne sont pas impliqués dans le processus de luminescence et choisi parmi l'yttrium (Y), le gadolinium (Gd), le lanthane (La), le bismuth (Bi), le lutécium (Lu) et leurs mélanges,
- $0 < x < 1$, en particulier $0,1 \leq x \leq 0,9$, en particulier $0,2 \leq x \leq 0,6$, notamment $0,2 \leq x \leq 0,4$ et plus particulièrement x vaut 0,4 et
- les valeurs de p, q et a sont telles que l'électroneutralité de $(A_{1-x} Ln_x)_a(M_pO_q)$ est respectée.

**2.** Utilisation selon la revendication précédente, dans laquelle la substance à analyser dudit échantillon est immobilisée à la surface d'un support, ladite surface étant passivée de manière à ce que lesdites particules luminescentes ne s'y fixent pas en l'absence de la substance à analyser.

**3.** Utilisation selon la revendication 1 ou 2, pour la quantification d'une substance d'intérêt présente dans l'échantillon en une teneur inférieure à 1 pM, voire inférieure à 0,1 pM, ou encore inférieure à 0,01 pM.

**4.** Utilisation selon l'une quelconque des revendications précédentes, dans laquelle l'échantillon est un échantillon biologique, en particulier un échantillon de prélèvement humain, et plus particulièrement choisi parmi le sang, le sérum, le plasma, la salive, l'urine, le liquide cérébro-spinal, le frottis nasal, le frottis vaginal, l'expectoration et la matière fécale diluée.

**5.** Utilisation selon l'une quelconque des revendications précédentes, pour la quantification de biomarqueurs, d'anticorps, d'ADN et/ou d'ARN dans un échantillon biologique.

**6.** Utilisation selon l'une quelconque des revendications précédentes, dans laquelle ledit agent de ciblage est choisi parmi un anticorps polyclonal ou monoclonal, un fragment d'anticorps, un nanobody, un oligonucléide, un peptide, une hormone, un ligand, une cytokine, un peptidomimétique, une protéine, un carbohydrate, une protéine modifiée chimiquement, un acide nucléique modifié chimiquement, un carbohydrate modifié chimiquement qui cible une protéine de surface cellulaire connue, un aptamère, un assemblage de protéines et d'ADN/ARN ou un chloroalcane utilisé par les marquages de type HaloTag, en particulier est un anticorps ou fragment d'anticorps.

**7.** Utilisation selon l'une quelconque des revendications précédentes, dans laquelle la nanoparticule présente une cristallinité imparfaite.

**8.** Utilisation selon l'une quelconque des revendications précédentes, dans laquelle les nanoparticules présentent une durée de vie d'émission supérieure ou égale à 5 $\mu$s, en particulier supérieure ou égale à 10 $\mu$s, notamment supérieure ou égale à 20 $\mu$s, voire supérieure ou égale à 50 $\mu$s.

**9.** Utilisation selon l'une quelconque des revendications précédentes, dans laquelle les nanoparticules présentent une taille moyenne comprise entre 20 nm et 500 nm, de préférence entre 20 nm et 200 nm et notamment entre 20 et 100 nm, en particulier comprise entre 25 nm et 100 nm, notamment comprise entre 30 et 60 nm.

**10.** Utilisation selon l'une quelconque des revendications précédentes, dans laquelle les ions lanthanides sont Eu.

**11.** Utilisation selon l'une quelconque des revendications précédentes, dans laquelle A représente Y ;

**12.** Utilisation pour la quantification ultra-sensible *in vitro* d'une teneur strictement inférieure à 10 pM d'une substance d'intérêt biologique ou chimique dans un échantillon, de nanoparticules inorganiques photoluminescentes, formées en tout ou partie d'une nanoparticule inorganique photoluminescente formée d'une matrice cristalline présentant au moins $10^3$ ions de terres rares, et couplées à au moins un agent de ciblage de la substance à analyser, lesdites nanoparticules présentant une taille moyenne supérieure ou égale à 20 nm et strictement inférieure à 1 $\mu$m, et étant aptes à émettre de la luminescence après absorption d'un photon ; ladite utilisation mettant en outre en œuvre

(i) une excitation des ions de terres rares des particules associées avec la substance à analyser, par un dispositif d'illumination, en particulier de type laser, d'une puissance d'au moins 50 mW, de préférence d'au moins 500 mW, et d'une intensité d'excitation d'au moins 1 W/cm$^2$, de préférence d'au moins 10 W/cm$^2$ ;
(ii) une détection de l'émission de luminescence par les particules après une absorption à un photon, et
(iii) la détermination de la concentration de la substance par interprétation de ladite mesure de luminescence, par référence à un étalon ou étalonnage et dans laquelle les nanoparticules sont de formule (II) suivante :

$$A_{1-x}Ln_x\,(VO_4)_{(1-y)}(PO_4)_y \qquad (II)$$

dans laquelle :

. A est choisi parmi l'yttrium (Y), le gadolinium (Gd), le lanthane (La), le lutécium (Lu) et leurs mélanges, en particulier A représente Y ;
. Ln est choisi parmi l'europium (Eu), le dysprosium (Dy), le samarium (Sm), le néodyme (Nd), l'erbium (Er), l'ytterbium (Yb), le thulium (Tm), le terbium (Tb) et leurs mélanges, en particulier Ln représente Eu ;
. $0 < x < 1$; en particulier $0{,}2 \leq x \leq 0{,}6$ et plus particulièrement x vaut 0,4 ; et
. $0 \leq y < 1$, en particulier y vaut 0.

**13.** Utilisation selon la revendication 12, dans laquelle lesdites nanoparticules de formule (II) présentent en surface des cations tétraalkylammonium, en particulier des cations de formule $NR_4^+$ avec R, identiques ou différents, représentant un groupe $C_1$-$C_6$-alkyl, en particulier $C_1$-$C_4$-alkyl, plus particulièrement $C_1$-$C_3$-alkyl, et notamment choisis parmi les cations tétraméthylammonium, tétraéthylammonium, tétrapropylammonium, tétrabutylammonium et leurs mélanges, de préférence lesdits cations tétraalkylammonium sont des cations tétraméthylammonium.

**14.** Utilisation selon la revendication précédente, lesdites nanoparticules étant de formule (II')

$$A_{1-x}Ln_x(VO_4)_{(1-y)}(PO_4)_y\,.\,(NR_4^+)_z \qquad (II')$$

dans laquelle :

. A est tel que défini en revendication 12 ;
. Ln est tel que défini en revendication 12 ;
. $0<x<1$ ; en particulier $0{,}2 \leq x \leq 0{,}6$ et plus particulièrement x vaut 0,4 ;
. $0 \leq y<1$, en particulier y vaut 0 ;
. R, identiques ou différents, représentent un groupe $C_1$-$C_6$-alkyl, en particulier $C_1$-$C_4$-alkyl, notamment $C_1$-$C_3$-alkyl, et plus particulièrement méthyle ; et
. z représente le nombre de cations tétraalkylammonium $NR_4^+$ localisés à la surface de ladite nanoparticule, en particulier z est compris entre 100 et 10000.

**15.** Utilisation selon l'une quelconque des revendications précédentes, dans laquelle lesdites nanoparticules sont de formule $Y_{1-x}Eu_xVO_4$, dans laquelle $0<x<1$, en particulier lesdites nanoparticules présentant en surface des cations tétraalkylammonium, en particulier tels que définis selon la revendication 13.

**16.** Utilisation selon l'une quelconque des revendications précédentes, dans laquelle les nanoparticules présentent à l'issue de leur synthèse un potentiel zêta, noté $\zeta$, inférieur ou égal à -28 mV, de préférence inférieur ou égal à -30 mV, dans un milieu aqueux de pH $\geq$ 5, en particulier de pH $\geq$ 5,5, et plus particulièrement de pH $\geq$ 6, et de conductivité ionique strictement inférieure à 100 $\mu$S.cm$^{-1}$.

**17.** Utilisation selon l'une quelconque des revendications 2 à 16, dans laquelle ledit support est de type lamelles, plaques multi-puits, microplaques, gels membranes, bandelettes ou microcanaux.

**18.** Utilisation selon l'une quelconque des revendications 2 à 17, dans laquelle l'excitation (i) est opérée avec un faisceau d'excitation laser orienté de sorte à former un angle d'incidence avec la verticale du support présentant en surface lesdites particules associées à la substance à analyser, supérieur ou égal à 55°.

**19.** Utilisation selon l'une quelconque des revendications 2 à 18, dans laquelle l'excitation des particules est opérée par onde évanescente, en particulier à l'aide d'un montage d'illumination laser de type TIRF, permettant un angle d'incidence du faisceau excitateur supérieur ou égal à 61° dans le cas d'une interface verre/eau entre le support et l'échantillon à analyser.

**20.** Utilisation selon l'une quelconque des revendications précédentes, dans laquelle la durée de vie d'émission par les nanoparticules est supérieure ou égale à 1 $\mu$s, en particulier supérieure ou à égale à 50 $\mu$s, la détection de l'intensité lumineuse (ii) comprenant une détection résolue en temps de l'émission, en particulier une détection retardée du signal émis par les particules photoluminescentes.

**21.** Utilisation selon l'une quelconque des revendications précédentes, pour la quantification simultanée d'au moins deux substances différentes et présentes à des teneurs strictement inférieures à 10 pM dans un échantillon.

**22.** Utilisation selon la revendication précédente, dans laquelle les substances à analyser dudit échantillon sont immobilisées en des zones prédéfinies et distinctes à la surface d'un support, ladite surface étant passivée de manière à ce que les particules photoluminescentes ne s'y fixent pas en l'absence des substances à analyser.

**23.** Utilisation selon la revendication 21 ou 22, d'au moins deux types de nanoparticules ayant des longueurs d'ondes d'émission distinctes et couplées à des agents de ciblage de chacune des substances à analyser.

**24.** Utilisation selon l'une quelconque des revendications précédentes, dans laquelle le dispositif d'illumination laser (i) comprend un montage optique, en particulier un système d'au moins une lentille, disposé sur la trajectoire du faisceau laser de manière à contrôler la taille du faisceau au niveau de la zone du support présentant les particules associées à la substance à analyser.

**25.** Utilisation selon l'une quelconque des revendications précédentes, dans laquelle la détection de l'émission de luminescence (ii) est opérée par un dispositif de détection de l'intensité lumineuse comprenant un détecteur unique, par exemple de type photomultiplicateur, photodiode, photodiode à avalanche, ou un détecteur de type réseau de dispositifs photosensibles consistant en une surface 2D de pixels de détection tel qu'une caméra CCD ou EM-CCD ou caméra CMOS.

**26.** Utilisation selon la revendication précédente, dans laquelle le dispositif de détection comprend un montage optique, en particulier un système d'au moins une lentille à grande ouverture numérique, pour focaliser l'émission de luminescence vers le détecteur, en particulier vers le photomultiplicateur.

**Patentansprüche**

**1.** Verwendung zur ultraempfindlichen In-vitro-Quantifizierung eines Gehalts von streng kleiner als 10 pM einer biologischen oder chemischen interessierenden Substanz in einer Probe mittels fotolumineszierender anorganischer Nanopartikel,
die ganz oder teilweise aus einem fotolumineszierenden anorganischen Nanopartikel bestehen, das aus einer kristallinen Matrix besteht, die mindestens $10^3$ Seltenerdionen aufweist, und die an mindestens ein Targeting-Mittel der zu analysierenden Substanz gekoppelt sind, wobei die Nanopartikel eine mittlere Größe größer als oder gleich 20 nm und streng kleiner als 1 $\mu$m aufweisen und dazu geeignet sind, nach der Absorption eines Photons Lumineszenz zu emittieren; wobei bei der Verwendung außerdem Folgendes angewandt wird:

(i) eine Anregung der Seltenerdionen der mit der zu analysierenden Substanz verbundenen Partikel durch eine Beleuchtungsvorrichtung, insbesondere vom Laser-Typ, mit einer Leistung von mindestens 50 mW, vorzugsweise mindestens 500 mW, und einer Anregungsintensität von mindestens 1 W/cm$^2$, vorzugsweise mindestens 10 W/cm$^2$;
(ii) ein Nachweis der Lumineszenzemission durch die Partikel nach einer Ein-Photonen-Absorption und
(iii) die Bestimmung der Konzentration der Substanz durch eine Interpretation der Lumineszenzmessung durch die Bezugnahme auf einen Standard oder eine Kalibrierung und wobei die Nanopartikel die folgende Formel (I):

$$(A_{1-x}Ln_x)_a(M_pO_q) \qquad (I)$$

aufweisen, wobei:

- M für ein oder mehrere Elemente steht, die aus V, P, W, Mo, As, Al, Hf, Zr, Ge, Ti, Sn, Mn und Si ausgewählt sind;
- Ln einem oder mehreren lumineszierenden Lantanoidionen entspricht, die aus Europium (Eu), Dysprosium (Dy), Samarium (Sm), Praseodym (Pr), Neodym (Nd), Erbium (Er), Ytterbium (Yb), Cer (Ce), Holmium (Ho), Terbium (Tb), Thulium (Tm) und Mischungen davon ausgewählt sind
- A einem oder mehreren einen Bestandteil der kristallinen Matrix bildenden Ionen entspricht, deren Elektronenniveaus nicht am Lumineszenzprozess beteiligt sind und die aus Yttrium (Y), Gadolinium (Gd), Lanthan (La), Wismut (Bi), Lutetium (Lu) und Mischungen davon ausgewählt sind,
- $0 < x < 1$, insbesondere $0{,}1 \leq x \leq 0{,}9$, insbesondere $0{,}2 \leq x \leq 0{,}6$, im Besonderen $0{,}2 \leq x \leq 0{,}4$ und noch spezifischer x 0,4 beträgt und
- die Werte von p, q und a derart sind, dass die Elektroneutralität von $(A_{1-x}Ln_x)_a(M_pO_q)$ eingehalten wird.

**2.** Verwendung nach dem vorhergehenden Anspruch, wobei die zu analysierende Substanz der Probe auf der Oberfläche eines Trägers immobilisiert wird, wobei die Oberfläche so passiviert ist, dass die lumineszierenden Partikel sich in Abwesenheit der zu analysierenden Substanz nicht binden.

**3.** Verwendung nach Anspruch 1 oder 2 zur Quantifizierung einer interessierenden Substanz, die in der Probe mit einem Gehalt von weniger als 1 pM oder sogar weniger als 0,1 pM oder sogar weniger als 0,01 pM vorhanden ist.

**4.** Verwendung nach einem der vorhergehenden Ansprüche, wobei es sich bei der Probe um eine biologische Probe, insbesondere eine vom Menschen entnommen Probe, handelt und die noch spezifischer aus Blut, Serum, Plasma, Speichel, Urin, Zerebrospinalflüssigkeit, Nasenabstrich, Vaginalabstrich, Sputum und verdünnten Fäkalien ausgewählt ist.

**5.** Verwendung nach einem der vorhergehenden Ansprüche zur Quantifizierung von Biomarkern, Antikörpern, DNA und/oder RNA in einer biologischen Probe.

**6.** Verwendung nach einem der vorhergehenden Ansprüche, wobei das Targeting-Mittel aus einem polyklonalen oder monoklonalen Antikörper, einem Antikörperfragment, einem Nanobody, einem Oligonucleotid, einem Peptid, einem Hormon, einem Liganden, einem Zytokin, einem Peptidomimetikum, einem Protein, einem Kohlenhydrat, einem chemisch modifizierten Protein, einer chemisch modifizierten Nucleinsäure, einem chemisch modifizierten Kohlenhydrat, das auf ein bekanntes Zelloberflächenprotein abzielt, einem Aptamer, einer Anordnung von Proteinen und DNA/RNA oder einem für Markierungen vom HaloTag-Typ verwendeten Chloralkan ausgewählt ist, insbesondere ein Antikörper oder ein Antikörperfragment ist.

**7.** Verwendung nach einem der vorhergehenden Ansprüche, wobei das Nanopartikel eine unvollkommene Kristallinität aufweist.

**8.** Verwendung nach einem der vorhergehenden Ansprüche, wobei die Nanopartikel eine Emissionsdauer von größer als oder gleich 5 $\mu$s, insbesondere größer als oder gleich 10 $\mu$s, im Besonderen größer oder gleich 20 $\mu$s oder sogar größer oder gleich 50 $\mu$s, aufweisen.

**9.** Verwendung nach einem der vorhergehenden Ansprüche, wobei die Nanopartikel eine mittlere Größe zwischen 20 nm und 500 nm, vorzugsweise zwischen 20 nm und 200 nm und im Besonderen zwischen 20 und 100 nm, insbesondere zwischen 25 nm und 100 nm, im Besonderen zwischen 30 und 60 nm, aufweisen.

**10.** Verwendung nach einem der vorhergehenden Ansprüche, wobei es sich bei den Lantanoidionen um Eu handelt.

**11.** Verwendung nach einem der vorhergehenden Ansprüche, wobei A für Y steht.

**12.** Verwendung zur ultraempfindlichen In-vitro-Quantifizierung eines Gehalts von streng kleiner als 10 pM einer biologischen oder chemischen interessierenden Substanz in einer Probe mittels fotolumineszierender anorganischer Nanopartikel, die ganz oder teilweise aus einem fotolumineszierenden anorganischen Nanopartikel bestehen, das

aus einer kristallinen Matrix besteht, die mindestens $10^3$ Seltenerdionen aufweist, und die an mindestens ein Targeting-Mittel der zu analysierenden Substanz gekoppelt sind, wobei die Nanopartikel eine mittlere Größe größer als oder gleich 20 nm und streng kleiner als 1 µm aufweisen und dazu geeignet sind, nach der Absorption eines Photons Lumineszenz zu emittieren; wobei bei der Verwendung außerdem Folgendes angewandt wird:

(i) eine Anregung der Seltenerdionen der mit der zu analysierenden Substanz verbundenen Partikel durch eine Beleuchtungsvorrichtung, insbesondere vom Laser-Typ, mit einer Leistung von mindestens 50 mW, vorzugsweise mindestens 500 mW, und einer Anregungsintensität von mindestens 1 W/cm$^2$, vorzugsweise mindestens 10 W/cm$^2$;
(ii) ein Nachweis der Lumineszenzemission durch die Partikel nach einer Ein-Photonen-Absorption und
(iii) die Bestimmung der Konzentration der Substanz durch eine Interpretation der Lumineszenzmessung durch die Bezugnahme auf einen Standard oder eine Kalibrierung und wobei die Nanopartikel die folgende Formel (II) aufweisen:

$$A_{1-x}Ln_x(VO_4)_{(1-y)}(PO_4)_y \qquad \text{(II)}$$

aufweisen, wobei:

. A aus Yttrium (Y), Gadolinium (Gd), Lanthan (La), Lutetium (Lu) und Mischungen davon ausgewählt ist, A insbesondere für Y steht;
. Ln aus Europium (Eu), Dysprosium (Dy), Samarium (Sm), Neodym (Nd), Erbium (Er), Ytterbium (Yb), Thulium (Tm), Terbium (Tb) und Mischungen davon ausgewählt ist, Ln insbesondere für Eu steht;
. $0 < x < 1$; insbesondere $0{,}2 \leq x \leq 0{,}6$ und noch spezifischer x 0,4 beträgt und
. $0 \leq y < 1$, wobei y insbesondere 0 beträgt.

**13.** Verwendung nach Anspruch 12, wobei die Nanopartikel der Formel (II) auf der Oberfläche Tetraalkylammonium-Kationen, insbesondere Kationen der Formel $NR_4^+$ aufweisen, wobei R, die gleich oder verschieden sind, für eine $C_1$-$C_6$-Alkyl-, insbesondere eine $C_1$-$C_4$-Alkyl-, noch spezifischer eine $C_1$-$C_3$-Alkylgruppe, stehen und insbesondere aus Tetramethylammonium-, Tetraethylammonium-, Tetrapropylammonium-, Tetrabutylammonium-Kationen und Mischungen davon ausgewählt sind, wobei es sich bei den Tetraalkylammonium-Kationen vorzugsweise um Tetramethylammonium-Kationen handelt.

**14.** Verwendung nach dem vorhergehenden Anspruch, wobei die Nanopartikel die Formel (II')

$$A_{1-x}Ln_x(VO_4)_{(1-y)}(PO_4)_y.\ (NR_4^+)_z \qquad \text{(II')}$$

aufweisen, wobei:

. A wie in Anspruch 12 definiert ist;
. Ln wie in Anspruch 12 definiert ist;
. $0 < x < 1$; insbesondere $0{,}2 \leq x \leq 0{,}6$ und noch spezifischer x 0,4 beträgt;
. $0 \leq y < 1$, wobei y insbesondere 0 beträgt;
. R, die gleich oder verschieden sind, für eine $C_1$-$C_6$-Alkyl-, insbesondere eine $C_1$-$C_4$-Alkyl-, insbesondere eine $C_1$-$C_3$-Alkyl- und noch spezifischer eine Methylgruppe stehen; und
. z für die Zahl von Tetraalkylammonium-Kationen $NR_4^+$ steht, die sich auf der Oberfläche des Nanopartikels befinden, wobei z insbesondere zwischen 100 und 10 000 beträgt.

**15.** Verwendung nach einem der vorhergehenden Ansprüche, wobei die Nanopartikel die Formel $Y_{1-x}Eu_xVO_4$ aufweisen, wobei $0 < x < 1$, wobei die Nanopartikel insbesondere auf der Oberfläche Tetraalkylammonium-Kationen, insbesondere gemäß der Definition in Anspruch 13, aufweisen.

**16.** Verwendung nach einem der vorhergehenden Ansprüche, wobei die Nanopartikel nach ihrer Synthese ein als ζ bezeichnetes Zeta-Potential kleiner als oder gleich - 28 mV, vorzugsweise kleiner als oder gleich -30 mV, in einem wässrigen Medium mit einem pH-Wert ≥ 5, insbesondere mit einem pH-Wert ≥ 5,5 und noch spezifischer mit einem pH-Wert ≥ 6, und mit einer Ionenleitfähigkeit streng kleiner als 100 µS.cm$^{-1}$ aufweisen.

**17.** Verwendung nach einem der Ansprüche 2 bis 16, wobei der Träger vom Lamellen-, Multiwellplatten-, Mikrotiterplatten-, Gelmembran-, Streifen- oder Mikrokanaltyp ist.

**18.** Verwendung nach einem der Ansprüche 2 bis 17, wobei die Anregung (i) mit einem Laser-Anregungsstrahl erfolgt, der so ausgerichtet ist, dass ein Einfallswinkel größer als oder gleich 55° mit der Senkrechten des Trägers gebildet wird, der auf seiner Oberfläche die an die zu analysierende Substanz gebundenen Partikel aufweist.

**19.** Verwendung nach einem der Ansprüche 2 bis 18, wobei die Anregung der Partikel mittels einer evaneszenten Welle, insbesondere mittels einer Laser-Beleuchtungsanordnung vom TIRF-Typ, erfolgt, wodurch im Fall einer Glas/-Wasser-Grenzfläche zwischen dem Träger und der zu analysierenden Probe ein Einfallswinkel des Anregungsstrahls größer als oder gleich 61° ermöglicht wird.

**20.** Verwendung nach einem der vorhergehenden Ansprüche, wobei die Emissionsdauer durch die Nanopartikel größer als oder gleich 1 μs, insbesondere größer als oder gleich 50 μs, ist, die Detektion der Lichtstärke (ii) eine zeitaufgelöste Detektion der Emission, insbesondere eine verzögerte Detektion des von den fotolumineszierenden Partikeln emittierten Signals, umfasst.

**21.** Verwendung nach einem der vorhergehenden Ansprüche zur gleichzeitigen Quantifizierung von mindestens zwei Substanzen, die verschieden und mit Gehalten von streng weniger als 10 pM in einer Probe vorhanden sind.

**22.** Verwendung nach dem vorhergehenden Anspruch, wobei die zu analysierenden Substanzen der Probe in vordefinierten und getrennten Zonen auf der Oberfläche eines Trägers immobilisiert werden, wobei die Oberfläche so passiviert ist, dass die fotolumineszierenden Partikel sich in Abwesenheit der zu analysierenden Substanzen nicht binden.

**23.** Verwendung nach Anspruch 21 oder 22 von mindestens zwei Typen von Nanopartikeln mit Emissionswellenlängen, die verschieden und mit den Targeting-Mitteln einer jeden der zu analysierenden Substanzen gekoppelt sind.

**24.** Verwendung nach einem der vorhergehenden Ansprüche, wobei die Laser-Beleuchtungsvorrichtung (i) eine optische Anordnung, insbesondere ein System mit mindestens einer Linse, umfasst, das auf der Bahn des Laserstrahls so angeordnet ist, dass die Größe des Strahls im Bereich der Zone des Trägers gesteuert wird, der die Partikel aufweist, die mit der zu analysierenden Substanz verbunden sind.

**25.** Verwendung nach einem der vorhergehenden Ansprüche, wobei die Detektion der Lumineszenzemission (ii) mittels einer Vorrichtung zur Detektion der Lichtstärke erfolgt, die einen einzelnen Detektor, beispielsweise vom Fotovervielfacher-, Fotodioden-, Avalanche-Fotodioden-Typ, oder einen Detektor vom Typ eines Arrays von lichtempfindlichen Bauelementen umfasst, die aus einer 2D-Fläche von Detektionspixeln bestehen, wie einer CCD- oder EM-CCD-Kamera oder CMOS-Kamera.

**26.** Verwendung nach dem vorhergehenden Anspruch, wobei die Detektionsvorrichtung eine optische Anordnung, insbesondere ein System mit mindestens einer Linse mit einer großen numerischen Apertur, zum Fokussieren der Lumineszenzemission auf den Detektor, insbesondere auf den Fotovervielfacher, umfasst.

## Claims

**1.** Use, for ultra-sensitive quantification in vitro of an amount strictly less than 10 pM of a substance of biological or chemical interest in a sample, of photoluminescent inorganic nanoparticles,
which are formed in whole or in part of a photoluminescent inorganic nanoparticle formed from a crystalline matrix having at least $10^3$ rare-earth ions, and which are coupled to at least one targeting agent targeting the substance to be analysed, said nanoparticles having an average size greater than or equal to 20 nm and strictly less than 1 μm, and being capable of emitting luminescence after absorption of a photon; said use further implementing

(i) excitation of the rare-earth ions of the particles associated with the substance to be analysed, by an illuminating device, in particular a laser, of a power of at least 50 mW, and preferably of at least 500 mW, and of an excitation intensity of at least 1 W/cm$^2$, and preferably of at least 10 W/cm$^2$;
(ii) detection of the emission of luminescence by the particles after single-photon absorption, and
(iii) determination of the concentration of the substance through interpretation of said luminescence measurement, with reference to a standard or calibration and wherein the nanoparticles are of following formula (I) :

$$(A_{1-x}Ln_x)_a(M_pO_q) \qquad (I)$$

in which:

- M is one or more elements selected from V, P, W, Mo, As, Al, Hf, Zr, Ge, Ti, Sn, Mn and Si;
- Ln corresponds to one or more luminescent lanthanide ions selected from europium (Eu), dysprosium (Dy), samarium (Sm), praseodymium (Pr), neodymium (Nd), erbium (Er), ytterbium (Yb), cerium (Ce), holmium (Ho), terbium (Tb), thulium (Tm) and mixtures thereof
- A corresponds to one or more constituent ions of the crystalline matrix the electronic levels of which are not involved in the luminescence process and which are selected from yttrium (Y), gadolinium (Gd), lanthanum (La), bismuth (Bi), lutetium (Lu) and mixtures thereof,
- $0 < x < 1$, in particular $0.1 \leq x \leq 0.9$, in particular $0.2 \leq x \leq 0.6$, in particular $0.2 \leq x \leq 0.4$ and more particularly x is equal to 0.4 and
- the values of p, q and a are such that the electroneutrality of $(A_{1-x}Ln_x)_a(M_pO_q)$ is respected.

**2.** Use according to the preceding claim, wherein the substance to be analysed of said sample is immobilized on the surface of a medium, said surface being passivated so that said luminescent particles do not bind thereto in the absence of the substance to be analysed.

**3.** Use according to Claim 1 or 2, for quantification of a substance of interest present in the sample in an amount less than 1 pM, or even less than 0.1 pM, or indeed less than 0.01 pM.

**4.** Use according to any of the preceding claims, wherein the sample is a biological sample, in particular a sample taken from a human, and more particularly selected from blood, serum, plasma, saliva, urine, cerebrospinal fluid, a nasal smear, a vaginal smear, sputum and diluted faecal matter.

**5.** Use according to any of the preceding claims, for quantification of biomarkers, antibodies, DNA and/or RNA in a biological sample.

**6.** Use according to any of the preceding claims, wherein said targeting agent is selected from a polyclonal or monoclonal antibody, an antibody fragment, a nanobody, an oligonucleotide, a peptide, a hormone, a ligand, a cytokine, a peptidomimetic, a protein, a carbohydrate, a chemically modified protein, a chemically modified nucleic acid, a chemically modified carbohydrate that targets a known cell surface protein, an aptamer, a DNA/RNA and protein assembly, or a chloroalkane used by HaloTag tags, and in particular is an antibody or antibody fragment.

**7.** Use according to any of the preceding claims, wherein the nanoparticle has imperfect crystallinity.

**8.** Use according to any of the preceding claims, wherein the nanoparticles have an emission lifetime greater than or equal to 5 $\mu$s, in particular greater than or equal to 10 $\mu$s, in particular greater than or equal to 20 $\mu$s, or even greater than or equal to 50 $\mu$s.

**9.** Use according to any of the preceding claims, wherein the nanoparticles have an average size of between 20 nm and 500 nm, preferably between 20 nm and 200 nm and in particular between 20 and 100 nm, in particular between 25 nm and 100 nm, in particular between 30 and 60 nm.

**10.** Use according to any of the preceding claims, wherein the lanthanide ions are Eu.

**11.** Use according to any of the preceding claims, wherein A is Y.

**12.** Use, for ultra-sensitive quantification in vitro of an amount strictly less than 10 pM of a substance of biological or chemical interest in a sample, of photoluminescent inorganic nanoparticles, which are formed in whole or in part of a photoluminescent inorganic nanoparticle formed from a crystalline matrix having at least $10^3$ rare-earth ions, and which are coupled to at least one targeting agent targeting the substance to be analysed, said nanoparticles having an average size greater than or equal to 20 nm and strictly less than 1 $\mu$m, and being capable of emitting luminescence after absorption of a photon; said use further implementing

(i) excitation of the rare-earth ions of the particles associated with the substance to be analysed, by an illuminating device, in particular a laser, of a power of at least 50 mW, and preferably of at least 500 mW, and of an excitation intensity of at least 1 W/cm$^2$, and preferably of at least 10 W/cm$^2$;

(ii) detection of the emission of luminescence by the particles after single-photon absorption, and
(iii) determination of the concentration of the substance through interpretation of said luminescence measurement, with reference to a standard or calibration and wherein the nanoparticles are of following formula (II) :

$$A_{1-x}Ln_x(VO_4)_{(1-y)}(PO_4)_y \qquad (II)$$

in which:

. A is selected from yttrium (Y), gadolinium (Gd), lanthanum (La), lutetium (Lu) and mixtures thereof, and in particular A is Y;
. Ln is selected from europium (Eu), dysprosium (Dy), samarium (Sm), neodymium (Nd), erbium (Er), ytterbium (Yb), thulium (Tm), terbium (Tb) and mixtures thereof, and in particular Ln is Eu;
. $0 < x < 1$; in particular $0.2 \leq x \leq 0.6$ and more particularly x is equal to 0.4; and
. $0 \leq y < 1$, and in particular y is equal to 0.

**13.** Use according to Claim 12, wherein said nanoparticles of formula (II) have tetraalkylammonium cations on their surface, in particular cations of formula $NR_4^+$ with each R, whether identical to or different from the others, being a $C_1$-$C_6$-alkyl group, in particular a $C_1$-$C_4$-alkyl group, more particularly a $C_1$-$C_3$-alkyl group, and in particular being selected from the cations tetramethylammonium, tetraethylammonium, tetrapropylammonium, tetrabutylammonium and mixtures thereof, said tetraalkylammonium cations preferably being tetramethylammonium cations.

**14.** Use according to the preceding claim, said nanoparticles being of formula (II')

$$A_{1-x}Ln_x(VO_4)_{(1-y)}(PO_4)_y.\ (NR_4^+)_z \qquad (II')$$

in which:

. A is as defined in Claim 12;
. Ln is as defined in Claim 12;
. $0 < x < 1$; in particular $0.2 \leq x \leq 0.6$ and more particularly x is equal to 0.4;
. $0 \leq y < 1$, in particular y is equal to 0;
. each R, whether identical to or different from the others, being a $C_1$-$C_6$-alkyl group, in particular a $C_1$-$C_4$-alkyl group, in particular a $C_1$-$C_3$-alkyl group, and more particularly methyl group; and
. z is the number of tetraalkylammonium cations $NR_4^+$ located on the surface of said nanoparticle, z in particular being between 100 and 10 000.

**15.** Use according to any of the preceding claims, wherein said nanoparticles are of the formula $Y_{1-x}Eu_xVO_4$, in which $0 < x < 1$, said nanoparticles in particular having tetraalkylammonium cations on their surface, in particular tetraalkylammonium cations as defined in Claim 13.

**16.** Use according to any of the preceding claims, wherein the nanoparticles have, at the end of their synthesis, a zeta potential, denoted $\zeta$, of less than or equal to -28 mV, and preferably less than or equal to - 30 mV, in an aqueous medium of $pH \geq 5$, in particular of $pH \geq 5.5$, and more particularly of $pH \geq 6$, and of ionic conductivity strictly less than 100 $\mu S.cm^{-1}$.

**17.** Use according to any of Claims 2 to 16, wherein said medium is a slide, a multi-well plate, a microplate, a membrane gel, a strip or a microchannel.

**18.** Use according to any of Claims 2 to 17, wherein the excitation (i) is carried out with a laser excitation beam oriented so as to make an angle of incidence to the vertical of the medium having on its surface said particles associated with the substance to be analysed, greater than or equal to 55°.

**19.** Use according to any of Claims 2 to 18, wherein the excitation of the particles is carried out via an evanescent wave, in particular using a laser illumination arrangement of TIRF type, allowing an angle of incidence of the excitation beam greater than or equal to 61° in the case of a glass/water interface between the medium and the sample to be analysed.

**20.** Use according to any of the preceding claims, wherein the lifetime of emission by the nanoparticles is greater than or equal to 1 $\mu s$, and in particular greater than or equal to 50 $\mu s$, the detection (ii) of light intensity comprising time-resolved detection of the emission, and in particular delayed detection of the signal emitted by the photoluminescent particles.

**21.** Use according to any of the preceding claims, for simultaneous quantification of at least two different substances present in amounts strictly less than 10 pM in a sample.

**22.** Use according to the preceding claim, wherein the substances to be analysed of said sample are immobilized in predefined and distinct regions on the surface of a medium, said surface being passivated in such a way that the photoluminescent particles do not bind thereto in the absence of the substances to be analysed.

**23.** Use according to Claim 21 or 22, of at least two types of nanoparticles that have distinct emission wavelengths and that are coupled to targeting agents targeting each of the substances to be analysed.

**24.** Use according to any of the preceding claims, wherein the laser illuminating device (i) comprises an optical arrangement, in particular a system of at least one lens, placed on the path of the laser beam so as to control the size of the beam in the region of the medium having particles associated with the substance to be analysed.

**25.** Use according to any of the preceding claims, wherein the detection (ii) of the emission of luminescence is carried out by a device for detecting light intensity comprising a single detector, for example a photomultiplier, a photodiode, an avalanche photodiode, or a detector of the type comprising an array of photosensitive devices forming a 2D area of detection pixels, such as a CCD or EM-CCD camera or CMOS camera.

**26.** Use according to the preceding claim, wherein the detecting device comprises an optical arrangement, in particular a system of at least one lens of large numerical aperture, for focusing the emission of luminescence towards the detector, and in particular towards the photomultiplier.

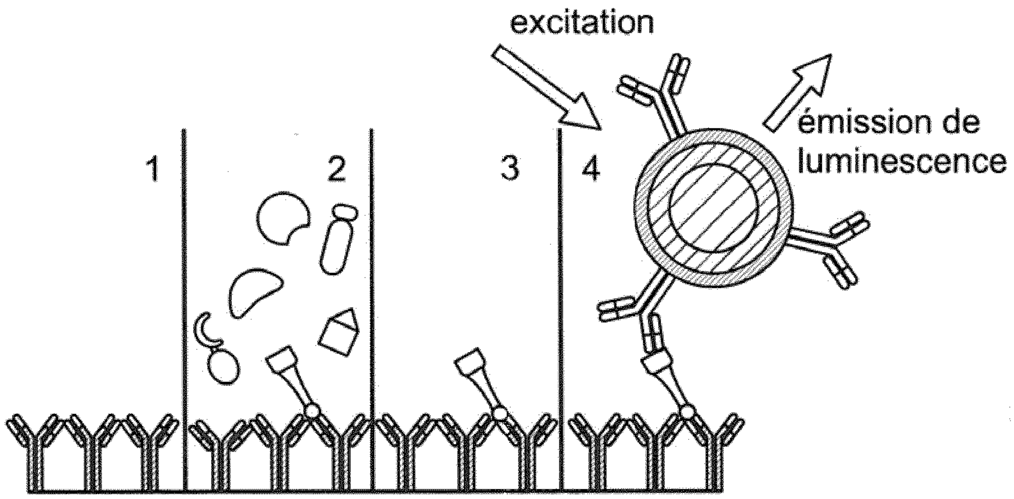


Fig. 1

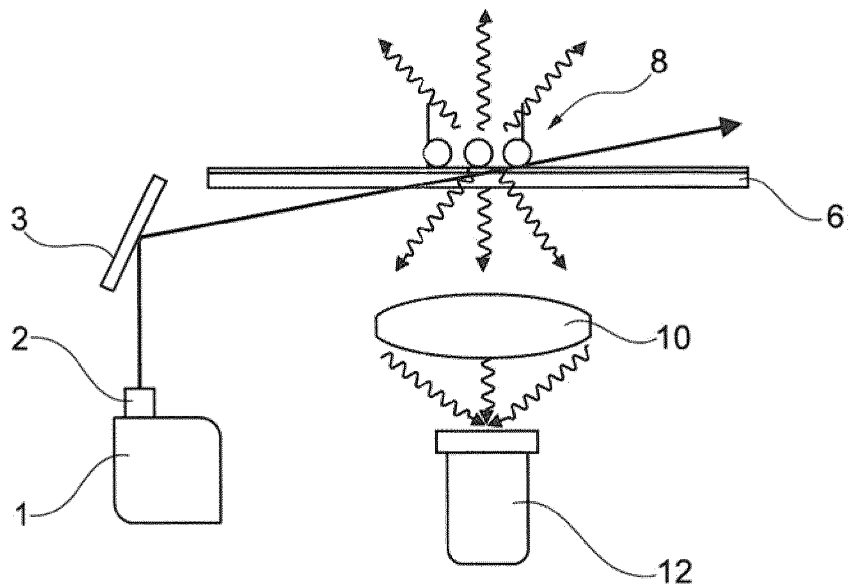


Fig. 2

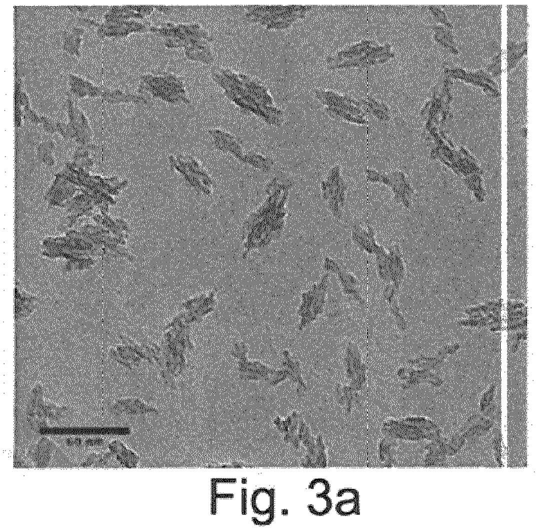

Fig. 3a

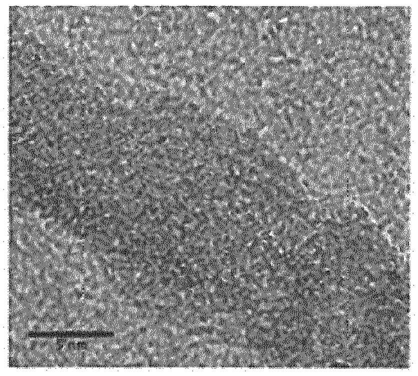

Fig. 3b

Fig. 3

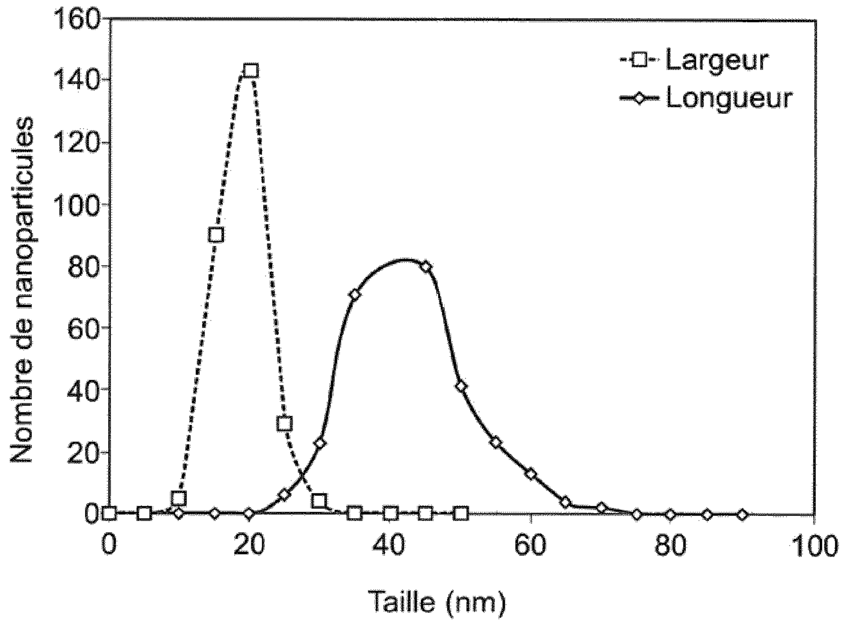


Fig. 4

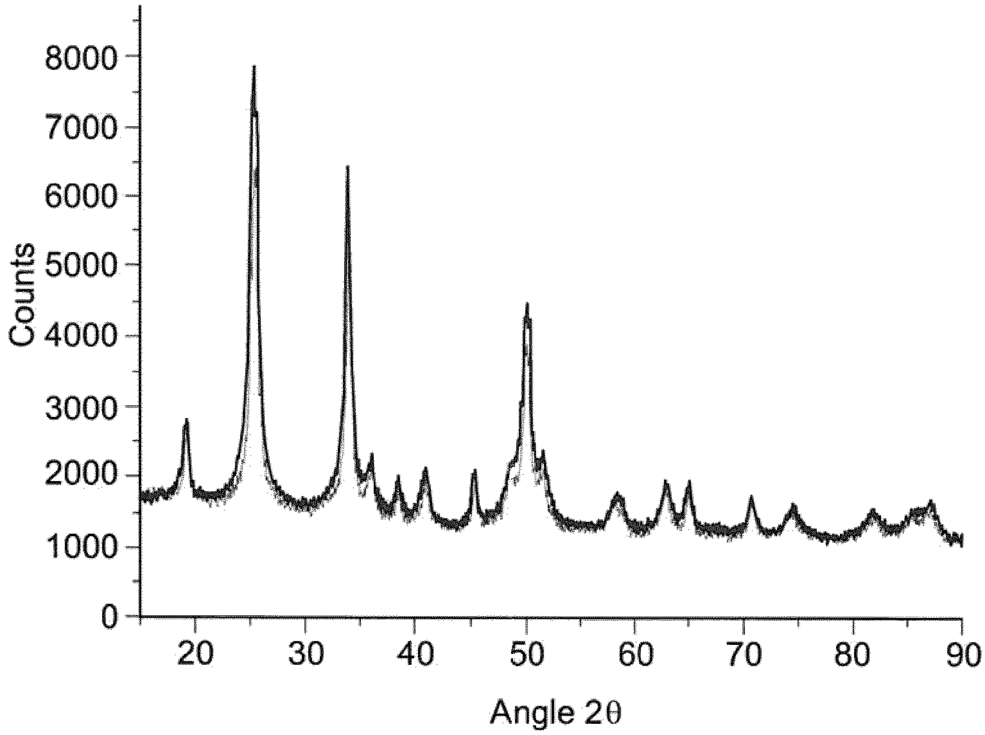


Fig. 5

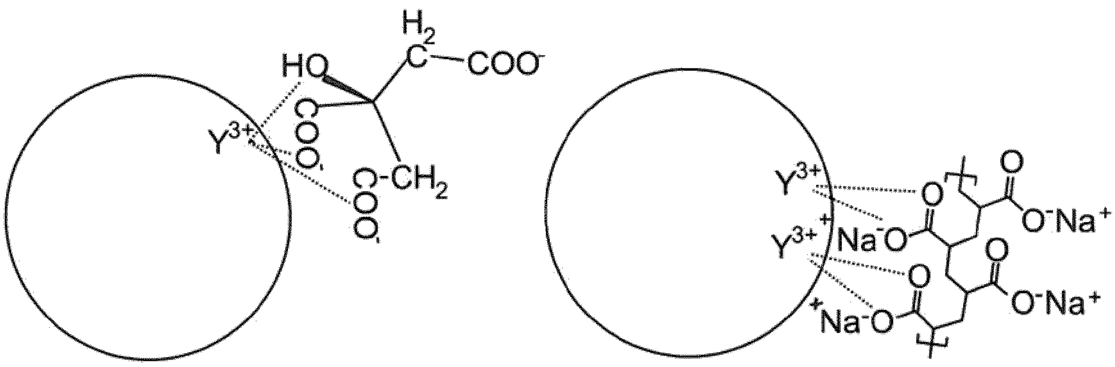

Fig. 6a

Fig. 6b

Fig. 6

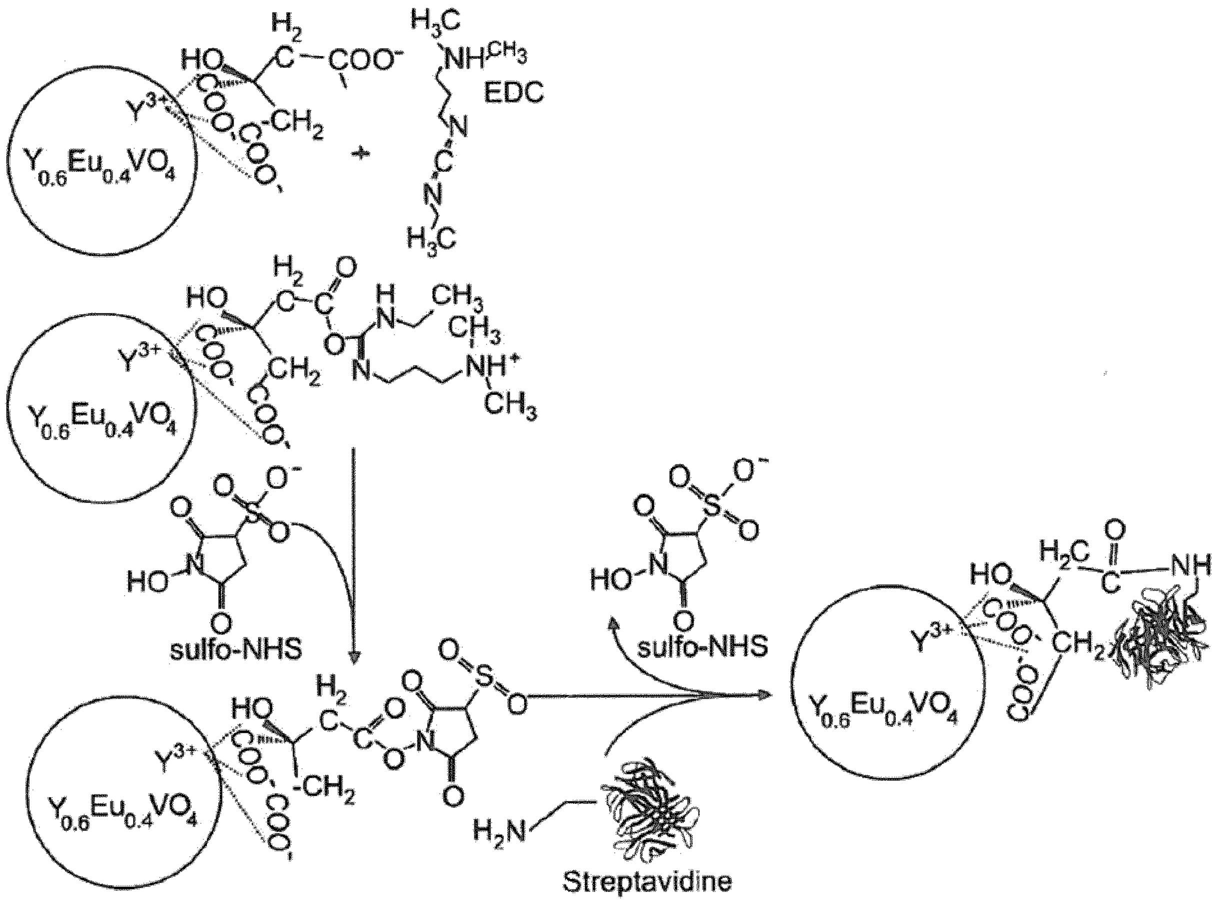


Fig. 7

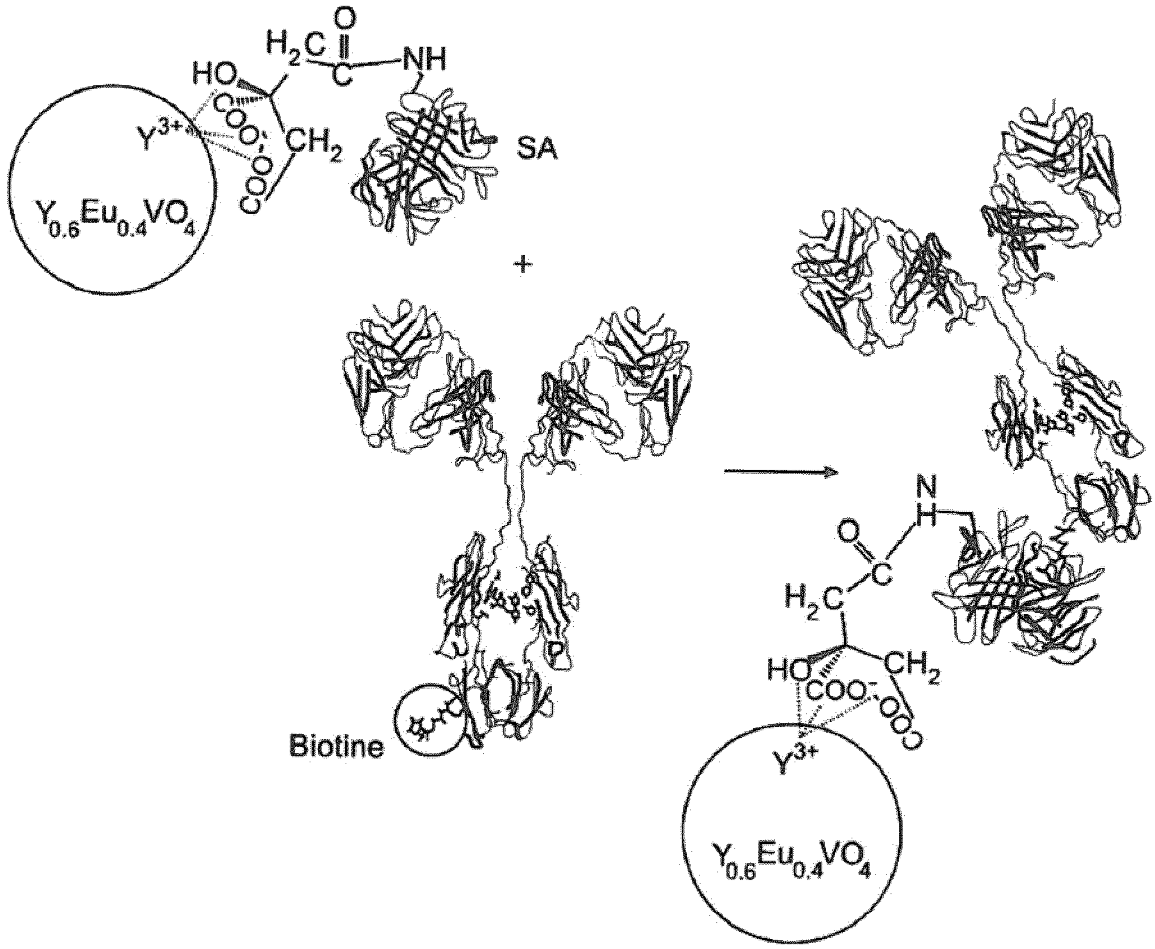


Fig. 8

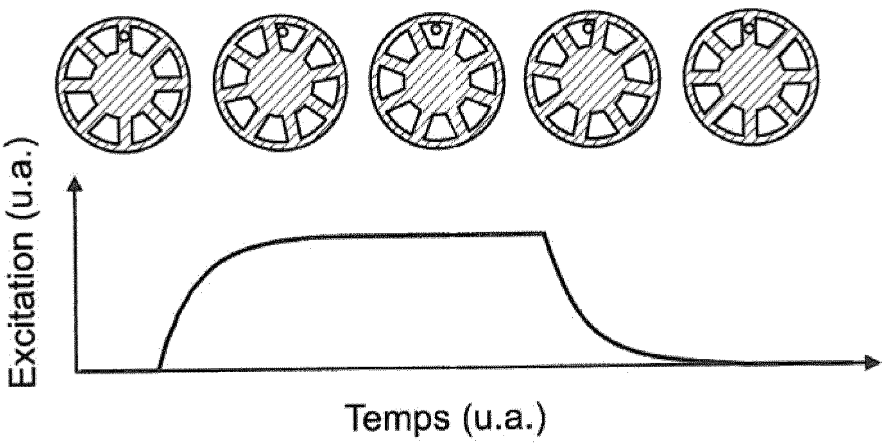
Excitation (u.a.)
Temps (u.a.)

Fig. 9

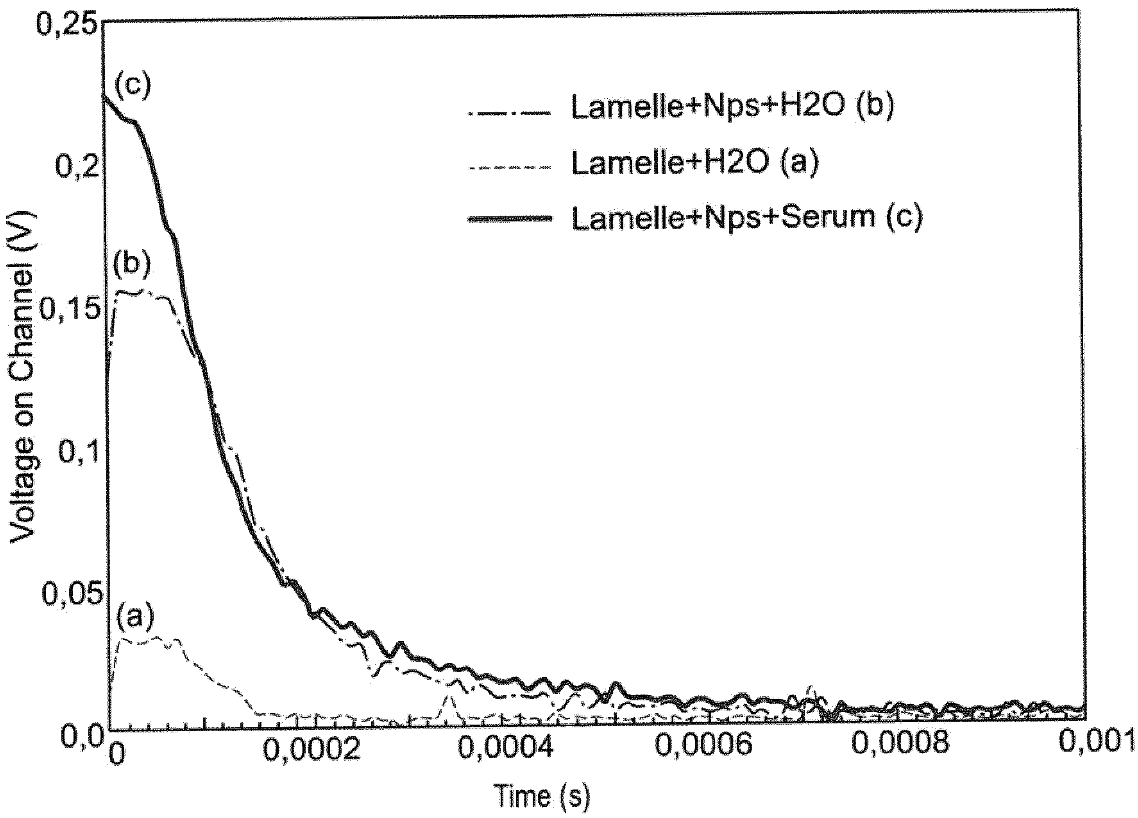
0,25
0,2
0,15
0,1
0,05
0,0
Voltage on Channel (V)
(c)
(b)
(a)
Lamelle+Nps+H2O (b)
Lamelle+H2O (a)
Lamelle+Nps+Serum (c)
0
0,0002
0,0004
0,0006
0,0008
0,001
Time (s)

Fig. 10a

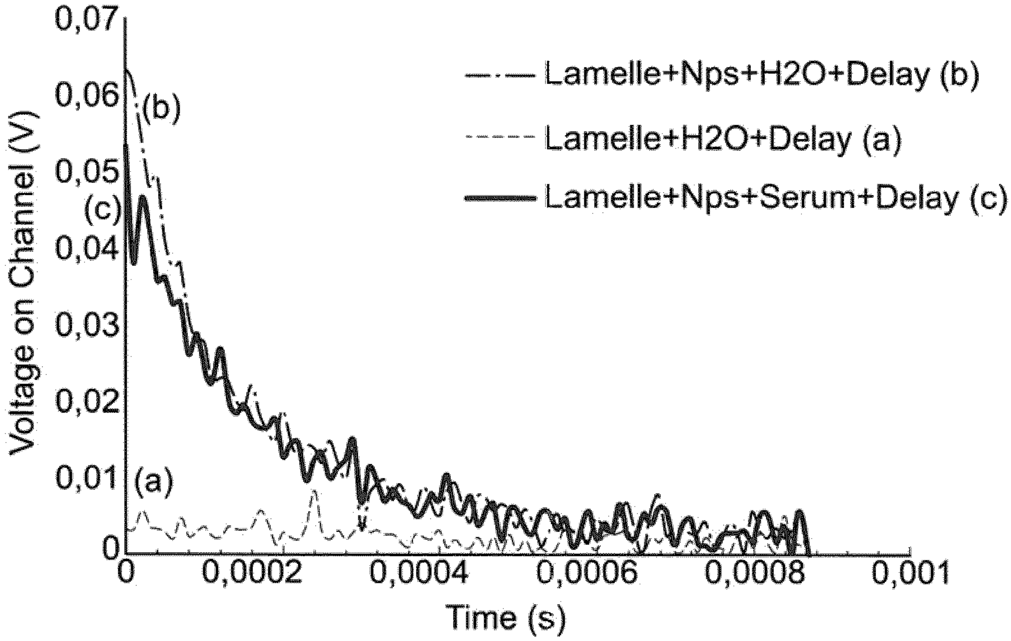


Fig. 10b

Fig. 10

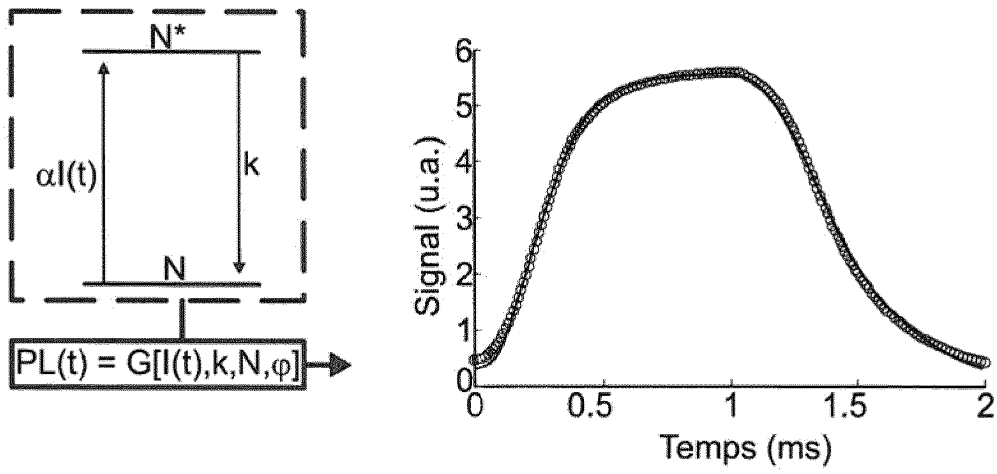


Fig. 11

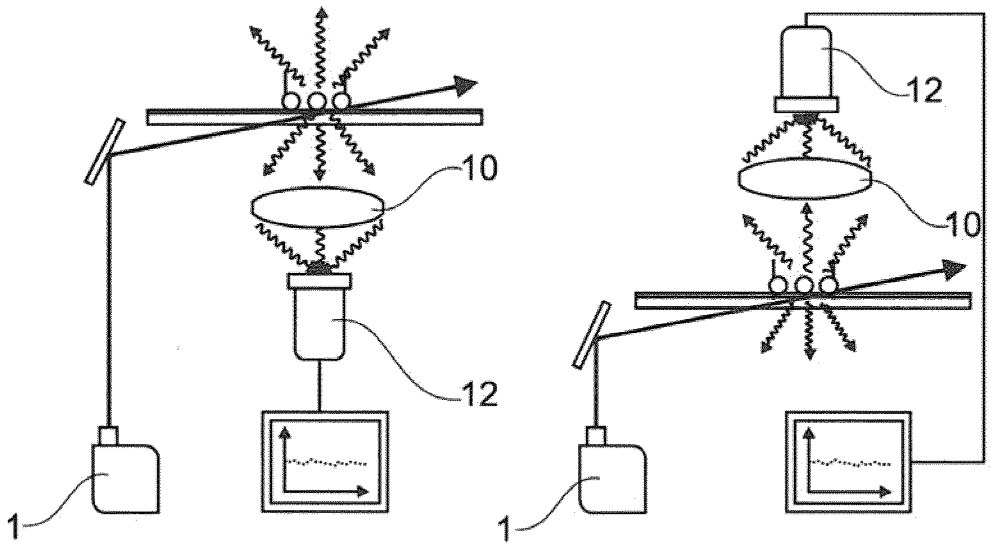


Fig. 12-a Fig. 12 Fig. 12-b

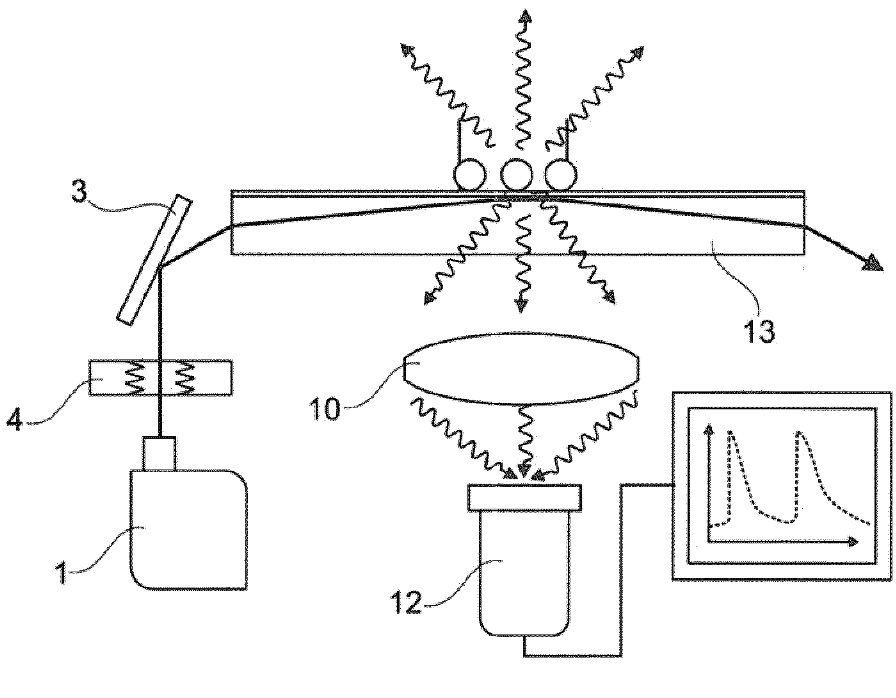


Fig. 13

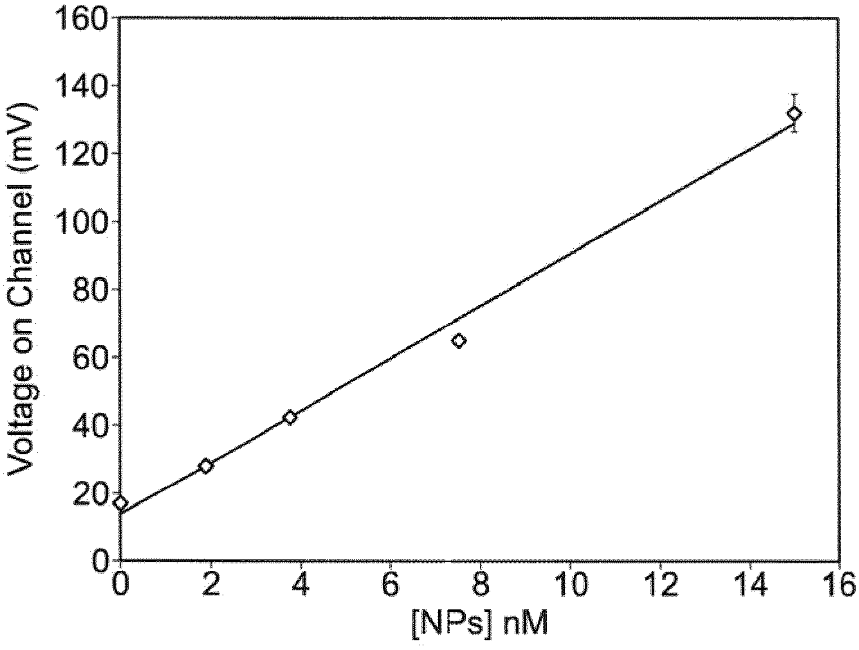


Fig. 14a

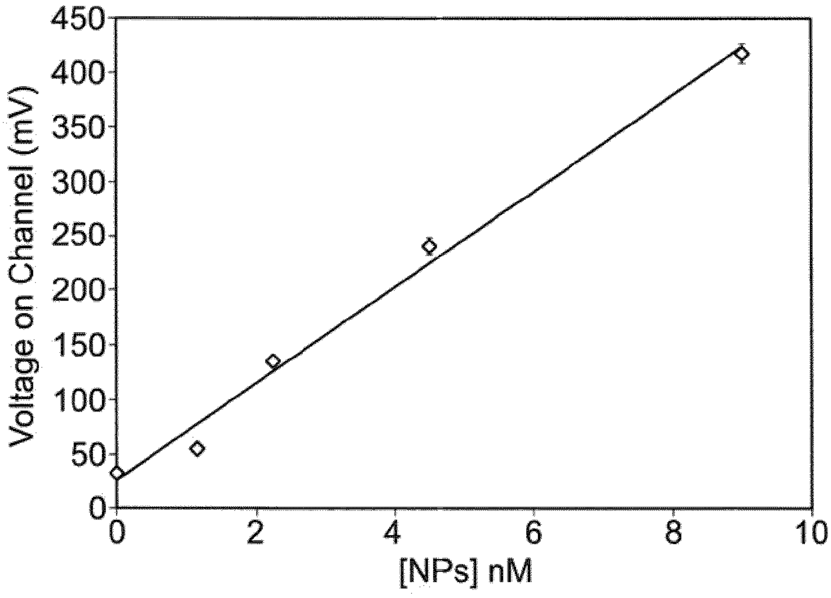


Fig. 14b

Fig. 14

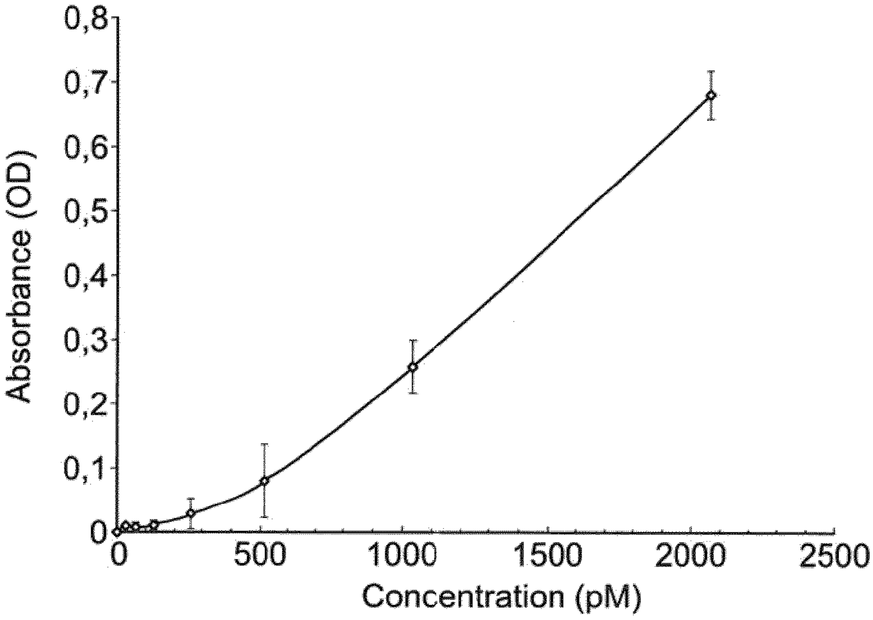

Absorbance (OD)
0,8
0,7
0,6
0,5
0,4
0,3
0,2
0,1
0
0
500
1000
1500
2000
2500
Concentration (pM)


Fig. 15

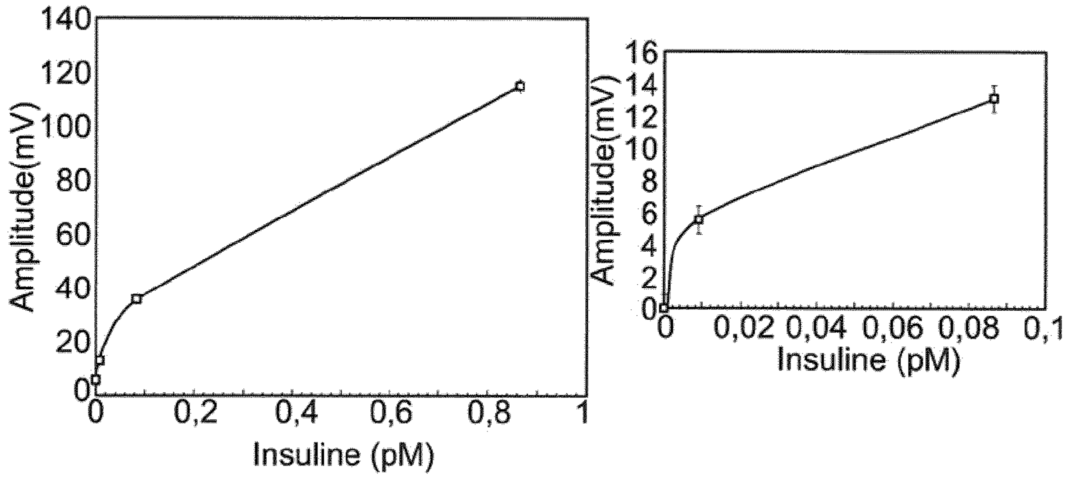

Amplitude(mV)
140
120
100
80
60
40
20
0
0
0,2
0,4
0,6
0,8
1
Insuline (pM)
Amplitude(mV)
16
14
12
10
8
6
4
2
0
0
0,02
0,04
0,06
0,08
0,1
Insuline (pM)


Fig. 16

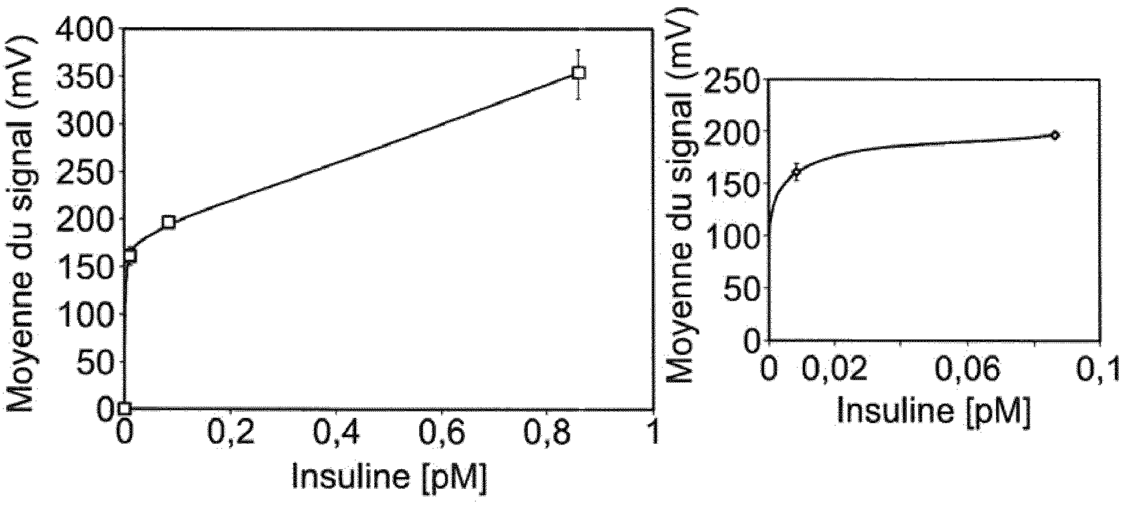

Moyenne du signal (mV)
400
350
300
250
200
150
100
50
0
0
0,2
0,4
0,6
0,8
1
Insuline [pM]
Moyenne du signal (mV)
250
200
150
100
50
0
0
0,02
0,06
0,1
Insuline [pM]


Fig. 17

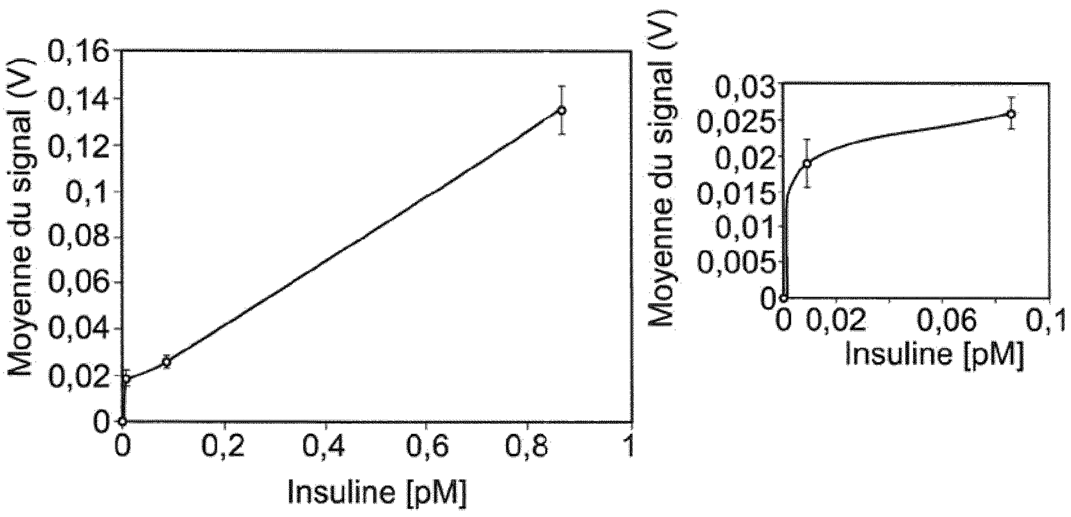

Moyenne du signal (V)
0,16
0,14
0,12
0,1
0,08
0,06
0,04
0,02
0
0
0,2
0,4
0,6
0,8
1
Insuline [pM]
Moyenne du signal (V)
0,03
0,025
0,02
0,015
0,01
0,005
0
0
0,02
0,06
0,1
Insuline [pM]


Fig. 18

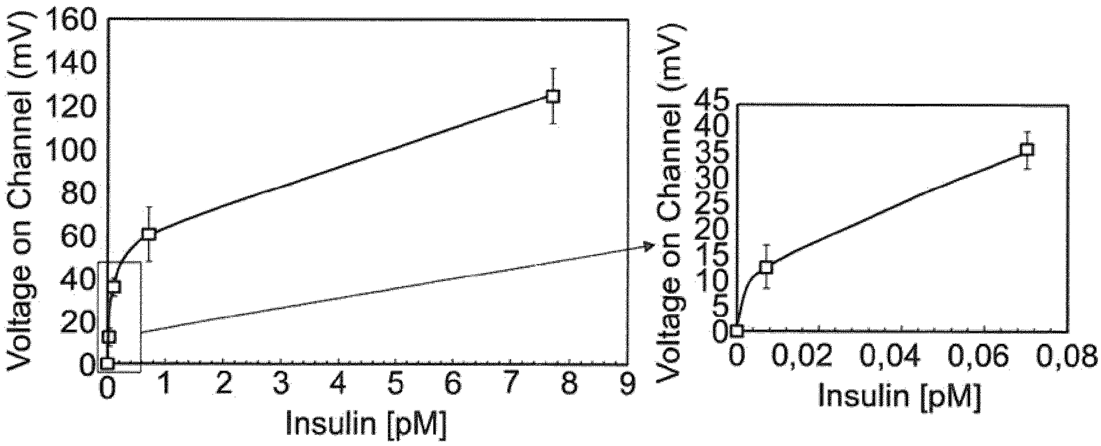
Voltage on Channel (mV)
160
140
120
100
80
60
40
20
0
0 1 2 3 4 5 6 7 8 9
Insulin [pM]
Voltage on Channel (mV)
45
40
35
30
25
20
15
10
5
0
0 0,02 0,04 0,06 0,08
Insulin [pM]

Fig. 19

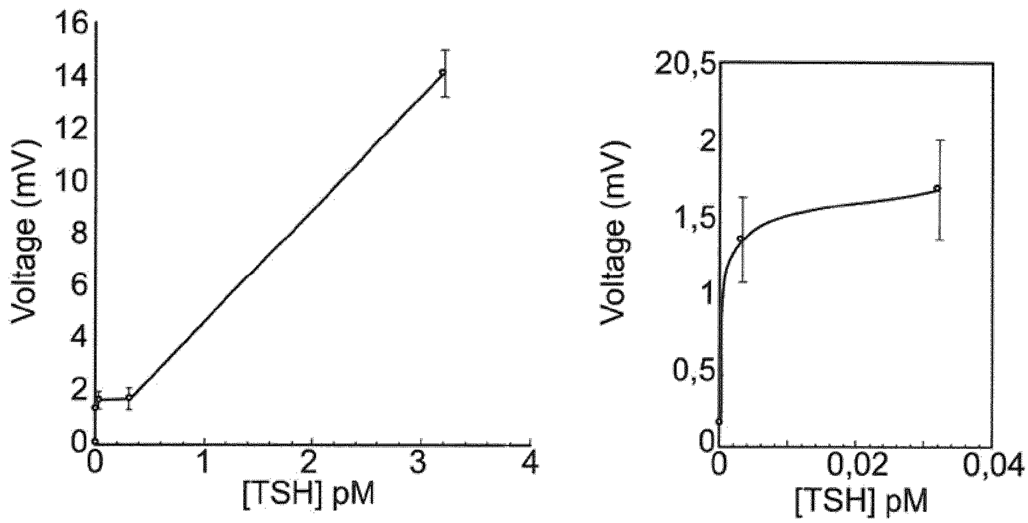
Voltage (mV)
16
14
12
10
8
6
4
2
0
0 1 2 3 4
[TSH] pM
Voltage (mV)
20,5
2
1,5
1
0,5
0
0 0,02 0,04
[TSH] pM

Fig. 20

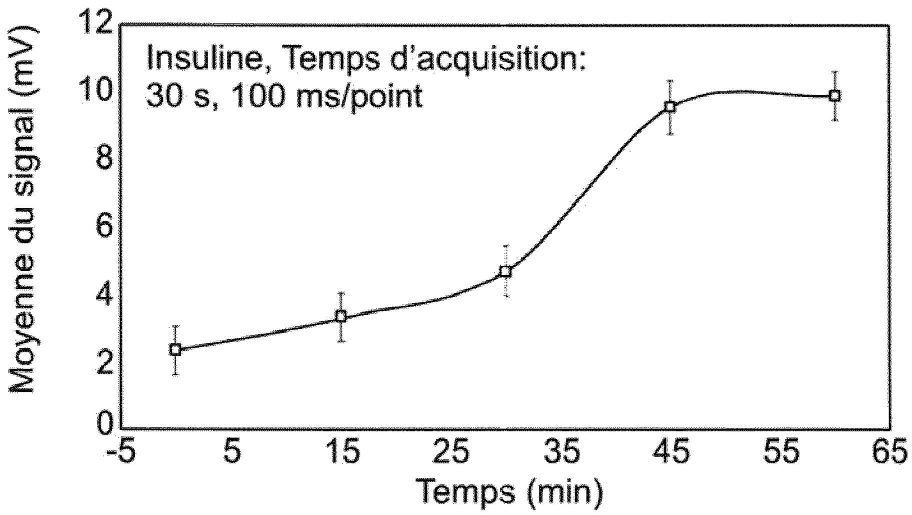
Insuline, Temps d'acquisition:
30 s, 100 ms/point
Moyenne du signal (mV)
12
10
8
6
4
2
0
-5
5
15
25
35
45
55
65
Temps (min)

Fig. 21

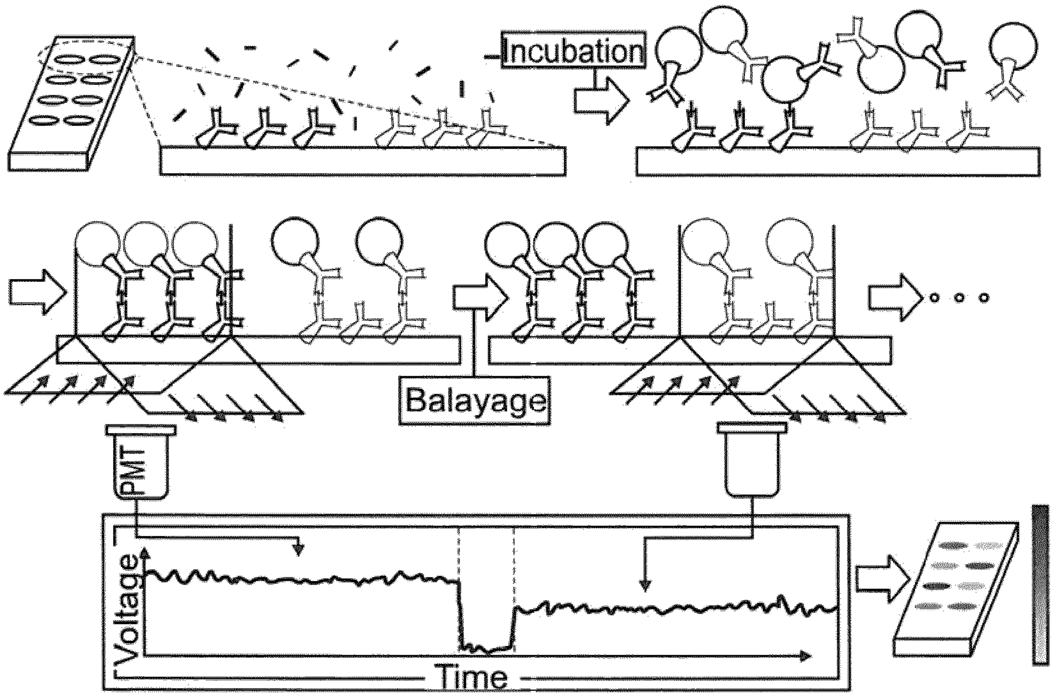
Incubation
Balayage
PMT
Voltage
Time

Fig. 22

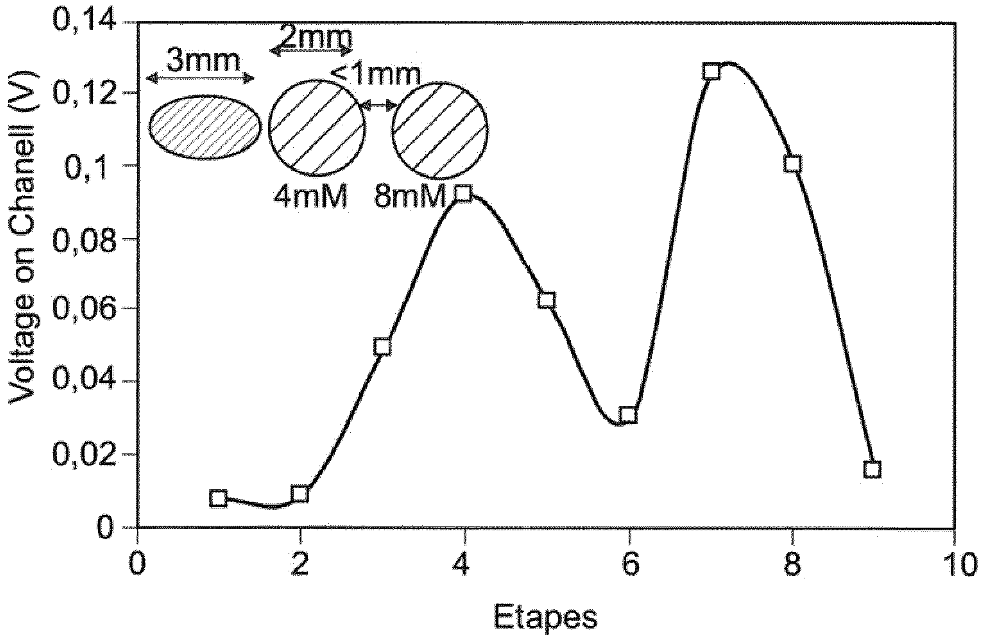
0,14
0,12
0,1
0,08
0,06
0,04
0,02
0
Voltage on Chanell (V)
3mm
2mm
<1mm
4mM
8mM
0
2
4
6
8
10
Etapes

Fig. 23

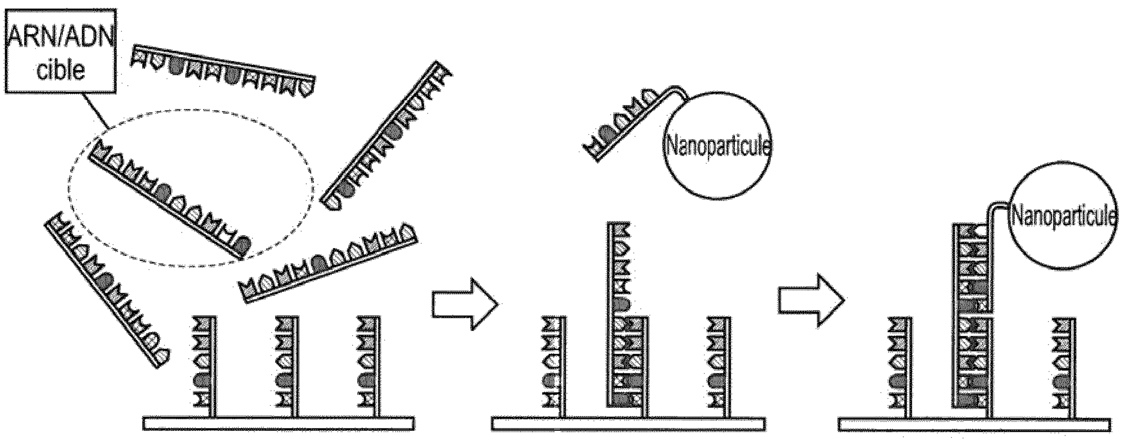
ARN/ADN cible
Nanoparticule
Nanoparticule

Fig. 24

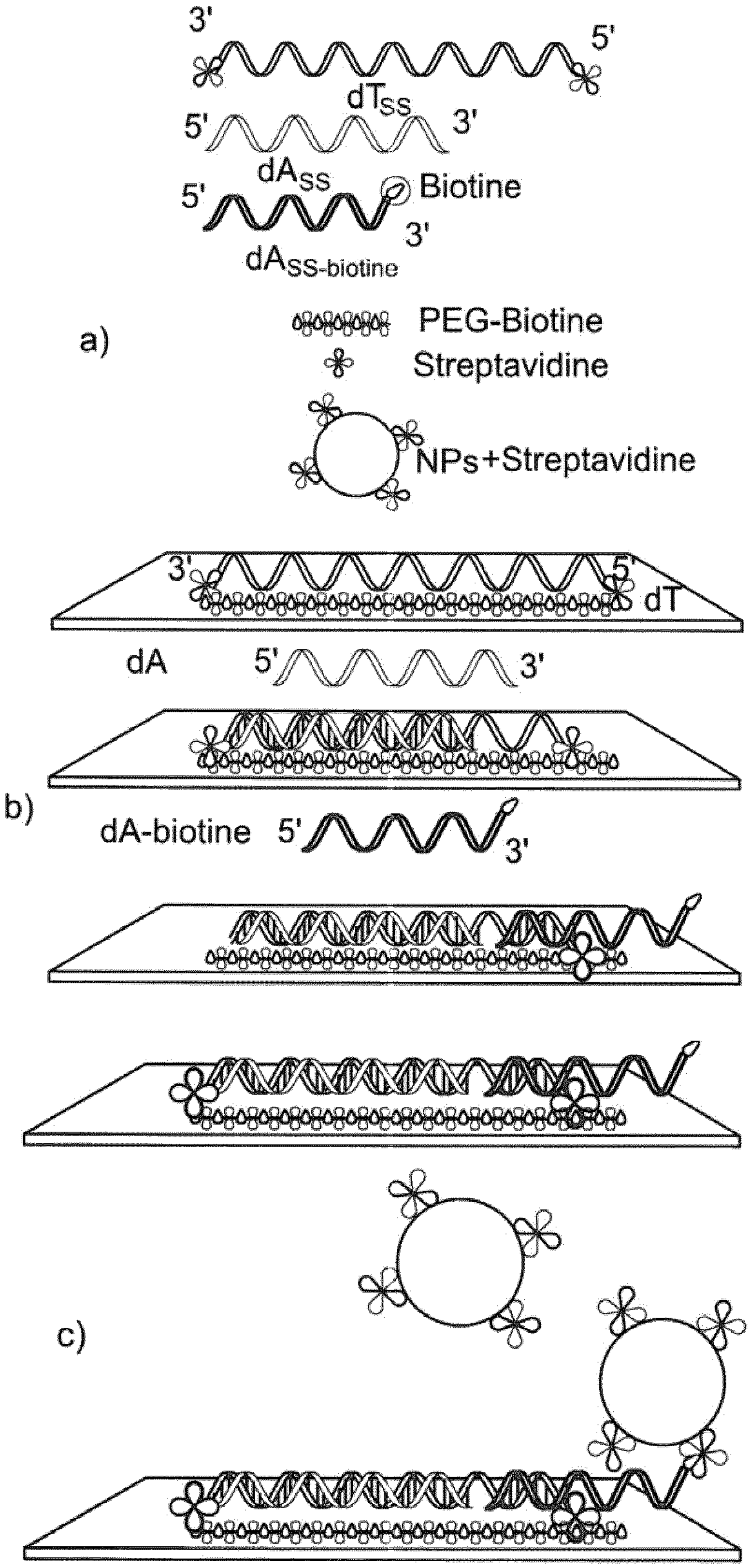
3'
5'
dTSS
5'
3'
dASS
5'
Biotine
3'
dASS-biotine
a)
PEG-Biotine
Streptavidine
NPs+Streptavidine
3'
5'
dT
dA
5'
3'
b)
dA-biotine
5'
3'
c)

Fig. 25

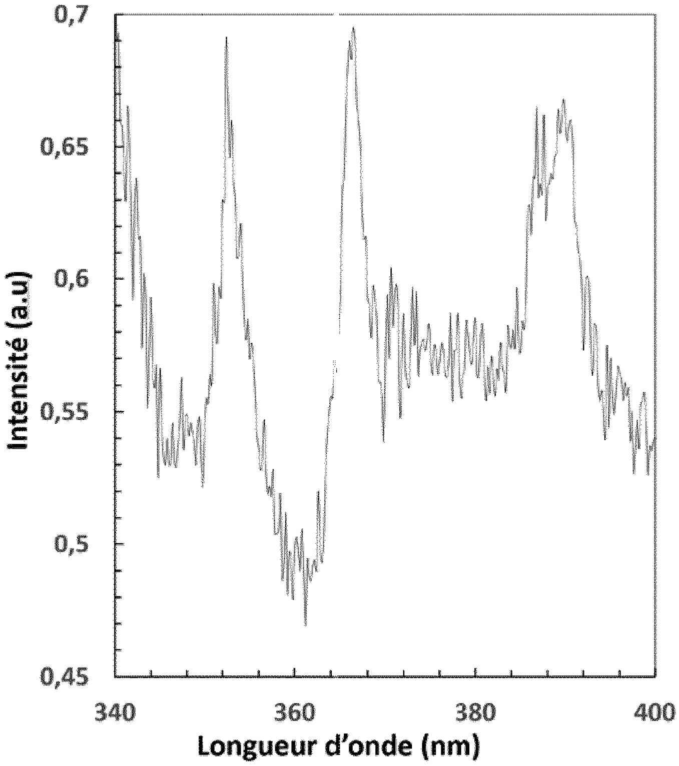


Fig. 26a

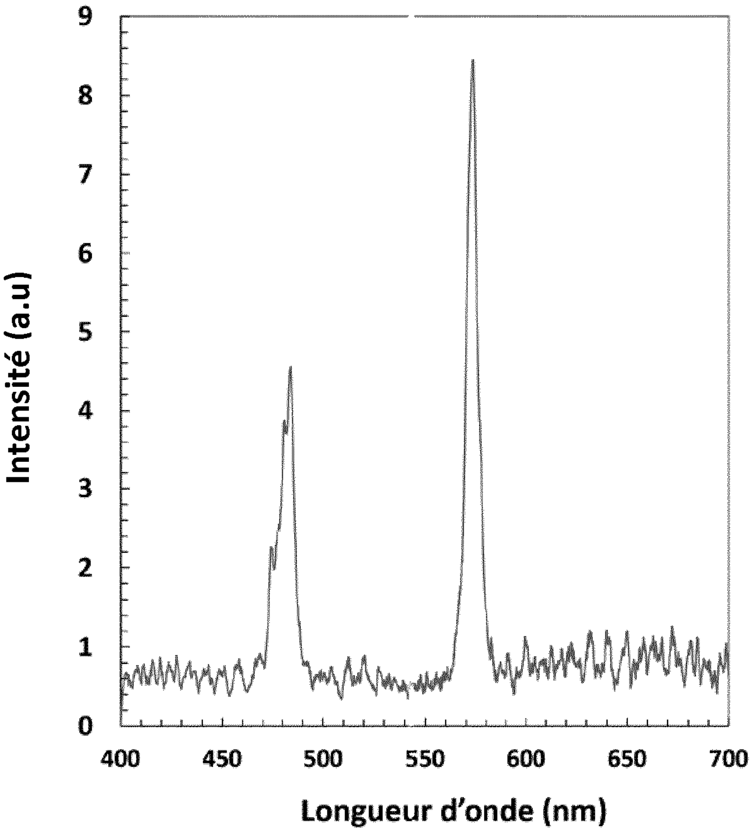


Fig. 26b

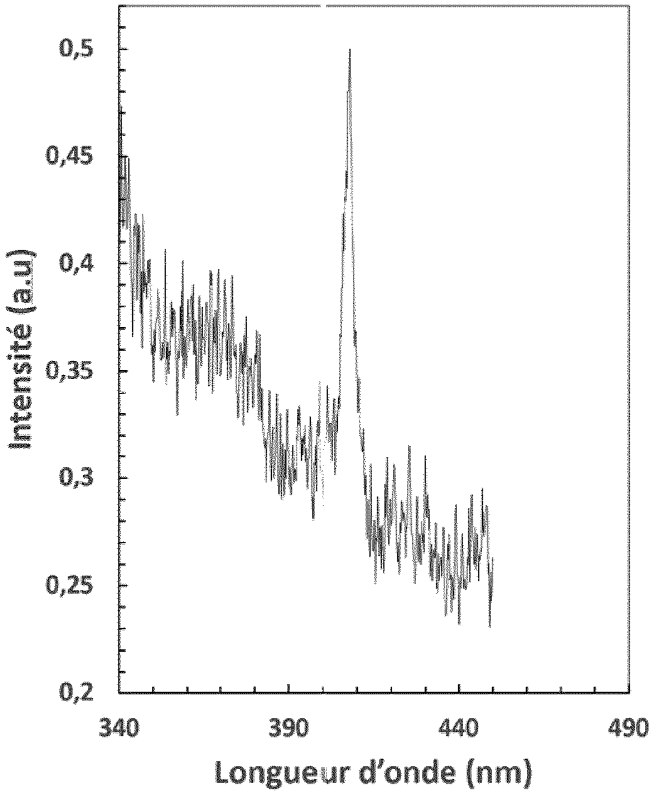


Fig. 27a

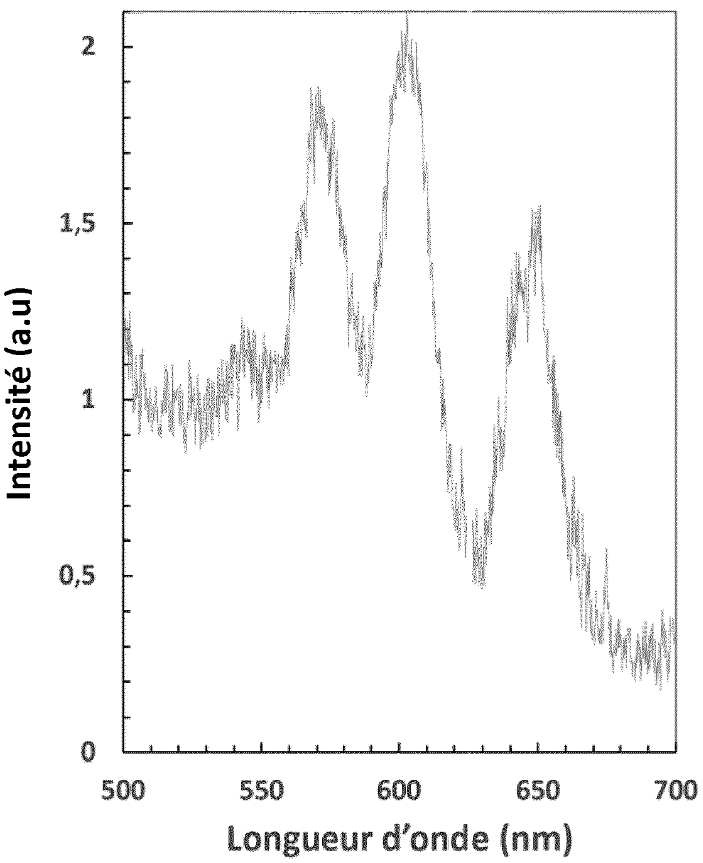


Fig. 27b

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2013132197 A
- EP 1282824 A
- US 7550201 B
- WO 03008974 A


### Littérature non-brevet citée dans la description


- **DOMITILLE GIAUME et al.** *MRS Proceedings*, 11 January 2006, vol. 942
- **ABDESSELEM et al.** *ACS Nano*, 2014, vol. 8, 11126-11137
- **HERMANSON**. Bioconjugate Techniques. Academic Press, 1996
- Fluorescent and Luminescent Probes for Biological Activity. **MASON**. A Practical Guide to Technology for Quantitative Real-Time Analysis. Academic Press, 1999
- **MEDINTZ et al.** Nat. Mat.. 2005, vol. 4, 435-446
- **NAM et al.** *Science*, 2003, vol. 301, 1884-1886
- **H. LI et al.** *Analyst*, 2011, vol. 136, 1399-1405
- **Z. CAO et al.** *Anal. Chim. Acta*, 2011, vol. 698, 44-50
- **PISANIC et al.** *Analyst*, 2014, vol. 139 (12), 2968-2981
- **GEISSLER et al.** *Angewandte Chemie International Edition*, 2010, vol. 49 (8), 1396-1401
- **HOWES et al.** *Bionanotechnology*, 2014, vol. 346 (6205), 53-63
- **GILJOHANN et al.** *Angewandte Chem*, 2010, vol. 49, 3280-3294
- **DE LA RICA et al.** *Nature Nanotechnology*, 2012, vol. 7, 821-824
- **ZHU et al.** *Anal. Chem.*, 2013, vol. 85 (2), 1058-1064
- **CORDEIRO et al.** *Diagnostics*, 2016, vol. 6 (4), 43
- **RIWOTZKI et al.** *J. Phys. Chem. B*, 1998, vol. 105, 12709-127
- **HUIGNARD et al.** *Chem. Mater.*, 2000, vol. 12, 1090-1094
- **BOUZIGUES et al.** *ACS Nano*, 2011, vol. 5 (11), 8488-8505
- **DOSEV et al.** *J. Biomed. Opt.*, 2005, vol. 10 (6), 064003
- **YI et al.** *Nano Letters*, 2004, vol. 4 (11), 2191-2196
- **BEAUREPAIRE et al.** *Nano Letters*, 2004, vol. 4 (11), 2079-2083
- **TURKCAN et al.** *Biophysical Journal*, 2012, vol. 102, 2299-2308
- **YUAN et al.** *Trends in Analytical Chemistry*, 2006, vol. 25 (5), 490-500
- **TANG et al.** *Clin Vaccine Immunol*, 2009, vol. 16 (3), 408-413
- **HÄRMÄ et al.** *Clinical Chemistry*, 2001, vol. 47 (3), 561-568
- **CORSTJENS et al.** *Clinical Biochemistry*, 2008, vol. 41 (6), 440-444
- **HEMMILÄ et al.** *Analytical Biochemistry*, 1984, vol. 137 (2), 335-343
- **JIANG et al.** *Journal of Fluorescence*, 2010, vol. 20 (1), 321-328
- **TANJA et al.** *Analytical and Bioanalytical Chemistry*, 2004, vol. 380 (1), 24-30
- **ZHOU et al.** *Angewandte Chimie*, 2014, vol. 126 (46), 12706-12710
- **RIWOTZKI et al.** *J. Phys. Chem. B*, 1998, vol. 102, 10129
- **MEZA et al.** *J. Phys. Chem. A*, 2014, vol. 118, 1390
- **DORDEVIC et al.** *J. Res. Phys.*, 2013, vol. 37, 47
- **CASANOVA et al.** *J. Am. Chem. Soc.*, 2007, vol. 129, 12592
- **GIAUME et al.** *Langmuir*, 2008, vol. 24, 11018-11026
- **ABDESSELEM et al.** *ACS Nano*, 2014, vol. 8 (11), 11126-11137
- **CASANOVA et al.** *Appl. Phys. Lett*, 2006, vol. 89, 253103
- **SON et al.** *Journal of Nanoscience and Nanotechnology*, 2008, vol. 8, 2463-2467
- **NICHKOVA et al.** *Anal. Chem.*, 2005, vol. 77, 6864-6873